(19) Europäisches Patentamt
European Patent Office
Office européen des brevets

(11) EP 4 410 772 A2

(12) **EUROPEAN PATENT APPLICATION**

(43) Date of publication:
**07.08.2024 Bulletin 2024/32**

(21) Application number: **24155771.9**

(22) Date of filing: **05.02.2024**

(51) International Patent Classification (IPC):
***C07C 211/28*** *(2006.01)* ***C07C 211/48*** *(2006.01)*
***C07C 217/08*** *(2006.01)* ***C07D 209/08*** *(2006.01)*
***C07D 215/06*** *(2006.01)* ***C07D 217/18*** *(2006.01)*
***C07D 243/08*** *(2006.01)* ***C07D 295/03*** *(2006.01)*
***C07D 295/033*** *(2006.01)* ***C08F 12/00*** *(2006.01)*

(52) Cooperative Patent Classification (CPC):
**C07C 211/28; C07C 211/48; C07C 217/08; C07D 209/08; C07D 215/06; C07D 217/18; C07D 243/08; C07D 295/03; C07D 295/033; C08F 12/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.02.2023 US 202363483365 P**
**12.01.2024 US 202418411304**

(71) Applicant: **Infineum International Limited**
**Abingdon Oxfordshire OX13 6BB (GB)**

(72) Inventors:
- **COLLINS, Michael**
  **Abingdon, OX13 6BB (GB)**
- **CLARKSON, Robert**
  **Abingdon, OX13 6BB (GB)**
- **CHAPPELL, Benjamin**
  **Abingdon, OX13 6BB (GB)**
- **HAIDASZ, Evan**
  **Linden, 07036 (US)**

(74) Representative: **Hart, Richard Joseph et al**
**PO Box 1, Milton Hill**
**Abingdon, Oxfordshire OX13 6BB (GB)**

(54) **AMINE-FUNCTIONAL MONOMERS AND METHODS OF MAKING SAME**

(57) Disclosed herein are addition-polymerizable monomer composition including an amine-derivatized alpha-methyl styrene (ADAMS) monomer according to structure (I) and/or an aminated conjugated aliphatic methylated polyene (ACAMP) monomer according to structure (II):

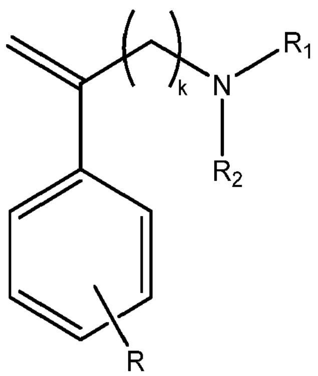

(I)

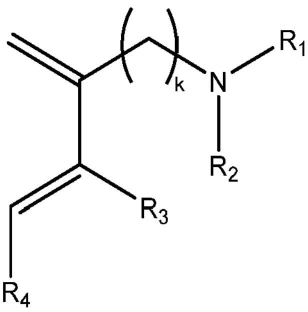

(II)

with k, $R_1$, $R_2$, $R_3$, and $R_4$ defined herein. Difunctional monomers of these types with two polymerizable loci and/or two amine loci are also disclosed herein. Further disclosed herein are methods for making such compositions.



Processed by Luminess, 75001 PARIS (FR)

## Description

### Field

This disclosure relates to addition-polymerizable monomer compositions containing aromatic and/or conjugated (non-aromatic) structures each containing at least one aminic nitrogen, as well as methods for synthesizing them.

### Background

The present disclosure is based on an unconventional way to functionalize a styrenic monomer with a nitrogen-containing moiety, other than as pendant to the phenyl ring.

There are myriad references disclosing nitrogen-containing (amine) groups pendant to the phenyl ring in styrenic monomers/polymers/copolymers. However, such pendant groups can be chemically difficult to synthesize reliably/repetitively. Even when such synthesis can be accomplished, there can be issues with propagation of polymers made from such amine-functionalized styrenic monomer, whether alone or as copolymer with other styrenic monomer.

Thus, frequently the amine group can be added to a smattering of the styrenic monomers post-polymerization. But post-polymerization chemistry can often come through with different issues, even if it may avoid the propagation issue of amine-functionalized styrenic monomers.

As such, it became desirable to develop an unconventional way to functionalize a styrenic monomer, particularly one that was neutral to, or perhaps even enhanced, the propagation of polymers made. Thus, the monomers of structure (I) were developed to achieve nitrogen-containing functionality on an alpha-substituted styrenic monomer without nitrogen-functionalizing the phenyl ring. This chemistry was also applied to amine-functionalize alkyl-substituted conjugated (non-aromatic) monomers, such as isoprene.

U.S. Patent No. 2,778,826 ("the ‘826 Schmidle patent") and a 1955 article by Schmidle and Mansfield, entitled "The Aminomethylation of Olefins. I. The Reaction of Secondary Amines, Formaldehyde, and Olefins," both disclose various reactions alleging formation of 3-aryl-3-butenyl-1-amines, in which the formaldehyde and secondary amine allegedly formed an iminium, which reacted with the styrenic olefin to form only the terminal (vinylidene) double-bond version of the amine-functional styrenic. The 1955 article also disclosed amine-functionalization of terpenoids such as $\alpha$- and $\beta$- pinene, camphene, and limonene, but not isoprene or similar conjugated non-aromatic compounds.

A 1983 article by Cohen and Onopchenko, entitled "Competing Hydride Transfer and Ene Reactions in the Aminoalkylation of 1-Alkenes with N,N-Dimethylmethyleniminium Ions. A Literature Correction" (citing, in part, to the ‘826 Schmidle patent, *inter alia*), further disclosed a mechanistic study of specifically dimethyliminimum compounds reacting with styrenic and non-styrenic olefins. Notably, at the beginning of the Discussion section, the 1983 article opines that the ‘826 Schmidle patent (and presumably the 1955 article containing strikingly similar experiments and results thereto) had made mistakes. Nonetheless, with respect to aminomethylation of $\alpha$-methylstyrene, the 1983 article indicated formation of vinylidene-based product, in tandem with a significant vinylene (not terminal double-bond) content and a quite significant (13%, in the case of the dimethylamino-version) saturated arylalkane-amine content.

GB Patent No. 1 381 755 also discloses amine-functional monomer compounds, but only with acrylamide functionality. Other examples of potentially relevant publications include, but are not necessarily limited to, U.S. Patent Nos. 7,790,661, 7,960,320, 8,778,854, and 10,414,999; and PCT Publication No. WO 2021/127183, *inter alia.*

### Summary

Accordingly, the present disclosure provides addition-polymerizable monomer compositions of structures (I), (II), (V), and (VI) below, with emphasis on the vinylidene (terminal double-bond, or "exo") version being the desired product. Other byproducts may be present, as dictated by the various synthesis schemes by which the products (and byproducts) are made, examples of which are also provided by the present disclosure. As the monomer compositions disclosed herein are disclosed to be addition-polymerizable, one theoretical utility for such monomer compositions is as a complete or partial monomer feed source for addition (co)polymerization.

Other aspects of the present disclosure may become apparent from the Detailed Description and Examples sections hereinbelow.

### Brief Description of the Drawings

Figures 1-2 show portions of sample quantitative $^1$H NMR spectra to illustrate how yields of different products and byproducts are assigned and evaluated.

Detailed Description

Addition-polymerizable monomer compositions according to the present disclosure can involve amine-derivatized alpha-methyl styrene (ADAMS) monomers according to structure (I) and/or aminated conjugated (non-aromatic) aliphatic methylated polyene (ACAMP) monomers according to structure (II).

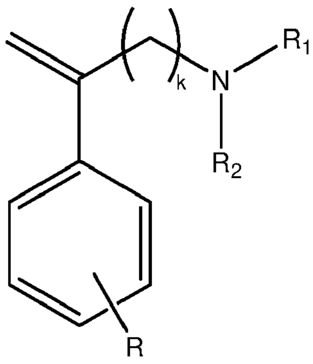

(I)

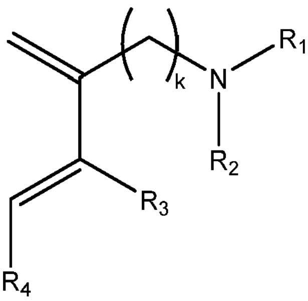

(II)

wherein k is an integer from 1 to 3, preferably 2; wherein $R_1$ and $R_2$ are each independently a hydrocarbyl group or a hydrocarbonaceous group having 1 to 4 additional heteroatoms, such as O, N, S, P, Se, and combinations thereof, or wherein $R_1$ and $R_2$ are connected to form a moiety containing at least one 5- to 12- membered ring, from 3 to 24 carbons, and optionally 1 to 6 additional heteroatoms (such as O, N, S, P, Se, and combinations thereof); wherein R, in structure (I), is hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group (such as a methyl group), a $C_1$-$C_6$ hydrocarbyl group containing 1 to 4 additional heteroatoms (such as O, N, S, P, Se, and combinations thereof), or a second amino-functional group having structure (III)

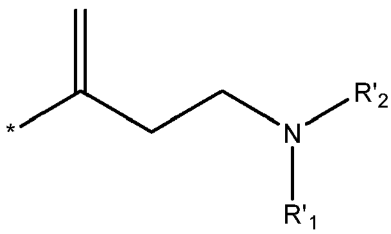

(III)

attached via the asterisk, with $R'_1$ and $R'_2$ being independently the same or different as $R_1$ and $R_2$ but defined identically; and wherein $R_3$ and $R_4$, in structure (II), are each independently hydrogen, a methyl group, or a second amino-functional group having structure (III) attached via the asterisk, with $R'_1$ and $R'_2$ being independently the same or different as $R_1$ and $R_2$ but defined identically, or an alkenyl group having structure (IV)

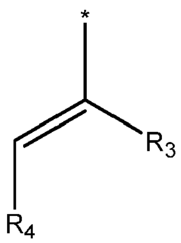

(IV)

attached via the asterisk, but specifically which do not collectively achieve aromaticity in combination with the other (conjugated) alkenes in structure (II), and with the proviso that $R_3$ and $R_4$ are not both alkenyl groups having structure (IV) and are not both methyl groups.

Examples of ADAMS monomers according to structure (I) may include, but are not limited to, 1-dimethylamino-3-phenylbut-3-ene, 1-diethylamino-3-phenylbut-3-ene, 1-di-n-propylamino-3-phenylbut-3-ene, 1-diisopropylamino-3-phenylbut-3-ene, 1-di-2-propenylamino-3-phenylbut-3-ene, 1-di-n-butylamino-3-phenylbut-3-ene, 1-di-sec-butylamino-3-phenylbut-3-ene, 1-diisobutylamino-3-phenylbut-3-ene, 1-di-tert-butylamino-3-phenylbut-3-ene, 1-cyclohexylmethylamino-3-phenylbut-3-ene, 1-dicyclohexylamino-3-phenylbut-3-ene, 1-di-(2-ethylhexyl)amino-3-phenylbut-3-ene, 1-di-(methoxyethyl)amino-3-phenylbut-3-ene, 1-di-(ethoxyethyl)amino-3-phenylbut-3-ene, 1-di-(phenoxyethyl)amino-3-phenylbut-3-ene, 1-di-(methylthioethyl)amino-3-phenylbut-3-ene, 1-di-(ethylthioethyl)amino-3-phenylbut-3-ene, 1-benzylmethylamino-3-phenylbut-3-ene, 1-dibenzylamino-3-phenylbut-3-ene, 1-benzylphenylamino-3-phenylbut-3-ene, 1-diphenylamino-3-phenylbut-3-ene, 1-dipyridylamino-3-phenylbut-3-ene, 1-phenylmethylamino-3-phenylbut-3-ene, 1-phenylmethoxyethylamino-3-phenylbut-3-ene, 1-benzylmethoxyethylamino-3-phenylbut-3-ene, 1-(N-morpholinyl)-3-phenylbut-3-ene, 1-(N-thiomorpholinyl)-3-phenylbut-3-ene, 1-(N-piperidinyl)-3-phenylbut-3-ene, 1-(N-piperazinyl)-3-phenylbut-3-ene, 1-(N-diazepanyl)-3-phenylbut-3-ene, 1-(N-pyrrolidinyl)-3-phenylbut-3-ene, 1-(N-pyrrolyl)-3-phenylbut-3-ene, 1-(1,2,3,4-tetrahydro-1-quinolinyl)-3-phenylbut-3-ene, 1-(1,2,3,4-tetrahydro-2-isoquinolinyl)-3-phenylbut-3-ene, 1-(N-indolinyl)-3-phenylbut-3-ene, 1-(N-indolyl)-3-phenylbut-3-ene, 1-(N-carbazolyl)-3-phenylbut-3-ene, 1-(N-phenothiazinyl)-3-phenylbut-3-ene, 1-(N-phenothiazinyl-S-oxide)-3-phenylbut-3-ene, 1-(N-phenothiazinyl-S,S-dioxide)-3-phenylbut-3-ene, 1-(N-phenoxazinyl)-3-phenylbut-3-ene, 1-(4-methyl-1-piperazinyl)-3-phenylbut-3-ene, 1-(5-methyl-2,5-diazabicyclo[2.2.1]heptan-2-yl)-3-phenylbut-3-ene, 1-(5-methyl-2,5-diazabicyclo[2.2.2]octan-2-yl)-3-phenylbut-3-ene, 1-(4-cyclopentyl-1-piperazinyl)-3-phenylbut-3-ene, 1-(4-cyclopentadienyl-1-piperazinyl)-3-phenylbut-3-ene, 1-(4-phenyl-1-piperazinyl)-3-phenylbut-3-ene, 1-(4-(thiazolyl)-1-piperazinyl)-3-phenylbut-3-ene, 1-(4-(thiadiazolyl)-1-piperazinyl)-3-phenylbut-3-ene, 1-(4-(triazolyl)-1-piperazinyl)-3-phenylbut-3-ene, 1-(4-(1,2,3-benzotriazolyl)-1-piperazinyl)-3-phenylbut-3-ene, 1-(N'-methyl-N-diazepanyl)-3-phenylbut-3-ene, N,N'-bis(3-phenylbut-3-enyl)diazepane, N,N'-bis(3-phenylbut-3-enyl)piperazine, N,N'-bis(3-phenylbut-3-enyl)dihydrophenazine, N,N'-bis(3-phenylbut-3-enyl)dihydrobenzoindazole, N,N'-bis(3-phenylbut-3-enyl)dihydropermidine, N,N'-bis(3-phenylbut-3-enyl)octahydropyridoquinoline, N,N'-bis(3-phenylbut-3-enyl)octahydropyridoisoquinoline, N,N'-bis(3-phenylbut-3-enyl)hexahydropyrroloquinoline, N,N'-bis(3-phenylbut-3-enyl)hexahydropyrroloisoquinoline, N,N'-bis(3-phenylbut-3-enyl)hexahydropyrroloisoindole, N,N'-bis(3-phenylbut-3-enyl)diazabicyclo[2.2.1]heptane, N,N' -bis(3 -phenylbut-3 - enyl)diazabicyclo [2.2.2] octane, 1,3-bis(1-(3 -phenylbut-3 -enyl)piperidin-4-yl)propane, bis(1-dimethylamino-3-phenylbut-3-enyl)benzene, bis(1-benzylmethylamino-3-phenylbut-3-enyl)benzene, bis(1-(N-morpholinyl)-3-phenylbut-3-enyl)benzene, bis(1-(N-thiomorpholinyl)-3-phenylbut-3-enyl)benzene, bis(1-(di-methoxyethyl)amino-3-phenylbut-3-enyl)benzene, bis(1-(N-piperidinyl)-3-phenylbut-3-enyl)benzene, bis(1-(N-pyrrolidinyl)-3-phenylbut-3-enyl)benzene, bis(1-(4-methyl-1-piperazinyl))-3-phenylbut-3-enyl)benzene, and combinations thereof.

In some embodiments, the ADAMS monomers according to structure (I) may exhibit a k value of exactly 2. Additionally or alternatively, the ADAMS monomers according to structure (I) may specifically exclude, or comprise substantially no, 1-dimethylamino-3-phenylbut-3-ene, 1-diethylamino-3-phenylbut-3-ene, N-morpholinyl-3-phenylbut-3-ene, 1-benzylmethylamino-3-phenylbut-3-ene, 1-dimethylamino-3-p-tolylbut-3-ene, 1-diethylamino-3-p-tolylbut-3-ene, N-morpholinyl-3-p-tolylbut-3-ene, N-piperidinyl-3-p-tolylbut-3-ene, and N-pyrrolidinyl-3-p-tolylbut-3-ene. Further additionally or alternatively, the ADAMS monomers according to structure (I) may specifically exclude, or comprise substantially none of, embodiments (a) in which $R_1$ and $R_2$ are each independently a benzyl group or either a "lower" alkyl or a $C_1$-$C_{12}$ alkyl group, and (b) in which $R_1$ and $R_2$ are connected to form a singular (unsubstituted) nitrogen-containing 5- or 6- membered heterocyclic ring (as written, inclusive of 4-morpholino and 4-thiomorpholino moieties).

For clarity, and as used herein, the ADAMS monomers of structure (I) have a vinylidene bond, such as originating from the olefinic double-bond in alpha-methylstyrene, which can be reflected in -3-ene/-3-enyl language of the IUPAC nomenclature, for example. The isomer containing this vinylidene bond is herein termed the "exo" isomer. Isomers containing an internal vinylene bond, such as reflected in -2-ene/-2-enyl language in IUPAC nomenclature, are herein called "endo" isomers.

Based on certain reaction mechanisms (discussed further below) from synthesis of exo isomer monomers

according to structure (I), alternatively or in addition to endo isomer byproducts, saturation byproduct can be formed. "Saturation byproduct", with respect to the exo isomer monomers according to structure (I), refers collectively to isomers in which the double bond (exo or endo) has been saturated, such as reflected in -ane/-anyl language in IUPAC nomenclature. While there may be multiple different individual saturation byproduct isomers in any given case, one example can include where one of the two moieties on the amine can undergo an alkylative demethylation to saturate the double bond to form a saturated secondary amine (*e.g.*, in the case of aza-prins reaction of alpha-methylstyrene and an iminium intermediate that can be formed by a combination of formaldehyde and dimethylamine or substitute/conjugate reactants, the exo isomer would be 1-dimethylamino-3-phenylbut-3-ene; the endo isomer would be 1-dimethylamino-3-phenylbut-2-ene; and the alkylatively demethylated saturation byproduct would be 1-methylamino-3-phenylbutane, as noted in T. Cohen et al., J. Org. Chem., 1983, v. 48, pp. 4531-37, abbreviated herein as "the Cohen article").

Based on certain reaction mechanisms (discussed further below) from synthesis of exo isomer monomers according to structure (I), alternatively or in addition to endo isomer byproducts and/or saturation byproduct, hydroxylation byproduct can be formed. "Hydroxylation byproduct", with respect to the exo isomer monomers according to structure (I), refers collectively to isomers in which the double bond (exo or endo) has been hydrolyzed, such as reflected in -anol language in IUPAC nomenclature, as well as to compounds formed from reaction of less than all three reactant sources. While there may be multiple different individual hydroxylation byproduct isomers in any given case, one example can include where the double bond (of the exo and/or endo monomer isomer(s)) is hydrated by water and/or where the saturated acid conjugate is hydrolyzed, while another example can include direct reaction of (one or two moles of) formaldehyde with the aromatic starting material (thus, *e.g.,* in the case of aza-prins reaction of alpha-methylstyrene and an iminium intermediate that can be formed by a combination of formaldehyde and dimethylamine or substitute/conjugate reactants, the exo isomer would be 1-dimethylamino-3-phenylbut-3-ene; the endo isomer would be 1-dimethylamino-3-phenylbut-2-ene; one possible saturation byproduct would be 1-methylamino-3-phenylbutane; and possible examples of hydroxylation byproduct would be 1-dimethylamino-3-phenyl-3-hydroxybutane, from the hydrolyzation of the exo/endo isomer, and 3-phenyl-1-hydroxybut-3-ene, 3-phenyl-1-hydroxybut-2-ene, and/or 4-methyl-4-phenyl-1,3-dioxane, from reaction of one, one, and/or two moles formaldehyde with one mole of alpha-methylstyrene, respectively - even though the dioxane byproduct possesses no hydroxyl moieties per se, the acetal is formed through one of the other two hydroxylated intermediates and thus is categorized as a "hydroxylated byproduct" herein).

Based on certain reaction mechanisms (discussed further below) from synthesis of exo isomer monomers according to structure (I), alternatively or in addition to endo isomer byproducts, saturation byproduct, and/or hydroxylation byproduct, residual alpha-methylstyrene can be present (*e.g.*, as an unconsumed reactant in an aza-prins reaction).

Based on certain reaction mechanisms (discussed further below) from synthesis of exo isomer monomers according to structure (I), alternatively or in addition to endo isomer byproducts, saturation byproduct, hydroxylation byproduct, and/or residual alpha-methylstyrene reactant, dimerized and/or oligomerized byproduct can be formed. Without being bound by theory (and as discussed further below), such reactions are believed to result from too acidic a pH and may include dimers/oligomers of reactant (such as alpha-methylstyrene), of exo (or endo) product (according to the present disclosure), or of a combination thereof.

Additionally or alternatively, the addition-polymerizable monomer compositions according to the disclosure can contain, in addition to (exo isomer) monomers according to structure (I), one or more of the following: (1) at least about 0.5 wt%, based on the reaction-medium free composition weight (*e.g.*, at least about 0.8 wt%, at least about 1.0 wt%, at least about 1.3 wt%, at least about 1.5 wt%, at least about 1.8 wt%, at least about 2.0 wt%, at least about 2.5 wt%, at least about 3.0 wt%, at least about 3.5 wt%, at least about 4.0 wt%, at least about 4.5 wt%, or at least about 5.0 wt%), of endo isomer monomer corresponding to the relevant exo isomer monomer, optionally up to about 20 wt% (*e.g.*, up to about 18 wt%, up to about 15 wt%, up to about 13 wt%, up to about 10 wt%, up to about 9.0 wt%, up to about 8.0 wt%, up to about 7.5 wt%, up to about 7.0 wt%, up to about 6.5 wt%, up to about 6.0 wt%, up to about 5.5 wt%, or up to about 5.0 wt%); (2) not more than about 12 wt%, based on the reaction-medium free composition weight (*e.g.*, not more than about 10 wt%, not more than about 9.5 wt%, not more than about 9.0 wt%, not more than about 8.5 wt%, not more than about 8.0 wt%, not more than about 7.5 wt%, not more than about 7.0 wt%, not more than about 6.5 wt%, not more than about 6.0 wt%, not more than about 5.5 wt%, not more than about 5.0 wt%, not more than about 4.5 wt%, not more than about 4.0 wt%, not more than about 3.5 wt%, not more than about 3.0 wt%, not more than about 2.5 wt%, not more than about 2.0 wt%, not more than about 1.5 wt%, not more than about 1.0 wt%, or not more than about 0.5 wt%), of saturation byproduct; (3) at least about 50 ppm by weight, or "wppm", based on the reaction-medium free composition weight (*e.g.*, at least about 75 wppm, at least about 100 wppm, at least about 150 wppm, at least about 200 wppm, at least about 250 wppm, at least about 300 wppm, at least about 400 wppm, at least about 500 wppm, at least about 750 wppm, or at least about 0.1 wt%), of optionally ring-substituted alpha-methylstyrene, optionally up to about 10 wt% (*e.g.,* up to about 7.5 wt%, up to about 5.0 wt%, up to about 2.5 wt%, up to about 2.0 wt%, up to about 1.5 wt%, up to about 1.0 wt%, up to about 0.8 wt%, up to about 0.5 wt%, up to about 0.4 wt%, up to about 0.3 wt%, or up to about 0.2 wt%); (4) not more than about 0.3 wt%, based on the reaction-medium free composition weight (*e.g.,* not more than about 0.2 wt%, not more than about 0.1 wt%, not more than about 800 wppm, not more than about 700

wppm, not more than about 600 wppm, not more than about 500 wppm, not more than about 400 wppm, not more than about 300 wppm, not more than about 200 wppm, or not more than about 100 wppm), of hydroxylation byproduct; (5) not more than about 5.0 wt%, based on the reaction-medium free composition weight (*e.g.,* not more than about 4.0 wt%, not more than about 3.0 wt%, not more than about 2.5 wt%, not more than about 2.0 wt%, not more than about 1.5 wt%, not more than about 1.0 wt%, not more than about 0.5 wt%, not more than about 0.3 wt%, not more than about 0.2 wt%, not more than about 0.1 wt%, not more than about 800 wppm, not more than about 700 wppm, not more than about 600 wppm, not more than about 500 wppm, not more than about 400 wppm, not more than about 300 wppm, not more than about 200 wppm, or not more than about 100 wppm), of dimerization/oligomerization byproduct; and (6) not more than about 0.5 wt%, based on the reaction-medium free composition weight (*e.g.,* not more than about 0.3 wt%, not more than about 0.2 wt%, not more than about 0.1 wt%, not more than about 800 wppm, not more than about 700 wppm, not more than about 600 wppm, not more than about 500 wppm, not more than about 400 wppm, not more than about 300 wppm, not more than about 200 wppm, or not more than about 100 wppm), of an acid anhydride (preferably a conjugate anhydride of acid used in the synthesis process, such as acetic anhydride when acetic acid is present in an aza-prins synthesis reaction to form the exo isomer monomer product; however, as used herein, the term "acid anhydride" should be understood to further include acid chlorides and/or acid bromides, which, when used, would preferably refer to a conjugate chloride and/or bromide of the acid used in the synthesis process, *e.g.,* acetic acid). As used herein, "reaction medium-free composition weight" is defined as the total composition weight minus the collective amount of any reaction medium/media (*e.g.*, in the form of acid, solvent, and/or other diluent that may be present in the synthesis process). For example, in a typical aza-prins synthesis reaction process, the total composition can comprise one, some, or all of exo isomer, endo isomer, acid conjugate intermediate, hydrate byproduct, saturated byproduct, residual (*e*.g,, alpha-methyl styrene) monomer, acid anhydride, derivatives of the starting reactants, other reaction intermediates, other reaction products/byproducts, and reaction media (including but not limited to acid, solvent, and other diluent), whereas the reaction medium-free composition weight would exclude the reaction media.

Examples of ACAMP monomers according to structure (II) may include, but are not limited to, 1-dimethylamino-3-methylenepent-4-ene, 1-diethylamino-3-methylenepent-4-ene, 1-di-n-propylamino-3-methylenepent-4-ene, 1-diisopropylamino-3-methylenepent-4-ene, 1-di-2-propenylamino-3-methylenepent-4-ene, 1-di-n-butylamino-3-methylenepent-4-ene, 1-di-sec-butylamino-3-methylenepent-4-ene, 1-diisobutylamino-3-methylenepent-4-ene, 1-di-tert-butylamino-3-methylenepent-4-ene, 1-cyclohexylmethylamino-3-methylenepent-4-ene, 1-dicyclohexylamino-3-methylenepent-4-ene, 1-di-(2-ethylhexyl)amino-3-methylenepent-4-ene, 1-di-(methoxyethyl)amino-3-methylenepent-4-ene, 1-di-(ethoxyethyl)amino-3-methylenepent-4-ene, 1-di-(phenoxyethyl)amino-3-methylenepent-4-ene, 1-di-(methylthioethyl)amino-3-methylenepent-4-ene, 1-di-(ethylthioethyl)amino-3-methylenepent-4-ene, 1-benzylmethylamino-3-methylenepent-4-ene, 1-dibenzylamino-3-methylenepent-4-ene, 1-benzylphenylamino-3-methylenepent-4-ene, 1-diphenylamino-3-methylenepent-4-ene, 1-dipyridylamino-3-methylenepent-4-ene, 1-phenylmethylamino-3-methylenepent-4-ene, 1-phenylmethoxyethylamino-3-methylenepent-4-ene, 1-benzylmethoxyethylamino-3-methylenepent-4-ene, 1-(N-morpholinyl)-3-methylenepent-4-ene, 1-(N-thiomorpholinyl)-3-methylenepent-4-ene, 1-(N-piperidinyl)-3-methylenepent-4-ene, 1-(N-piperazinyl)-3-methylenepent-4-ene, 1-(N-diazepanyl)-3-methylenepent-4-ene, 1-(N-pyrrolidinyl)-3-methylenepent-4-ene, 1-(N-pyrrolyl)-3-methylenepent-4-ene, 1-(1,2,3,4-tetrahydro-1-quinolinyl)-3-methylenepent-4-ene, 1-(1,2,3,4-tetrahydro-2-isoquinolinyl)-3-methylenepent-4-ene, 1-(N-indolinyl)-3-methylenepent-4-ene, 1-(N-indolyl)-3-methylenepent-4-ene, 1-(N-carbazolyl)-3-methylenepent-4-ene, 1-(N-phenothiazinyl)-3-methylenepent-4-ene, 1-(N-phenothiazinyl-S-oxide)-3-methylenepent-4-ene, 1-(N-phenothiazinyl-S,S-dioxide)-3-methylenepent-4-ene, 1-(N-phenoxazinyl)-3-methylenepent-4-ene, 1-(4-methyl-1-piperazinyl)-3-methylenepent-4-ene, 1-(5-methyl-2,5-diazabicyclo[2.2.1]heptan-2-yl)-3-methylenepent-4-ene, 1-(5-methyl-2,5-diazabicyclo[2.2.2]octan-2-yl)-3-methylenepent-4-ene, 1-(4-cyclopentyl-1-piperazinyl)-3-methylenepent-4-ene, 1-(4-cyclopentadienyl-1-piperazinyl)-3-methylenepent-4-ene, 1-(4-phenyl-1-piperazinyl)-3-methylenepent-4-ene, 1-(4-(thiazolyl)-1-piperazinyl)-3-methylenepent-4-ene, 1-(4-(thiadiazolyl)-1-piperazinyl)-3-methylenepent-4-ene, 1-(4-(triazolyl)-1-piperazinyl)-3-methylenepent-4-ene, 1-(4-(1,2,3-benzotriazolyl)-1-piperazinyl)-3-methylenepent-4-ene, 1-(N'-methyl-N-diazepanyl)-3-methylenepent-4-ene, 1-dimethylamino-3-methylenehepta-4,6-diene, 1-diethylamino-3-methylenehepta-4,6-diene, 1-di-n-propylamino-3-methylenehepta-4,6-diene, 1-diisopropylamino-3-methylenehepta-4,6-diene, 1-di-2-propenylamino-3-methylenehepta-4,6-diene, 1-di-n-butylamino-3-methylenehepta-4,6-diene, 1-di-sec-butylamino-3-methylenehepta-4,6-diene, 1-diisobutylamino-3-methylenehepta-4,6-diene, 1-di-tert-butylamino-3-methylenehepta-4,6-diene, 1-cyclohexylmethylamino-3-methylenehepta-4,6-diene, 1-dicyclohexylamino-3-methylenehepta-4,6-diene, 1-di-(2-ethylhexyl)amino-3-methylenehepta-4,6-diene, 1-di-(methoxyethyl)amino-3-methylenehepta-4,6-diene, 1-di-(ethoxyethyl)amino-3-methylenehepta-4,6-diene, 1-di-(phenoxyethyl)amino-3-methylenehepta-4,6-diene, 1-di-(methylthioethyl)amino-3-methylenehepta-4,6-diene, 1-di-(ethylthioethyl)amino-3-methylenehepta-4,6-diene, 1-benzylmethylamino-3-methylenehepta-4,6-diene, 1-dibenzylamino-3-methylenehepta-4,6-diene, 1-benzylphenylamino-3-methylenehepta-4,6-diene, 1-diphenylamino-3-methylenehepta-4,6-diene, 1-dipyridylamino-3-methylenehepta-4,6-diene, 1-phenylmethylamino-3-methylenehepta-4,6-diene, 1-phenylmethoxyethylamino-3-methylenehepta-4,6-diene, 1-benzylmethoxyethylamino-3-methylenehepta-4,6-diene, 1-(N-morpholinyl)-3-methylenehepta-4,6-diene, 1-(N-thiomorpholinyl)-3-methylenehepta-4,6-diene, 1-(N-piperidinyl)-3-methylenehepta-4,6-diene, 1-(N-piperazinyl)-3-methylenehepta-4,6-diene, 1-(N-diazepanyl)-3-methylenehepta-4,6-diene, 1-(N-pyrrolidinyl)-3-methylenehepta-4,6-diene, 1-(N-pyrrolyl)-3-methylenehepta-4,6-diene, 1-(1,2,3,4-tetrahydro-1-quinolinyl)-3-methylenehepta-4,6-diene, 1-(1,2,3,4-tetrahydro-2-isoquinolinyl)-3-methylenehepta-4,6-diene, 1-(N-indolinyl)-3-methylenehepta-4,6-diene, 1-(N-indolyl)-3-methylenehepta-4,6-diene, 1-(N-carbazolyl)-3-methylenehepta-4,6-diene, 1-(N-phenothiazinyl)-3-methylenehepta-4,6-diene, 1-(N-phenothiazinyl-S-oxide)-3-methylenehepta-4,6-diene, 1-(N-phenothiazinyl-S,S-dioxide)-3-methylenehepta-4,6-diene, 1-(N-phenoxazinyl)-3-methylenehepta-4,6-diene, 1-(4-methyl-1-piperazinyl)-3-methylenehepta-4,6-diene, 1-(5-methyl-2,5-diazabicyclo[2.2.1]heptan-2-yl)-3-methylenehepta-4,6-diene, 1-(5-methyl-2,5-diazabicyclo[2.2.2]octan-2-yl)-3-methylenehepta-4,6-diene, 1-(4-cyclopentyl-1-piperazinyl)-3-methylenehepta-4,6-diene, 1-(4-cyclopentadienyl-1-piperazinyl)-3-methylenehepta-4,6-diene, 1-(4-phenyl-1-piperazinyl)-3-methylenehepta-4,6-diene, 1-(4-(thiazolyl)-1-piperazinyl)-3-methylenehepta-4,6-diene, 1-(4-(thiadiazolyl)-1-piperazinyl)-3-methylenehepta-4,6-diene, 1-(4-(triazolyl)-1-piperazinyl)-3-methylenehepta-4,6-diene, 1-(4-(1,2,3-benzotriazolyl)-1-piperazinyl)-3-methylenehepta-4,6-diene, 1-(N'-methyl-N-diazepanyl)-3-methylenehepta-4,6-diene, 1-dimethylamino-3,4-dimethylenehex-5-ene, 1-diethylamino-3,4-dimethylenehex-5-ene, 1-di-n-propylamino-3,4-dimethylenehex-5-ene, 1-diisopropylamino-3,4-dimethylenehex-5-ene, 1-di-2-propenylamino-3,4-dimethylenehex-5-ene, 1-di-n-butylamino-3,4-dimethylenehex-5-ene, 1-di-sec-butylamino-3,4-dimethylenehex-5-ene, 1-diisobutylamino-3,4-dimethylenehex-5-ene, 1-di-tert-butylamino-3,4-dimethylenehex-5-ene, 1-cyclohexylmethylamino-3,4-dimethylenehex-5-ene, 1-dicyclohexylamino-3,4-dimethylenehex-5-ene, 1-di-(2-ethylhexyl)amino-3,4-dimethylenehex-5-ene, 1-di-(methoxyethyl)amino-3,4-dimethylenehex-5-ene, 1-di-(ethoxyethyl)amino-3,4-dimethylenehex-5-ene, 1-di-(phenoxyethyl)amino-3,4-dimethylenehex-5-ene, 1-di-(methylthioethyl)amino-3,4-dimethylenehex-5-ene, 1-di-(ethylthioethyl)amino-3,4-dimethylenehex-5-ene, 1-benzylmethylamino-3,4-dimethylenehex-5-ene, 1-dibenzylamino-3,4-dimethylenehex-5-ene, 1-benzylphenylamino-3,4-dimethylenehex-5-ene, 1-diphenylamino-3,4-dimethylenehex-5-ene, 1-dipyridylamino-3,4-dimethylenehex-5-ene, 1-phenylmethylamino-3,4-dimethylenehex-5-ene, 1-phenylmethoxyethylamino-3,4-dimethylenehex-5-ene, 1-benzylmethoxyethylamino-3,4-dimethylenehex-5-ene, 1-(N-morpholinyl)-3,4-dimethylenehex-5-ene, 1-(N-thiomorpholinyl)-3,4-dimethylenehex-5-ene, 1-(N-piperidinyl)-3,4-dimethylenehex-5-ene, 1-(N-piperazinyl)-3,4-dimethylenehex-5-ene, 1-(N-diazepanyl)-3,4-dimethylenehex-5-ene, 1-(N-pyrrolidinyl)-3,4-dimethylenehex-5-ene, 1-(N-pyrrolyl)-3,4-dimethylenehex-5-ene, 1-(1,2,3,4-tetrahydro-1-quinolinyl)-3,4-dimethylenehex-5-ene, 1-(1,2,3,4-tetrahydro-2-isoquinolinyl)-3,4-dimethylenehex-5-ene, 1-(N-indolinyl)-3,4-dimethylenehex-5-ene, 1-(N-indolyl)-3,4-dimethylenehex-5-ene, 1-(N-carbazolyl)-3,4-dimethylenehex-5-ene, 1-(N-phenothiazinyl)-3,4-dimethylenehex-5-ene, 1-(N-phenothiazinyl-S-oxide)-3,4-dimethylenehex-5-ene, 1-(N-phenothiazinyl-S,S-dioxide)-3,4-dimethylenehex-5-ene, 1-(N-phenoxazinyl)-3,4-dimethylenehex-5-ene, 1-(4-methyl-1-piperazinyl)-3,4-dimethylenehex-5-ene, 1-(5-methyl-2,5-diazabicyclo[2.2.1]heptan-2-yl)-3,4-dimethylenehex-5-ene, 1-(5-methyl-2,5-diazabicyclo[2.2.2]octan-2-yl)-3,4-dimethylenehex-5-ene, 1-(4-cyclopentyl-1-piperazinyl)-3,4-dimethylenehex-5-ene, 1-(4-cyclopentadienyl-1-piperazinyl)-3,4-dimethylenehex-5-ene, 1-(4-phenyl-1-piperazinyl)-3,4-dimethylenehex-5-ene, 1-(4-(thiazolyl)-1-piperazinyl)-3,4-dimethylenehex-5-ene, 1-(4-(thiadiazolyl)-1-piperazinyl)-3,4-dimethylenehex-5-ene, 1-(4-(triazolyl)-1-piperazinyl)-3,4-dimethylenehex-5-ene, 1-(4-(1,2,3-benzotriazolyl)-1-piperazinyl)-3,4-dimethylenehex-5-ene, 1-(N'-methyl-N-diazepanyl)-3,4-dimethylenehex-5-ene, N,N'-bis(3-methylenepent-4-enyl)diazepane, N,N'-bis(3-methylenepent-4-enyl)piperazine, N,N'-bis(3-methylenepent-4-enyl)dihydrophenazine, N,N'-bis(3-methylenepent-4-enyl)dihydrobenzoindazole, N,N'-bis(3-methylenepent-4-enyl)dihydropermidine, N,N'-bis(3-methylenepent-4-enyl)octahydropyridoquinoline, N,N'-bis(3-methylenepent-4-enyl)octahydropyridoisoquinoline, N,N'-bis(3-methylenepent-4-enyl)hexahydropyrroloquinoline, N,N'-bis(3-methylenepent-4-enyl)hexahydropyrroloisoquinoline, N,N' -bis(3-methylenepent-4-enyl)hexahydropyrroloisoindole, N,N'-bis(3-methylenepent-4-enyl)diazabicyclo[2.2.1]heptane, N,N'-bis(3-methylenepent-4-enyl)diazabicyclo [2.2.2] octane, 1,3-bis(1-(3 -methylenepent-4-enyl)piperidin-4-yl)propane, N,N'-bis(3-methylenehepta-4,6-dien-1-yl)diazepane, N,N'-bis(3-methylenehepta-4,6-dien-1-yl)piperazine, N,N'-bis(3-methylenehepta-4,6-dien-1-yl)dihydrophenazine, N,N'-bis(3-methylenehepta-4,6-dien-1-yl)dihydrobenzoindazole, N,N'-bis(3-methylenehepta-4,6-dien-1-yl)dihydropermidine, N,N'-bis(3-methylenehepta-4,6-dien-1-yl)octahydropyridoquinoline, N,N'-bis(3-methylenehepta-4,6-dien-1-yl)octahydropyridoisoquinoline, N,N'-bis(3-methylenehepta-4,6-dien-1-yl)hexahydropyrroloquinoline, N,N'-bis(3-methylenehepta-4,6-dien-1-yl)hexahydropyrroloisoquinoline, N,N'-bis(3-methylenehepta-4,6-dien-1-yl)hexahydropyrroloisoindole, N,N'-bis(3-methylenehepta-4,6-dien-1-yl)diazabicyclo[2.2.1]heptane, N,N'-bis(3-methylenehepta-4,6-dien-1-yl)diazabicyclo[2.2.2]octane, 1,3-bis(1-(3-methylenehepta-4,6-dienyl)piperidin-4-yl)propane, N,N'-bis(3,4-dimethylenehex-5-en-1-yl)diazepane, N,N'-bis(3,4-dimethylenehex-5-en-1-yl)piperazine, N,N'-bis(3,4-dimethylenehex-5-en-1-yl)dihydrophenazine, N,N'-bis(3,4-dimethylenehex-5-en-1-yl)dihydrobenzoindazole, N,N'-bis(3,4-dimethylenehex-5-en-1-yl)dihydropermidine, N,N'-bis(3,4-dimethylenehex-5-en-1-yl)octahydropyridoquinoline, N,N'-bis(3,4-dimethylenehex-5-en-1-yl)octahydropyridoisoquinoline, N,N' -bis(3,4-dimethylenehex-5-en-1-yl)hexahydropyrroloquinoline, N,N'-bis(3,4-dimethylenehex-5-en-1-yl)hexahydropyrroloisoquinoline, N,N'-bis(3,4-dimethylenehex-5-en-1-yl)hexahydropyrroloisoindole, N,N'-bis(3,4-dimethylenehex-5-en-1-yl)diazabicyclo[2.2.1]heptane, N,N'-bis(3,4-dimethylenehex-5-en-1-yl)diazabicyclo[2.2.2]octane, 1,3-bis(1-(3,4-dimethylenehex-5-enyl)piperidin-4-yl)propane, N,N,N'N'-tetramethyl-3,6-dimethyleneoct-4-ene-1,8-diamine, N,N,N'N'-tetrakis(2-methoxyethyl)-3,6-dimethyleneoct-4-ene-1,8-diamine, N,N'-dimethyl-N,N'-dibenzyl-3,6-dimethyleneoct-4-ene-1,8-diamine, N,N'-(3,6-dimethyleneoct-4-ene-1,8-diyl)bis(morpholine), N,N'-(3,6-dimethyleneoct-4-ene-1,8-diyl)bis(thiomorpholine), N,N'-(3,6-dimethyleneoct-4-ene-1,8-diyl)bis(piperidine), N,N'-(3,6-dimethyleneoct-4-ene-1,8-diyl)bis(pyrrolidine), 4,4'-(3,6-dimethyleneoct-4-ene-1,8-diyl)bis(1-methylpiperazine), N,N,N'N'-tetramethyl-3,4-dimethylenehexane-1,6-diamine, N,N,N'N'-tetrakis(2-methoxyethyl)-3,4-dimethylenehexane-1,6-diamine, N,N'-dimethyl-N,N'-dibenzyl-3,4-dimethylenehexane-1,6-diamine, N,N'-(3,4-dimethylenehexane-1,6-diyl)bis(morpholine), N,N'-(3,4-dimethylenehexane-1,6-diyl)bis(thiomorpholine), N,N'-(3,4-dimethylenehexane-1,6-diyl)bis(piperidine), N,N'-(3,4-dimethylenehexane-1,6-diyl)bis(pyrrolidine), 4,4'-(3,4-dimethylenehexane-1,6-diyl)bis(1-methylpiperazine), N,N,N'N'-tetramethyl-3,4,5-trimethyleneheptane-1,7-diamine, N,N,N'N'-tetrakis(2-methoxyethyl)-3,4,5-trimethyleneheptane-1,7-diamine, N,N'-dimethyl-N,N'-dibenzyl-3,4,5-trimethyleneheptane-1,7-diamine, N,N'-(3,4,5-trimethyleneheptane-1,7-diyl)bis(morpholine), N,N'-(3,4,5-trimethyleneheptane-1,7-diyl)bis(thiomorpholine), N,N'-(3,4,5-trimethyleneheptane-1,7-diyl)bis(piperidine), N,N'-(3,4,5-trimethyleneheptane-1,7-diyl)bis(pyrrolidine), 4,4'-(3,4,5-trimethyleneheptane-1,7-diyl)bis(1-methylpiperazine), and combinations thereof.

In some embodiments, the ACAMP monomers according to structure (II) may exhibit a k value of exactly 2.

Alternatively, difunctional addition-polymerizable monomer compositions according to the present disclosure can involve amine-derivatized alpha-methyl styrene (ADAMS) monomers according to structure (V) and/or aminated conjugated (non-aromatic) aliphatic methylated polyene (ACAMP) monomers according to structure (VI).

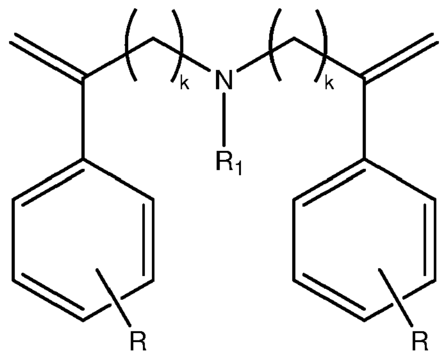

(V)

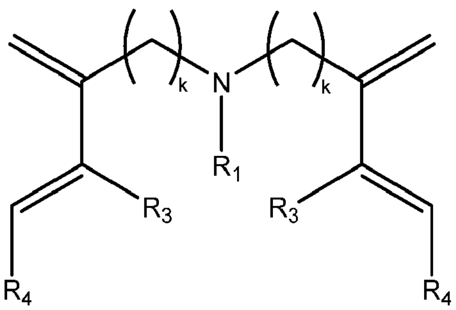

(VI)

wherein each k is an integer from 1 to 3, preferably 2; wherein $R_1$ is a hydrocarbyl group or a hydrocarbonaceous group having 1 to 4 additional heteroatoms, such as O, N, S, P, Se, and combinations thereof; wherein each R, in structure (V), is independently hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group (such as a methyl group), a $C_1$-$C_6$ hydrocarbyl group containing 1 to 4 additional heteroatoms (such as O, N, S, P, Se, and combinations thereof); and wherein each $R_3$ and $R_4$, in structure (VI), is independently hydrogen, a methyl group, or an alkenyl group having structure (IV) above, attached via the asterisk, but specifically which do not collectively achieve aromaticity in combination with the other (conjugated) alkenes in structure (VI), and with the proviso that $R_3$ and $R_4$ are not both alkenyl groups having structure (IV) and are not both methyl groups.

Addition-polymerizable monomer compositions according to the present disclosure may optionally contain initiators and/or co-initiators that are, or may be, used in living or pseudo-living anionic polymerization reactions. Non-limiting examples may include sec-butyllithium, n-butyllithium, t-butyllithium, and the like, and combinations, reaction products, and/or degradation products thereof.

Additionally or alternatively, addition polymerizable monomer compositions according to the present disclosure may optionally contain initiators and/or co-initiators that are, or may be, used in (free) radical polymerization reactions. Non-limiting examples may include, but are not necessarily limited to, azibisisobutyronitrile (AIBN), and the like, and combinations, reaction products, and/or degradation products thereof.

Methods of Making ADAMS/ACAMP Monomers - Aza-Prins Reaction

One method of making monomers according to the present disclosure (of structure (I) or (II), in which the k value is exactly 2) involves an aza-prins reaction scheme. In the case of ADAMS monomers according to structure (I), the starting aromatic reactant for a "monofunctional" product is typically alpha-methylstyrene, alpha-methylnaphthalene, an optionally ring substituted analog thereof, or the like, along with a source of formaldehyde and a source of a secondary amine. "Difunctional" product may involve the aromatic reactant being difunctional, *e.g.,* diisopropenylbenzene, diisopropenylnaphthalene, an optionally ring substituted analog thereof, or the like, or may involve the secondary amine being difunctional, *e.g.*, piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or the like, but not both. In the case of ACAMP monomers according to structure (II), the starting conjugated (non-aromatic) reactant for a "monofunctional" product is typically isoprene (2-methyl-1,3-butadiene), 2-methyl-1,3,5-hexatriene, or 2-methyl-3-methylenepenta-1,4-diene, or the like, along with a source of formaldehyde and a source of a secondary amine. "Difunctional" product may involve the conjugated (non-aromatic) reactant being difunctional, *e.g.,* 2,5-dimethyl-1,3,5-hexatriene, 2,4-dimethyl-3-methylenepenta-1,4-diene, 2,3-dimethylbuta-1,3-diene, or the like, or (as with the ADAMS monomers above) may involve the secondary amine being difunctional, but not both. Thus, in either ADAMS or ACAMP situations, difunctional reactant plus difunctional amine can result in multiplicative chain extension (effective "oligomerization" or "polymerization"), which in many embodiments may be undesirable.

The source of formaldehyde can simply be formaldehyde itself. However, because formaldehyde can be difficult to handle at certain useful reaction temperatures and/or pressures, formaldehyde may alternatively be brought into the reaction as a higher molecular weight compound that can react (or decompose) *in situ* to form formaldehyde. Non-limiting examples of such formaldehyde sources can include paraformaldehyde, trioxane, or the like, or combinations thereof, optionally in tandem with formaldehyde itself.

The source of secondary amine can likewise simply be the secondary amine itself. However, for certain (typically lower molecular weight) secondary amines such as dimethylamine, for example (which, like formaldehyde, can be difficult to handle at certain useful reaction temperatures and/or pressures), secondary amine(s) may alternatively be brought into the reaction as a conjugate that may represent a partial or complete reaction with formaldehyde (or a formaldehyde source). One non-limiting example of such a secondary amine-formaldehyde conjugate is N,N,N',N'-tetramethyl-diaminomethane, which effectively brings ~2 equivalents of dimethylamine reacted with ~1 equivalent of formaldehyde - because of the mismatch in equivalents of formaldehyde and secondary amine, such a conjugate will typically still require additional source of the underrepresented reactant (in this case, formaldehyde or a substitute).

In these aza-prins reactions, the reaction medium can be important to attaining an appreciably high content of desired (*i.e.,* exo isomer) product with appreciably low contents of undesirable (*i.e.*, saturation byproduct and/or hydroxylation byproduct) and/or less desired (*e.g.*, endo isomer) (by)products, particularly as it relates to the pH under which the reaction proceeds.

For instance, the pH of the reaction can advantageously be from about 0.8 to about 4.8, *e.g.,* from about 0.8 to about 4.5, from about 0.8 to about 4.0, from about 1.0 to about 4.8, from about 1.0 to about 4.5, from about 1.0 to about 4.0, from about 1.5 to about 4.8, from about 1.5 to about 4.5, from about 1.5 to about 4.0, from about 2.0 to about 4.8, from about 2.0 to about 4.5, or from about 2.0 to about 4.0 (in particular from about 0.8 to about 4.8, from about 1.0 to about 4.5, or from about 2.0 to about 4.0). Without being bound by theory, too low of a reaction pH can promote undesirable and/or irreversible side reactions, such as but not limited to dimerization and/or oligomerization (byproduct) reaction of the aromatic/conjugated reactant, formation of increasing amounts of saturation byproduct, formation of hydroxylation byproduct (such as by hydrolyzation of an acid conjugate intermediate in the path of exo/endo isomer formation), formation of an increasing ratio of endo to exo isomer products, or the like, or combinations thereof. Additionally or alternatively, without being bound by theory, too high of a reaction pH can hinder reaction completion and/or can shift reaction equilibrium backward to reactants from products, such as but not limited to (likely reversible) un-reaction of the iminium cation back to the formaldehyde and secondary amine reactants (or to some conjugate precursor), failing to provide acidic enough conditions to initiate formation of the iminium cation from the formaldehyde and secondary amine reactants, stalling significant completion of the reaction undesirably at the saturated acid conjugate stage and optionally of promoting the undesired hydrolysis reaction to increasingly form hydroxylation byproduct, or the like, or combinations thereof.

To enable an appropriate reaction pH, the reaction medium can advantageously comprise a protic acid with a "moderate" pKa, optionally in combination with one or more acids each with a "strong" pKa. The "moderate" pKa protic acid can, in a preferred embodiment, comprise or be acetic acid. However, examples of other "moderate" pKa protic acids can include, but are not limited to, formic acid, propionic acid, a butanoic acid, a pentanoic acid, a hexanoic acid, benzoic acid, chloroacetic acid, fluoroacetic acid, oxalic acid, malonic acid, maleic acid, fumaric acid, citric acid, or the like, or a combination thereof. If a "strong" pKa acid is used in combination with one or more "moderate" pKa protic

acids, the "strong" pKa acid should not be so low as to push the reaction pH too low. For example, without being bound by theory, sulfuric acid is believed to be too strong, because even a ~9/1 molar ratio of acetic to sulfuric acid seemed to create too low a reaction pH. With similar pKa's, p-toluenesulfonic acid and benzene sulfonic acid are also believed to be too strong. Examples of "strong" pKa acids that might be useful, of course in proportions with "moderate" pKa acids that would allow the pH to remain within an advantageous range such as the one described hereinabove, can include, but may not be limited to, orthophosphoric acid, nitric acid, dichloroacetic acid, difluoroacetic acid, trichloroacetic acid, trifluoroacetic acid, methane sulfonic acid, perchloric acid, or the like, or combinations thereof.

Reaction Scheme 1 below utilizes dimethylamine as the secondary amine source and alpha-methylstyrene as the aromatic reactant, along with the formaldehyde source, in an acetic acid reaction medium. Although this Reaction Scheme is provided in acetic acid with a specific secondary amine and a "monofunctional" aromatic (ADAMS) reactant source, it should be understood that the aza-prins reaction should proceed mechanistically similarly with other acids/mixtures, with other secondary amines, with difunctional reactant sources, and/or with conjugated (non-aromatic/ACAMP) monomers containing a penultimate methyl group.

Reaction Scheme 1.

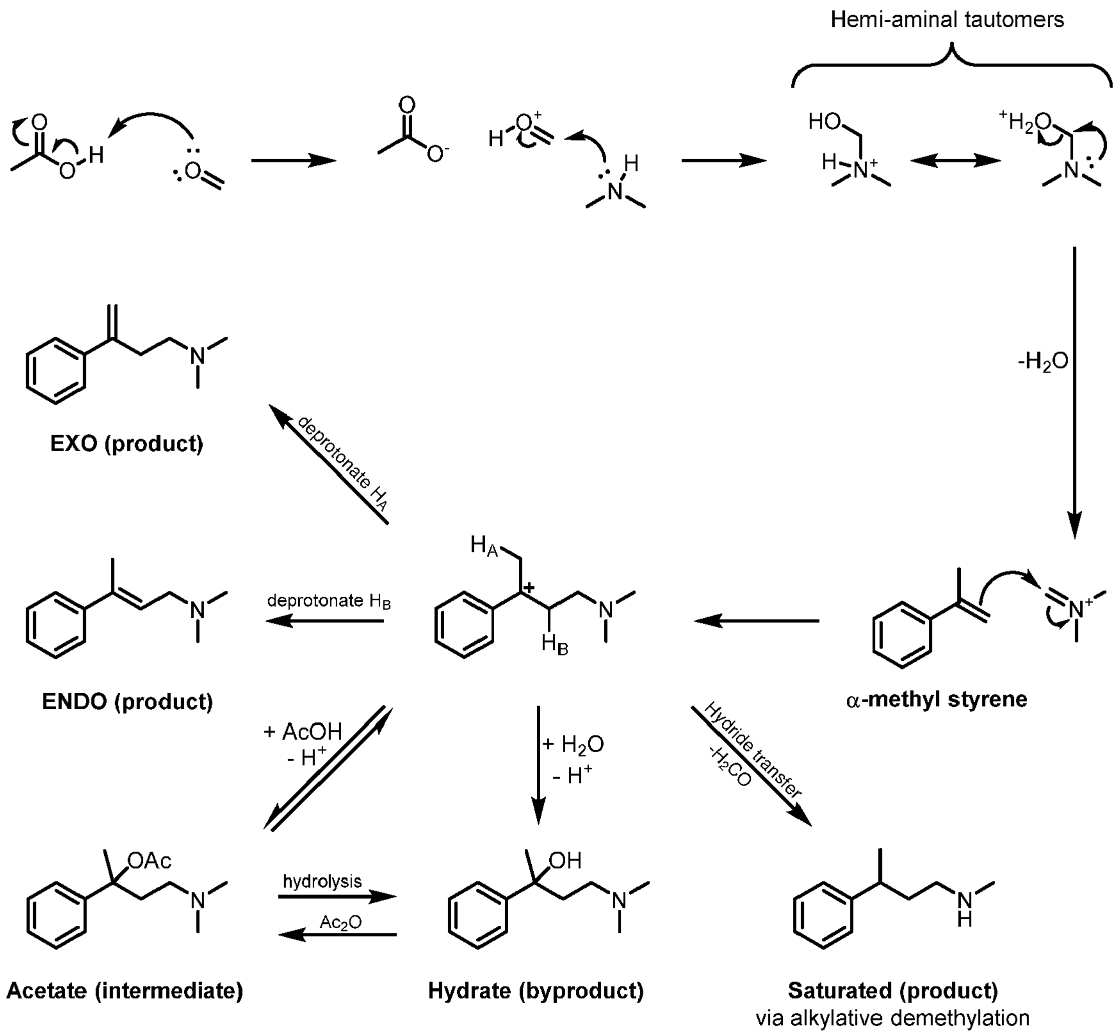


In Reaction Scheme 1, the acetic acid medium facilitates protonation of the formaldehyde, which enables formation of the iminium cation from the secondary amine. The iminium moiety and the vinyl bond of the alpha-methylstyrene then undergo the aza-prins reaction to form a saturated carbocation intermediate (center). That intermediate can desaturate at the terminal carbon nearest the carbocation to form the exo isomer product (upper left) or at the bonded carbon nearest the carbocation to form the endo isomer product (middle left). At favorable reaction conditions, the selection for exo isomer is at least ~2/1 compared to endo isomer. Whether these desaturations mechanistically are non-concerted from the carbocation or concerted from the acetate intermediate (bottom left) is merely semantic. However, under certain conditions, some/considerable amounts of acetate intermediate may remain. Under certain (similar/other)

conditions, the carbocation intermediate (and/or the acetate intermediate) may be hydrolyzed (*e.g.*, from water formed during iminium cation creation and/or present from the reaction medium acid(s) or reactants/sources) to form the hydroxylation byproduct (in this case, the hydrate byproduct; middle bottom). The hydroxylation byproduct is typically not highly reversible (at least not at synthesis conditions that are favorable for high exo isomer content formation). However, introduction of acetic anhydride (shown above) and/or a slight-to-moderate increase in temperature (not shown) can reform the acetate intermediate, which can more easily be encouraged to form exo (or endo) isomer product. Under certain (similar/other) conditions, the carbocation intermediate may undergo hydride transfer from the C-H bonds alpha to the amine functional group (*e.g.*, the N-methyl groups in Reaction Scheme 1), followed by hydrolysis of the resulting iminium ion, to produce saturated byproducts (bottom right) via an alkylative demethylation reaction, such as described in the Cohen article.

Furthermore, as noted above, in the situation regarding difunctional monomers (product) of structure (I) or (II) according to the present disclosure, the aza-prins reaction scheme may have a difunctional secondary amine or a difunctional aromatic (structure (I)) or conjugated/non-aromatic (structure (II)) reactant, but not both (to avoid multiplicative chain extension, as described in the context of the "difunctional" product definition above). The stoichiometry of the exemplary monofunctional aza-prins Reaction Scheme 1 may be easily adapted by the ordinary skilled artisan to accommodate difunctional product.

Nevertheless, in the context of difunctionality of addition-polymerizable groups, with two potential addition-polymerizable loci comes the possibility of a hybrid product containing two different product/byproduct chemistries within the same molecule. Molecules having two "exo" (vinylidene) bonds are obviously considered "exo" isomer products - same with two "endo" (vinylene) bonds being considered "endo" isomer product, two saturation byproduct events being considered saturation byproduct, two hydroxylation byproduct events being considered hydroxylation byproduct, etc. However, in certain situations (analogous to genetics), a hybrid difunctional product can be categorized with a product due to its "dominant" chemistry, or can be categorized with a product due to its "recessive" chemistry. "Exo" isomer product chemistry is considered "dominant" herein, as is hydroxylation product chemistry, but hydroxylation byproduct chemistry is "dominant" over "exo" isomer product chemistry. For example, for difunctional product with two addition-polymerizable loci, a hybrid product containing one hydroxylation byproduct chemistry locus and one "exo" isomer product chemistry locus is categorized herein as being a hydroxylation byproduct. Indeed, a hybrid containing even one hydroxylation byproduct locus is considered a hydroxylation byproduct herein. Also relevant is that "endo" isomer product chemistry is considered "recessive" herein, as is saturation byproduct chemistry, but saturation byproduct chemistry is "recessive" over "endo" isomer product chemistry. Thus, for example, for difunctional product with two addition-polymerizable loci, a hybrid product containing one "endo" isomer product chemistry locus and one saturation byproduct chemistry locus is categorized herein as being an "endo" isomer product. Indeed, only a product containing two saturation byproducts is considered a saturation byproduct herein - a hybrid containing only one saturation byproduct locus is categorized herein by its other locus. Because of the potential reactive nature of the acetate intermediate chemistry in Reaction Scheme 1 to have the acetate leave to form "exo" or "endo" isomer product chemistry and/or to hydrolyze to form hydroxylation byproduct chemistry, the acetate intermediate chemistry is considered to be "neutral" herein, which chemistry is in between "dominant" and "recessive". As such, for example, for difunctional product with two addition-polymerizable loci, a hybrid product containing one "endo" isomer product chemistry locus and one acetate intermediate chemistry locus is categorized herein as being an acetate intermediate, whereas a hybrid product containing one "exo" isomer product chemistry locus and one acetate intermediate chemistry locus is categorized herein as being an "exo" isomer product.

In the alternative situation regarding difunctional monomers (product) of structure (V) or (VI) according to the present disclosure (in which the k value is exactly 2), the aza-prins reaction scheme can be as described above, except that a source of a monofunctional primary amine replaces the source of secondary amine, and monofunctional aromatic or conjugated (non-aromatic) reactants should preferably be used, to the desirable exclusion of corresponding difunctional reactants. Indeed, in such a situation, the dual aza-prins reactivity of the primary amine with more than one monofunctional aromatic or conjugated (non-aromatic) reactant can create difunctionality (without being bound by theory, *e.g.,* the primary amine/iminium may attach to a first aromatic/conjugated reactant to form a secondary amine, which may then form an iminium and attach to a second aromatic/conjugated reactant to form a tertiary amine), while avoiding multiplicative chain extension (described in the context of the "difunctional" product definition above). The same categorization of difunctional monomers (product) of structure (I) or (II) according to the present disclosure applies to these alternative difunctional monomers (product).

The aza-prins reaction conditions can be any under which effective reaction occurs, and preferably under which reaction completion occurs on a reasonable time scale with creation of significant amounts of "exo" isomer product and optionally with small enough amounts of byproducts, particularly with small enough amounts of hydroxylation byproduct and/or saturation byproduct. For example, with respect to ADAMS monomers according to structure (I) (and/or (V)), temperatures can range from ambient (*e.g.,* ~20°C) to elevated (*e.g.,* ~200°C). However, although ambient temperatures may provide some reaction, desired product formation can be extraordinarily slow. Relatedly, although elevated temperatures may provide much faster reaction rates, undesired byproduct formation can be substantially increased. Thus, in certain situations, advantageous sufficient reaction temperatures can range from about 40°C to about 190°C, about 50°C to about 180°C, 60°C to about 170°C, from about 70°C to about 160°C, or from about 80°C to about 150°C. Advantageous sufficient reaction times are dependent on the reaction temperature, and can range from ranging from about 20 minutes to 48 hours, about 40 minutes to 36 hours, or about 1 to 30 hours, or about 2 hours to 30 hours, or 3 to 24 hours, or 4 hours to 20 hours, or 6 to 18 hours.

In situations where aromatic and/or conjugated (non-aromatic) reactants have significant vapor pressures due to lower boiling points, for example (*e.g.,* such as in the case of isoprene or 2,3-dimethylbuta-1,3-diene), such advantageous temperatures may implicate a highly gaseous phase reaction, unless additional pressure is imposed. Similar situations can occur with formaldehyde and/or certain lower molecular weight amines (*e.g.*, dimethylamine, etc.). As such, when application of additional pressure is not desired, higher boiling point formaldehyde sources (such as paraformaldehyde and/or trioxane) and/or amine-formaldehyde conjugate sources (such as partial/complete reaction products) may be used. Nevertheless, in situations where higher than ambient pressures may be necessary or desirable, such pressure can typically depend on that necessary to keep the reactants (and/or reaction media, products, intermediates, etc.) in liquid phase (at sufficiently low vapor pressure to effect suitable reaction). Although sub-atmospheric pressures are possible (as are sub-ambient temperatures), they are rarely desirable - therefore, when pressurization is necessary or desired, the reaction pressure can range from approximately 1.0 bara (atmospheric) to about 31 bara, from about 1.5 bara to about 21 bara, or from about 2.0 bara to about 16 bara.

Although a popular reaction method can involve batch processing, continuous flow reaction processing may alternatively be achieved, such as through scale-up activities.

In some situations, such as those in which acetate intermediate formation may be significant and in which product/byproduct formation (reaction completion) may be lower than desired, an optional additional action that can be taken to decrease acetate intermediate "stalling" (and thus increase ultimate product/byproduct formation) is to increase temperature. As noted above, while a temperature increase may be advisable, it can simultaneously be desirable that the temperature increase not raise too high, so as to balance increased "exo" isomer product formation ("stalling" workaround) while avoiding as much additional "recessive"/"dominant" byproduct formation as possible. Without being bound by theory in such situations, although the chosen temperature increase can depend upon various inputs such as the initial reaction temperature, degree of acetate intermediate "stalling"/degree of reaction completion, and degree of undesired byproduct formation, *inter alia,* a reasonable range of temperature increase can be from about 5°C to about 80°C, from about 10°C to about 60°C, or from about 20°C to about 50°C. In some embodiments, the optional temperature increase may be accomplished in a downstream step from the initial reaction in continuous flow mode. Whether in batch or continuous flow mode, the optional temperature increase can be accomplished for a reasonable/effective amount of time - in accordance with the knowledge of ordinary skilled artisans, the higher the temperature increase, typically the shorter the amount of time is chosen for the appropriate balance between additional "exo" isomer product formation and (undesired) byproduct formation.

Additionally or alternatively, in situations in which hydroxylation byproduct formation may be significant or higher than desired, an optional additional action that can be taken to "reverse" that is to introduce a corresponding acid anhydride (*e.g.*, for reaction media comprising acetic acid, that could be acetic anhydride and/or acetyl chloride/bromide). Without being bound by theory, while the hydrolysis reaction that tends to form hydroxylation byproduct is not believed to be substantially reversible, the introduction of the corresponding acid anhydride is believed to drive replacement of the hydroxyl group with an acetate group, thereby reforming the acetate intermediate (as well as additional acidic reaction medium, if anhydride is used, but a hydrogen halide byproduct if an acid chloride/bromide is used), which can then desirable be driven to desirably form additional "exo" isomer product (and to less desirably form byproducts). This corresponding acid anhydride step can be accomplished at reaction temperature (or less desirably at below reaction temperature, unless reaction temperature is relatively high, in which case that might be desired) or optionally at increased temperatures (as mentioned previously in the acetate intermediate "stalling" context).

Product workup and/or isolation may be necessary/desirable and may involve one or more of phase separation, precipitation, washing, drying, filtration, neutralization, vacuum distillation, fractional distillation, and the like.

Methods of Making ADAMS/ACAMP Monomers - Wittig Reaction

One method of making monomers according to the present disclosure involves a Wittig reaction scheme. Although Wittig reaction schemes allow the k value to be manipulable (*i.e.*, 1, 2, and/or 3) with varied starting materials/reactants, the following discussion is based on a k value of 2 and is meant to be exemplary, not limiting.

In the case of ADAMS monomers according to structure (I), the starting aromatic reactant for a "monofunctional" product is typically a $C_1$-$C_3$ alkyl (corresponding to the k value) optionally substituted phenyl ketone, a $C_1$-$C_3$ alkyl (corresponding to the k value) optionally substituted naphthalenyl ketone, or the like, whose terminal alkyl carbon contains a suitable leaving group (*e.g.,* typically a halogen such a fluoro, chloro, iodo, or bromo group, although other leaving

groups suitable for condensation may be used) and a source of a secondary amine - in the k = 2 case, an example would include 3-chloro-1-phenylpropan-1-one. "Difunctional" product may involve the aromatic reactant being difunctional, *e.g.*, phenylene-1,1'-bis(3-chloropropan-1-one) or the like, or may involve the secondary amine being difunctional, *e.g.,* piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or the like, but not both. In the case of ACAMP monomers according to structure (II), the starting conjugated (non-aromatic) reactant for a "monofunctional" product is typically a $C_1$-$C_3$ alkyl (corresponding to the k value) olefinic (mono- or di- unsaturated) ketone whose terminal alkyl carbon contains a suitable leaving group (*e.g.,* typically a halogen such a fluoro, chloro, iodo, or bromo group, although other leaving groups suitable for condensation may be used) and a source of a secondary amine - in the k = 2 case, examples would include 1-chloropent-5-en-3-one, 1-chlorohepta-4,6-dien-3-one, 1-chloro-4-methylenehex-5-en-3-one, or the like. "Difunctional" product may involve the conjugated (non-aromatic) reactant being difunctional, *e.g.,* 1,8-dichlorooct-4-ene-3,6-dione, 1,7-dichloro-4-methyleneheptane-3,5-dione, 1,6-dichlorohexane-3,4-dione, or the like, or (as with the ADAMS monomers above) may involve the secondary amine being difunctional, but not both. Thus, in either ADAMS or ACAMP situations, difunctional reactant plus difunctional amine can result in multiplicative chain extension (effective "oligomerization" or "polymerization"), which in many embodiments may be undesirable.

Reaction Scheme 2 below utilizes methylpiperazine as the secondary amine source and 3-chloro-1-phenylpropan-1-one as the aromatic ketone reactant under basic conditions. Although this Reaction Scheme is provided with a specific secondary amine and a "monofunctional" aromatic (ADAMS) reactant source, it should be understood that the Wittig reaction scheme should proceed mechanistically similarly with other secondary amines, with difunctional reactant sources, and/or with conjugated (non-aromatic/ACAMP) reactants containing an olefinic alkyl ketone with an appropriate leaving group.

Reaction Scheme 2.

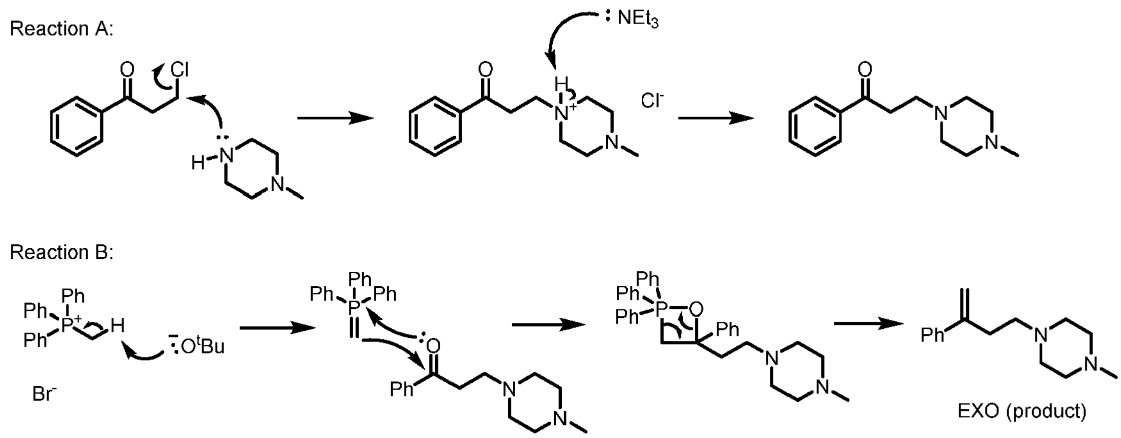


The initial condensation reaction of the functional ketone and the secondary amine is typically done under basic conditions (which may include hydroxide-based, ionic/basic, and/or Lewis basic reaction media such as sodium hydroxide, potassium carbonate, and/or triethylamine, or the like, for example). With a chloro leaving group, the condensation product in Reaction Scheme 2 is HCl, which the basic conditions are designed to sufficiently buffer.

The initial condensation reaction can also be done in cases where the ketone reactant does not include a leaving group on the terminal carbon, if instead a source of formaldehyde (*e.g.* formaldehyde, trioxane, paraformaldehyde, or a combination thereof) is added and the reaction is run under acidic conditions instead (for example, but not limited to hydrochloric acid), for example under Mannich Reaction conditions.

The subsequent Wittig reaction utilizes an ylide precursor reactant, such as a methyltriphenylphosphonium halide (*e.g.*, bromide, iodide, chloride, or the like, but typically not fluoride), with the aminated ketone condensation product under strong basic conditions (*e.g.*, using sodium and/or potassium t-butoxide, or the like), and optionally in an organic solvent medium (*e.g.*, acetonitrile, tetrahydrofuran, dimethylformamide, dimethylsulfoxide, or the like, or a combination thereof). The ylide effectively replaces the ketone carbonyl oxygen with a methylene carbon to form an "exo" (vinylidene) bond, forming triphenylphosphine oxide as a byproduct, as shown above. Because the Wittig reaction begins with the ketone carbonyl, the "exo" isomer product is by far the primary product, with the traditional aza-prins hydroxylation and saturation byproducts being largely (wholly) absent. Indeed, although "endo" isomer product is not made directly, certain highly basic conditions and/or higher reaction temperatures may enable isomerization of the "exo" isomer product to form some tangible amount of "endo" isomer product.

Furthermore, as noted above, in the situation regarding difunctional monomers (product) of structure (I) or (II) according to the present disclosure, the Wittig reaction scheme may have a difunctional secondary amine or a difunctional aromatic ketone (structure (I)) or conjugated/non-aromatic ketone (structure (II)) reactant, but not both (to avoid multiplicative chain extension, as described in the context of the "difunctional" product definition above). The stoichiometry of the exemplary monofunctional Wittig Reaction Scheme 2 may be easily adapted by the ordinary skilled artisan to accommodate difunctional product.

Nevertheless, in the context of difunctionality of addition-polymerizable groups, with two potential addition-polymerizable loci comes the possibility of a hybrid product containing two different product/byproduct chemistries within the same molecule. Although this may be unlikely, the same rules apply to hybrid products as detailed above in the aza-prins reaction scheme. Molecules having two "exo" (vinylidene) bonds are obviously considered "exo" isomer products, and molecules having two "endo" (vinylene) bonds are considered "endo" isomer product. As above, for difunctional product with two addition-polymerizable loci, a hybrid product containing one "exo" isomer product chemistry locus and one "endo" isomer chemistry locus is categorized herein as being an "exo" isomer product.

In the alternative situation regarding difunctional monomers (product) of structure (V) or (VI) according to the present disclosure (in which the k value is exactly 2), the Wittig reaction scheme can be as described above, except that a source of a monofunctional primary amine replaces the source of secondary amine, and monofunctional aromatic or conjugated (non-aromatic) ketone reactants should preferably be used, to the desirable exclusion of corresponding difunctional reactants. Indeed, in such a situation, the dual Wittig reactivity of the primary amine with more than one monofunctional aromatic or conjugated (non-aromatic) ketone reactant can create difunctionality, while avoiding multiplicative chain extension (described in the context of the "difunctional" product definition above). The same categorization of difunctional monomers (product) of structure (I) or (II) according to the present disclosure applies to these alternative difunctional monomers (product).

The Wittig reaction conditions can be any under which effective reaction occurs, and preferably under which reaction completion occurs on a reasonable time scale with creation of significant amounts of "exo" isomer product and optionally with small enough amounts of byproducts. For example, with respect to ADAMS monomers according to structure (I) (and/or (V)), temperatures can range from cryogenic (e.g., ~ -78°C) to elevated (e.g., ~200°C). Sufficient reaction temperature for the reactions of this disclosure is a temperature ranging from -78°C to 200°C, or -50°C to 180°C, or -20°C to 150°C, 0°C to 120°C, or 20°C to 100°C, or -40°C to 80°C, -10°C to 60°C, or 0°C to 25°C. However, although cryogenic temperatures may provide some reaction, desired product formation can be extraordinarily slow. Sufficient reaction times for the reactions of this disclosure is a time ranging from 20 minutes to 48 hours, 40 minutes to 36 hours, or 1 to 30 hours, or 2 hours to 30 hours, or 3 to 24 hours, or 4 hours to 20 hours, or 6 to 18 hours.

Although a popular reaction method can involve batch processing, continuous flow reaction processing may alternatively be achieved, such as through scale-up activities.

Product workup and/or isolation may be necessary/desirable and may involve one or more of phase separation, precipitation, washing, drying, filtration, neutralization, vacuum distillation, fractional distillation, column chromatography, and the like. Indeed, triphenylphosphine oxide byproduct can often be difficult/costly to remove, without losing significant "exo" isomer product yield.

## Additional Embodiments

Additionally or alternatively, the present disclosure may include one or more of the following embodiments.

Embodiment 1. An addition-polymerizable monomer composition comprising an amine-derivatized alpha-methyl styrene (ADAMS) monomer according to structure (I) and/or an aminated conjugated aliphatic methylated polyene (ACAMP) monomer according to structure (II):

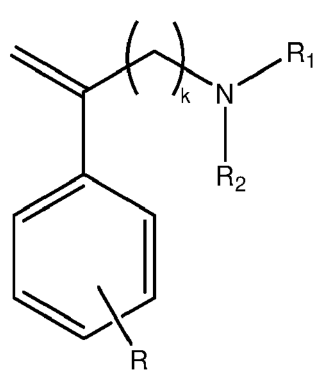

(I)

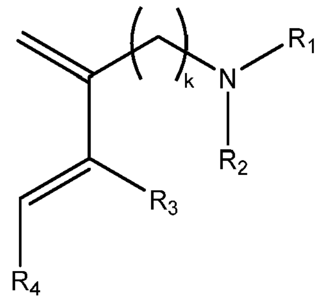

(II)

wherein:

k is an integer from 1 to 3, preferably 2;

$R_1$ and $R_2$ are each independently a hydrocarbyl group or a hydrocarbonaceous group having 1 to 4 additional heteroatoms selected from the group consisting of O, N, S, P, Se, and combinations thereof, or wherein $R_1$ and $R_2$ are connected to form a moiety containing at least one 5- to 12- membered ring, from 3 to 28 carbons, and optionally 1 to 6 additional heteroatoms selected from the group consisting of O, N, S, P, Se, and combinations thereof;

in structure (I), R is hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group, a $C_1$-$C_6$ hydrocarbyl group containing 1 to 4 additional heteroatoms selected from the group consisting of O, N, S, P, Se, and combinations thereof, or a second amino-functional group having structure (III)

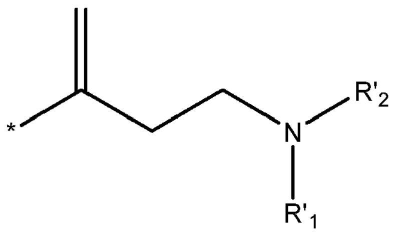

(III)

attached via the asterisk, with $R'_1$ and $R'_2$ being independently the same or different as $R_1$ and $R_2$ but defined identically; and

in structure (II), $R_3$ and $R_4$ are each independently hydrogen, a methyl group, a second amino-functional group having structure (III) attached via the asterisk, with $R'_1$ and $R'_2$ being independently the same or different as $R_1$ and $R_2$ but defined identically, or an alkenyl group having structure (IV)

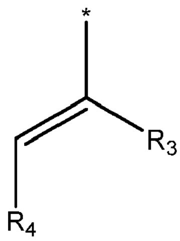

(IV)

attached via the asterisk, but specifically which do not collectively achieve aromaticity in combination with the other alkenes in structure (II), and with the proviso that $R_3$ and $R_4$ are not both alkenyl groups having structure (IV) and are not both methyl groups.

Embodiment 2. The addition-polymerizable monomer composition of embodiment 1, wherein the ADAMS monomer according to structure (I) comprises 1-dimethylamino-3-phenylbut-3-ene, 1-diethylamino-3-phenylbut-3-ene, 1-di-n-propylamino-3-phenylbut-3-ene, 1-diisopropylamino-3-phenylbut-3-ene, 1-di-2-propenylamino-3-phenylbut-3-ene, 1-di-n-butylamino-3-phenylbut-3-ene, 1-di-sec-butylamino-3-phenylbut-3-ene, 1-diisobutylamino-3-phenylbut-3-ene, 1-di-tert-butylamino-3-phenylbut-3-ene, 1-cyclohexylmethylamino-3-phenylbut-3-ene, 1-dicyclohexylamino-3-phenylbut-3-ene, 1-di-(2-ethylhexyl)amino-3-phenylbut-3-ene, 1-di-(methoxyethyl)amino-3-phenylbut-3-ene, 1-di-(ethoxyethyl)amino-3-phenylbut-3-ene, 1-di-(phenoxyethyl)amino-3-phenylbut-3-ene, 1-di-(methylthioethyl)amino-3-phenylbut-3-ene, 1-di-(ethylthioethyl)amino-3-phenylbut-3-ene, 1-benzylmethylamino-3-phenylbut-3-ene, 1-dibenzylamino-3-phenylbut-3-ene, 1-benzylphenylamino-3-phenylbut-3-ene, 1-diphenylamino-3-phenylbut-3-ene, 1-dipyridylamino-3-phenylbut-3-ene, 1-phenylmethylamino-3-phenylbut-3-ene, 1-phenylmethoxyethylamino-3-phenylbut-3-ene, 1-benzylmethoxyethylamino-3-phenylbut-3-ene, 1-(N-morpholinyl)-3-phenylbut-3-ene, 1-(N-thiomorpholinyl)-3-phenylbut-3-ene, 1-(N-piperidinyl)-3-phenylbut-3-ene, 1-(N-piperazinyl)-3-phenylbut-3-ene, 1-(N-diazepanyl)-3-phenylbut-3-ene, 1-(N-pyrrolidinyl)-3-phenylbut-3-ene, 1-(N-pyrrolyl)-3-phenylbut-3-ene, 1-(1,2,3,4-tetrahydro-1-quinolinyl)-3-phenylbut-3-ene, 1-(1,2,3,4-tetrahydro-2-isoquinolinyl)-3-phenylbut-3-ene, 1-(N-indolinyl)-3-phenylbut-3-ene, 1-(N-indolyl)-3-phenylbut-3-ene, 1-(N-carbazolyl)-3-phenylbut-3-ene, 1-(N-phenothiazinyl)-3-phenylbut-3-ene, 1-(N-phenothiazinyl-S-oxide)-3-phenylbut-3-ene, 1-(N-phenothiazinyl-S,S-dioxide)-3-phenylbut-3-ene, 1-(N-phenoxazinyl)-3-phenylbut-3-ene, 1-(4-methyl-1-piperazinyl)-3-phenylbut-3-ene, 1-(5-methyl-2,5-diazabicyclo[2.2.1]heptan-2-yl)-3-phenylbut-3-ene, 1-(5-methyl-2,5-diazabicyclo[2.2.2]octan-2-yl)-3-phenylbut-3-ene, 1-(4-cyclopentyl-1-piperazinyl)-3-phenylbut-3-ene, 1-(4-cyclopentadienyl-1-piperazinyl)-3-phenylbut-3-ene, 1-(4-phenyl-1-piperazinyl)-3-phenylbut-3-ene, 1-(4-(thiazolyl)-1-piperazinyl)-3-phenylbut-3-ene, 1-(4-(thiadiazolyl)-1-piperazinyl)-3-phenylbut-3-ene, 1-(4-(triazolyl)-1-piperazinyl)-3-phenylbut-3-ene, 1-(4-(1,2,3-benzotriazolyl)-1-piperazinyl)-3-phenylbut-3-ene, 1-(N'-methyl-N-diazepanyl)-3-phenylbut-3-ene, N,N'-bis(3-phenylbut-3-enyl)diazepane, N,N'-bis(3-phenylbut-3-enyl)piperazine, N,N'-bis(3-phenylbut-3-enyl)dihydrophenazine, N,N'-bis(3-phenylbut-3-enyl)dihydrobenzoindazole, N,N'-bis(3-phenylbut-3-enyl)dihydropermidine, N,N'-bis(3-phenylbut-3-enyl)octahydropyridoquinoline, N,N'-bis(3-phenylbut-3-enyl)octahydropyridoisoquinoline, N,N'-bis(3-phenylbut-3-enyl)hexahydropyrroloquinoline, N,N'-bis(3-phenylbut-3-enyl)hexahydropyrroloisoquinoline, N,N'-bis(3-phenylbut-3-enyl)hexahydropyrroloisoindole, N,N'-bis(3-phenylbut-3-enyl)diazabicyclo[2.2.1]heptane, N,N' -bis(3 -phenylbut-3 - enyl)diazabicyclo [2.2.2] octane, 1,3-bis(1-(3 -phenylbut-3 -enyl)piperidin-4-yl)propane, bis(1-dimethylamino-3-phenylbut-3-enyl)benzene, bis(1-benzylmethylamino-3-phenylbut-3-enyl)benzene, bis(1-(N-morpholinyl)-3-phenylbut-3-enyl)benzene, bis(1-(N-thiomorpholinyl)-3-phenylbut-3-enyl)benzene, bis(1-(di-methoxyethyl)amino-3-phenylbut-3-enyl)benzene, bis(1-(N-piperidinyl)-3-phenylbut-3-enyl)benzene, bis(1-(N-pyrrolidinyl)-3-phenylbut-3-enyl)benzene, bis(1-(4-methyl-1-piperazinyl))-3-phenylbut-3-enyl)benzene, or a combination thereof.

Embodiment 3. The addition-polymerizable monomer composition of embodiment 1 or embodiment 2, comprising substantially none of 1-dimethylamino-3-phenylbut-3-ene, 1-diethylamino-3-phenylbut-3-ene, N-morpholinyl-3-phenylbut-3-ene, 1-benzylmethylamino-3-phenylbut-3-ene, 1-dimethylamino-3-p-tolylbut-3-ene, 1-diethylamino-3-p-tolylbut-3-ene, N-morpholinyl-3-p-tolylbut-3-ene, N-piperidinyl-3-p-tolylbut-3-ene, and N-pyrrolidinyl-3-p-tolylbut-3-ene.

Embodiment 4. The addition-polymerizable monomer composition of embodiment 1 or embodiment 2, comprising substantially no compounds in which $R_1$ and $R_2$ are each independently a benzyl group or a $C_1$-$C_{12}$ alkyl group, and comprising substantially no compounds in which $R_1$ and $R_2$ are connected to form a singular unsubstituted nitrogen-containing 5- or 6- membered heterocyclic ring.

Embodiment 5. The addition-polymerizable monomer composition of any one of embodiments 1-4, wherein an ADAMS monomer according to structure (I) comprises an exo isomer monomer, further comprising one or more of the following:

(1) at least about 0.5 wt%, based on the reaction medium-free composition weight, of endo isomer monomer corresponding to the exo isomer monomer, optionally up to about 15 wt%;
(2) not more than about 12 wt%, based on the reaction medium-free composition weight, of saturation byproduct;
(3) at least about 100 wppm, based on the reaction medium-free composition weight, of optionally ring substituted alpha-methylstyrene, optionally up to about 5 wt%;
(4) not more than about 500 wppm, based on the reaction medium-free composition weight, of hydroxylation byproduct; and
(5) not more than about 1.0 wt%, based on the reaction medium-free composition weight, of dimerization/oligomerization byproduct.

Embodiment 6. The addition-polymerizable monomer composition of any one of embodiments 1-5, further comprising not more than about 0.3 wt%, based on the reaction medium-free composition weight, of an acid anhydride.

Embodiment 7. The addition-polymerizable monomer composition of embodiment 1, wherein the ACAMP monomer according to structure (II) comprises 1-dimethylamino-3-methylenepent-4-ene, 1-diethylamino-3-methylenepent-4-ene, 1-din-propylamino-3-methylenepent-4-ene, 1-diisopropylamino-3-methylenepent-4-ene, 1-di-2-propenylamino-3-methylenepent-4-ene, 1-di-n-butylamino-3-methylenepent-4-ene, 1-di-sec-butylamino-3-methylenepent-4-ene, 1-diisobutylamino-3-methylenepent-4-ene, 1-di-tert-butylamino-3-methylenepent-4-ene, 1-cyclohexylmethylamino-3-methylenepent-4-ene, 1-dicyclohexylamino-3-methylenepent-4-ene, 1-di-(2-ethylhexyl)amino-3-methylenepent-4-ene, 1-di-(methoxyethyl)amino-3-methylenepent-4-ene, 1-di-(ethoxyethyl)amino-3-methylenepent-4-ene, 1-di-(phenoxyethyl)amino-3-methylenepent-4-ene, 1-di-(methylthioethyl)amino-3-methylenepent-4-ene, 1-di-(ethylthioethyl)amino-3-methylenepent-4-ene, 1-benzylmethylamino-3-methylenepent-4-ene, 1-dibenzylamino-3-methylenepent-4-ene, 1-benzylphenylamino-3-methylenepent-4-ene, 1-diphenylamino-3-methylenepent-4-ene, 1-dipyridylamino-3-methylenepent-4-ene, 1-phenylmethylamino-3-methylenepent-4-ene, 1-phenylmethoxyethylamino-3-methylenepent-4-ene, 1-benzylmethoxyethylamino-3-methylenepent-4-ene, 1-(N-morpholinyl)-3-methylenepent-4-ene, 1-(N-thiomorpholinyl)-3-methylenepent-4-ene, 1-(N-piperidinyl)-3-methylenepent-4-ene, 1-(N-piperazinyl)-3-methylenepent-4-ene, 1-(N-diazepanyl)-3-methylenepent-4-ene, 1-(N-pyrrolidinyl)-3-methylenepent-4-ene, 1-(N-pyrrolyl)-3-methylenepent-4-ene, 1-(1,2,3,4-tetrahydro-1-quinolinyl)-3-methylenepent-4-ene, 1-(1,2,3,4-tetrahydro-2-isoquinolinyl)-3-methylenepent-4-ene, 1-(N-indolinyl)-3-methylenepent-4-ene, 1-(N-indolyl)-3-methylenepent-4-ene, 1-(N-carbazolyl)-3-methylenepent-4-ene, 1-(N-phenothiazinyl)-3-methylenepent-4-ene, 1-(N-phenothiazinyl-S-oxide)-3-methylenepent-4-ene, 1-(N-phenothiazinyl-S,S-dioxide)-3-methylenepent-4-ene, 1-(N-phenoxazinyl)-3-methylenepent-4-ene, 1-(4-methyl-1-piperazinyl)-3-methylenepent-4-ene, 1-(5-methyl-2,5-diazabicyclo[2.2.1]heptan-2-yl)-3-methylenepent-4-ene, 1-(5-methyl-2,5-diazabicyclo[2.2.2]octan-2-yl)-3-methylenepent-4-ene, 1-(4-cyclopentyl-1-piperazinyl)-3-methylenepent-4-ene, 1-(4-cyclopentadienyl-1-piperazinyl)-3-methylenepent-4-ene, 1-(4-phenyl-1-piperazinyl)-3-methylenepent-4-ene, 1-(4-(thiazolyl)-1-piperazinyl)-3-methylenepent-4-ene, 1-(4-(thiadiazolyl)-1-piperazinyl)-3-methylenepent-4-ene, 1-(4-(triazolyl)-1-piperazinyl)-3-methylenepent-4-ene, 1-(4-(1,2,3-benzotriazolyl)-1-piperazinyl)-3-methylenepent-4-ene, 1-(N'-methyl-N-diazepanyl)-3-methylenepent-4-ene, 1-dimethylamino-3-methylenehepta-4,6-diene, 1-diethylamino-3-methylenehepta-4,6-diene, 1-di-n-propylamino-3-methylenehepta-4,6-diene, 1-diisopropylamino-3-methylenehepta-4,6-diene, 1-di-2-propenylamino-3-methylenehepta-4,6-diene, 1-di-n-butylamino-3-methylenehepta-4,6-diene, 1-di-sec-butylamino-3-methylenehepta-4,6-diene, 1-diisobutylamino-3-methylenehepta-4,6-diene, 1-di-tert-butylamino-3-methylenehepta-4,6-diene, 1-cyclohexylmethylamino-3-methylenehepta-4,6-diene, 1-dicyclohexylamino-3-methylenehepta-4,6-diene, 1-di-(2-ethylhexyl)amino-3-methylenehepta-4,6-diene, 1-di-(methoxyethyl)amino-3-methylenehepta-4,6-diene, 1-di-(ethoxyethyl)amino-3-methylenehepta-4,6-diene, 1-di-(phenoxyethyl)amino-3-methylenehepta-4,6-diene, 1-di-(methylthioethyl)amino-3-methylenehepta-4,6-diene, 1-di-(ethylthioethyl)amino-3-methylenehepta-4,6-diene, 1-benzylmethylamino-3-methylenehepta-4,6-diene, 1-dibenzylamino-3-methylenehepta-4,6-diene, 1-benzylphenylamino-3-methylenehepta-4,6-diene, 1-diphenylamino-3-methylenehepta-4,6-diene, 1-dipyridylamino-3-methylenehepta-4,6-diene, 1-phenylmethylamino-3-methylenehepta-4,6-diene, 1-phenylmethoxyethylamino-3-methylenehepta-4,6-diene, 1-benzylmethoxyethylamino-3-methylenehepta-4,6-diene, 1-(N-morpholinyl)-3-methylenehepta-4,6-diene, 1-(N-thiomorpholinyl)-3-methylenehepta-4,6-diene, 1-(N-piperidinyl)-3-methylenehepta-4,6-diene, 1-(N-piperazinyl)-3-methylenehepta-4,6-diene, 1-(N-diazepanyl)-3-methylenehepta-4,6-diene, 1-(N-pyrrolidinyl)-3-methylenehepta-4,6-diene, 1-(N-pyrrolyl)-3-methylenehepta-4,6-diene, 1-(1,2,3,4-tetrahydro-1-quinolinyl)-3-methylenehepta-4,6-diene, 1-(1,2,3,4-tetrahydro-2-isoquinolinyl)-3-methylenehepta-4,6-diene, 1-(N-indolinyl)-3-methylenehepta-4,6-diene, 1-(N-indolyl)-3-methylenehepta-4,6-diene, 1-(N-carbazolyl)-3-methylenehepta-4,6-diene, 1-(N-phenothiazinyl)-3-methylenehepta-4,6-diene, 1-(N-phenothiazinyl-S-oxide)-3-methylenehepta-4,6-diene, 1-(N-phenothiazinyl-S,S-dioxide)-3-methylenehepta-4,6-diene, 1-(N-phenoxazinyl)-3-methylenehepta-4,6-diene, 1-(4-methyl-1-piperazinyl)-3-methylenehepta-4,6-diene, 1-(5-methyl-2,5-diazabicyclo[2.2.1]heptan-2-yl)-3-methylenehepta-4,6-diene, 1-(5-methyl-2,5-diazabicyclo[2.2.2]octan-2-yl)-3-methylenehepta-4,6-diene, 1-(4-cyclopentyl-1-piperazinyl)-3-methylenehepta-4,6-diene, 1-(4-cyclopentadienyl-1-piperazinyl)-3-methylenehepta-4,6-diene, 1-(4-phenyl-1-piperazinyl)-3-methylenehepta-4,6-diene, 1-(4-(thiazolyl)-1-piperazinyl)-3-methylenehepta-4,6-diene, 1-(4-(thiadiazolyl)-1-piperazinyl)-3-methylenehepta-4,6-diene, 1-(4-(triazolyl)-1-piperazinyl)-3-methylenehepta-4,6-diene, 1-(4-(1,2,3-benzotriazolyl)-1-piperazinyl)-3-methylenehepta-4,6-diene, 1-(N'-methyl-N-diazepanyl)-3-methylenehepta-4,6-diene, 1-dimethylamino-3,4-dimethylenehex-5-ene, 1-diethylamino-3,4-dimethylenehex-5-ene, 1-di-n-propylamino-3,4-dimethylenehex-5-ene, 1-diisopropylamino-3,4-dimethylenehex-5-ene, 1-di-2-propenylamino-3,4-dimethylenehex-5-ene, 1-di-n-butylamino-3,4-dimethylenehex-5-ene, 1-di-sec-butylamino-3,4-dimethylenehex-5-ene, 1-diisobutylamino-3,4-dimethylenehex-5-ene, 1-di-tert-butylamino-3,4-dimethylenehex-5-ene, 1-cyclohexylmethylamino-3,4-dimethylenehex-5-ene, 1-dicyclohexylamino-3,4-dimethylenehex-5-ene, 1-di-(2-ethylhexyl)amino-3,4-dimethylenehex-5-ene, 1-di-(methoxyethyl)amino-3,4-dimethylenehex-5-ene, 1-di-(ethoxyethyl)amino-3,4-dimethylenehex-5-ene, 1-di-(phenoxyethyl)amino-3,4-dimethylenehex-5-ene, 1-di-(methylthioethyl)amino-3,4-dimethylenehex-5-ene, 1-di-(ethylthioethyl)amino-3,4-dimethylenehex-5-ene, 1-benzylmethylamino-3,4-dimethylenehex-5-ene, 1-dibenzylamino-3,4-dimethylenehex-5-ene, 1-benzylphenylamino-3,4-dimethylenehex-5-ene, 1-diphenylamino-3,4-dimethylenehex-5-ene, 1-dipyridylamino-3,4-dimethylenehex-5-ene, 1-phenylmethylamino-3,4-dimethylenehex-5-ene, 1-phenylmethoxyethylamino-3,4-dimethylenehex-5-ene, 1-benzylmethoxyethylamino-3,4-dimethylenehex-5-ene, 1-(N-morpholinyl)-3,4-dimethylenehex-5-ene, 1-(N-thiomorpholinyl)-3,4-dimethylenehex-5-ene, 1-(N-piperidinyl)-3,4-dimethylenehex-5-ene, 1-(N-piperazinyl)-3,4-dimethylenehex-5-ene, 1-(N-diazepanyl)-3,4-dimethylenehex-5-ene, 1-(N-pyrrolidinyl)-3,4-dimethylenehex-5-ene, 1-(N-pyrrolyl)-3,4-dimethylenehex-5-ene, 1-(1,2,3,4-tetrahydro-1-quinolinyl)-3,4-dimethylenehex-5-ene, 1-(1,2,3,4-tetrahydro-2-isoquinolinyl)-3,4-dimethylenehex-5-ene, 1-(N-indolinyl)-3,4-dimethylenehex-5-ene, 1-(N-indolyl)-3,4-dimethylenehex-5-ene, 1-(N-carbazolyl)-3,4-dimethylenehex-5-ene, 1-(N-phenothiazinyl)-3,4-dimethylenehex-5-ene, 1-(N-phenothiazinyl-S-oxide)-3,4-dimethylenehex-5-ene, 1-(N-phenothiazinyl-S,S-dioxide)-3,4-dimethylenehex-5-ene, 1-(N-phenoxazinyl)-3,4-dimethylenehex-5-ene, 1-(4-methyl-1-piperazinyl)-3,4-dimethylenehex-5-ene, 1-(5-methyl-2,5-diazabicyclo[2.2.1]heptan-2-yl)-3,4-dimethylenehex-5-ene, 1-(5-methyl-2,5-diazabicyclo[2.2.2]octan-2-yl)-3,4-dimethylenehex-5-ene, 1-(4-cyclopentyl-1-piperazinyl)-3,4-dimethylenehex-5-ene, 1-(4-cyclopentadienyl-1-piperazinyl)-3,4-dimethylenehex-5-ene, 1-(4-phenyl-1-piperazinyl)-3,4-dimethylenehex-5-ene, 1-(4-(thiazolyl)-1-piperazinyl)-3,4-dimethylenehex-5-ene, 1-(4-(thiadiazolyl)-1-piperazinyl)-3,4-dimethylenehex-5-ene, 1-(4-(triazolyl)-1-piperazinyl)-3,4-dimethylenehex-5-ene, 1-(4-(1,2,3-benzotriazolyl)-1-piperazinyl)-3,4-dimethylenehex-5-ene, 1-(N'-methyl-N-diazepanyl)-3,4-dimethylenehex-5-ene, N,N'-bis(3-methylenepent-4-enyl)diazepane, N,N'-bis(3-methylenepent-4-enyl)piperazine, N,N'-bis(3-methylenepent-4-enyl)dihydrophenazine, N,N'-bis(3-methylenepent-4-enyl)dihydrobenzoindazole, N,N'-bis(3-methylenepent-4-enyl)dihydropermidine, N,N'-bis(3-methylenepent-4-enyl)octahydropyridoquinoline, N,N'-bis(3-methylenepent-4-enyl)octahydropyridoisoquinoline, N,N'-bis(3-methylenepent-4-enyl)hexahydropyrroloquinoline, N,N'-bis(3-methylenepent-4-enyl)hexahydropyrroloisoquinoline, N,N' -bis(3-methylenepent-4-enyl)hexahydropyrroloisoindole, N,N'-bis(3-methylenepent-4-enyl)diazabicyclo[2.2.1]heptane, N,N'-bis(3-methylenepent-4-enyl)diazabicyclo [2.2.2] octane, 1,3-bis(1-(3 -methylenepent-4-enyl)piperidin-4-yl)propane, N,N'-bis(3-methylenehepta-4,6-dien-1-yl)diazepane, N,N'-bis(3-methylenehepta-4,6-dien-1-yl)piperazine, N,N'-bis(3-methylenehepta-4,6-dien-1-yl)dihydrophenazine, N,N'-bis(3-methylenehepta-4,6-dien-1-yl)dihydrobenzoindazole, N,N'-bis(3-methylenehepta-4,6-dien-l-yl)dihydropermidine, N,N'-bis(3-methylenehepta-4,6-dien-1-yl)octahydropyridoquinoline, N,N'-bis(3-methylenehepta-4,6-dien-1-yl)octahydropyridoisoquinoline, N,N'-bis(3-methylenehepta-4,6-dien-1-yl)hexahydropyrroloquinoline, N,N'-bis(3-methylenehepta-4,6-dien-1-yl)hexahydropyrroloisoquinoline, N,N'-bis(3-methylenehepta-4,6-dien-1-yl)hexahydropyrroloisoindole, N,N'-bis(3-methylenehepta-4,6-dien-1-yl)diazabicyclo[2.2.1]heptane, N,N'-bis(3-methylenehepta-4,6-dien-1-yl)diazabicyclo[2.2.2]octane, 1,3-bis(1-(3-methylenehepta-4,6-dienyl)piperidin-4-yl)propane, N,N'-bis(3,4-dimethylenehex-5-en-1-yl)diazepane, N,N'-bis(3,4-dimethylenehex-5-en-1-yl)piperazine, N,N'-bis(3,4-dimethylenehex-5-en-1-yl)dihydrophenazine, N,N'-bis(3,4-dimethylenehex-5-en-1-yl)dihydrobenzoindazole, N,N'-bis(3,4-dimethylenehex-5-en-1-yl)dihydropermidine, N,N'-bis(3,4-dimethylenehex-5-en-1-yl)octahydropyridoquinoline, N,N'-bis(3,4-dimethylenehex-5-en-1-yl)octahydropyridoisoquinoline, N,N' -bis(3,4-dimethylenehex-5-en-1-yl)hexahydropyrroloquinoline, N,N'-bis(3,4-dimethylenehex-5-en-1-yl)hexahydropyrroloisoquinoline, N,N'-bis(3,4-dimethylenehex-5-en-1-yl)hexahydropyrroloisoindole, N,N'-bis(3,4-dimethylenehex-5-en-1-yl)diazabicyclo[2.2.1]heptane, N,N'-bis(3,4-dimethylenehex-5-en-1-yl)diazabicyclo[2.2.2]octane, 1,3-bis(1-(3,4-dimethylenehex-5-enyl)piperidin-4-yl)propane, N,N,N'N'-tetramethyl-3,6-dimethyleneoct-4-ene-1,8-diamine, N,N,N'N'-tetrakis(2-methoxyethyl)-3,6-dimethyleneoct-4-ene-1,8-diamine, N,N'-dimethyl-N,N'-dibenzyl-3,6-dimethyleneoct-4-ene-1,8-diamine, N,N'-(3,6-dimethyleneoct-4-ene-1,8-diyl)bis(morpholine), N,N'-(3,6-dimethyleneoct-4-ene-1,8-diyl)bis(thiomorpholine), N,N'-(3,6-dimethyleneoct-4-ene-1,8-diyl)bis(piperidine), N,N'-(3,6-dimethyleneoct-4-ene-1,8-diyl)bis(pyrrolidine), 4,4'-(3,6-dimethyleneoct-4-ene-1,8-diyl)bis(1-methylpiperazine), N,N,N'N'-tetramethyl-3,4-dimethylenehexane-1,6-diamine, N,N,N'N'-tetrakis(2-methoxyethyl)-3,4-dimethylenehexane-1,6-diamine, N,N'-dimethyl-N,N'-dibenzyl-3,4-dimethylenehexane-1,6-diamine, N,N' -(3,4-dimethylenehexane-1,6-diyl)bis(morpholine), N,N'-(3,4-dimethylenehexane-1,6-diyl)bis(thiomorpholine), N,N'-(3,4-dimethylenehexane-1,6-diyl)bis(piperidine), N,N'-(3,4-dimethylenehexane-1,6-diyl)bis(pyrrolidine), 4,4'-(3,4-dimethylenehexane- 1,6-diyl)bis(1-methylpiperazine), N,N,N'N'-tetramethyl-3,4,5-trimethyleneheptane-1,7-diamine, N,N,N'N'-tetrakis(2-methoxyethyl)-3,4,5-trimethyleneheptane-1,7-diamine, N,N'-dimethyl-N,N'-dibenzyl-3,4,5-trimethyleneheptane-1,7-diamine, N,N'-(3,4,5-trimethyleneheptane-1,7-diyl)bis(morpholine), N,N'-(3,4,5-trimethyleneheptane-1,7-diyl)bis(thiomorpholine), N,N'-(3,4,5-trimethyleneheptane-1,7-diyl)bis(piperidine), N,N'-(3,4,5-trimethyleneheptane-1,7-diyl)bis(pyrrolidine), 4,4'-(3,4,5-trimethyleneheptane-1,7-diyl)bis(1-methylpiperazine), or a combination thereof.

Embodiment 8. A difunctional addition-polymerizable monomer composition comprising an amine-derivatized alpha-methyl styrene (ADAMS) monomer according to structure (V) and/or an aminated conjugated aliphatic methylated polyene (ACAMP) monomer according to structure (VI):

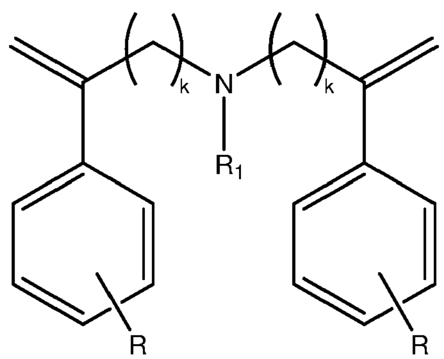

(V)

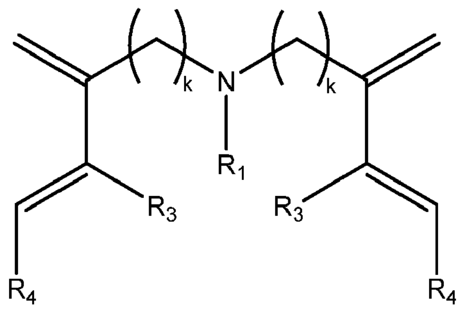

(VI)

wherein:

k is an integer from 1 to 3, such as 2;
$R_1$ and $R_2$ are each independently a hydrocarbyl group or a hydrocarbonaceous group having 1 to 4 additional heteroatoms selected from the group consisting of O, N, S, P, Se, and combinations thereof;
in structure (V), each R is independently hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group, or a $C_1$-$C_6$ hydrocarbyl group containing 1 to 4 additional heteroatoms selected from the group consisting of O, N, S, P, Se, and combinations thereof; and
in structure (VI), $R_3$ and $R_4$ are each independently hydrogen, a methyl group, or an alkenyl group having structure (IV)

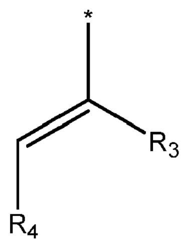

(IV)

attached via the asterisk, but specifically which do not collectively achieve aromaticity in combination with the other alkenes in structure (VI), and with the proviso that $R_3$ and $R_4$ are not both alkenyl groups having structure (IV) and are not both methyl groups.

Embodiment 9. A method of making an addition-polymerizable monomer composition comprising the steps of:

providing reactants comprising a source of formaldehyde, a source of a secondary amine, and a polymerizable reactant comprising one or more of alpha-methylstyrene, alpha-methyl-para-methylstyrene, alpha-methyl-ortho-methylstyrene, alpha-methyl-para-ethylstyrene, alpha-methyl-ortho-ethylstyrene, alpha-methyl-para-propylstyrene, alpha-methyl-ortho-propylstyrene, alpha-methyl-para-butylstyrene, alpha-methyl-ortho-butylstyrene, alpha-methyl-para-methoxystyrene, alpha-methyl-ortho-methoxystyrene, alpha-methyl-para-ethoxystyrene, alpha-methyl-ortho-ethoxystyrene, isopropenylnaphthalene, diisopropenylbenzene, diisopropenylnaphthalene, isoprene, 2,3-dimethylbuta-1,3-diene, 2-methylhexa-1,3,5-triene, 2,5-dimethylhexa-1,3,5-triene, 2-methyl-3-methylenepenta-1,4-diene, and 2,4-dimethyl-3-methylenepenta-1,4-diene;
providing an acidic reaction medium sufficient to provide, in the presence of the reactants, a pH from 0.8 to 4.8;
reacting the reactants in the acidic reaction medium for a sufficient reaction time and at a sufficient reaction temperature to thereby yield an amine-derivatized alpha-methyl styrene (ADAMS) monomer and/or an aminated conjugated aliphatic methylated polyene (ACAMP) monomer according to any one of embodiments 1-7.

Embodiment 10. The method of embodiment 9, wherein:

the source of formaldehyde comprises, at least in part, formaldehyde, trioxane, paraformaldehyde, or a combination thereof; and/or
the source of secondary amine comprises, at least in part, dimethylamine, N,N,N',N'-tetramethyl-diaminomethane, diethylamine, N,N,N',N'-tetraethyl-diaminomethane, di-n-propylamine, diisopropylamine, di-n-butylamine, di-sec-butylamine, di-isobutylamine, di-tert-butylamine, cyclohexylmethylamine, dicyclohexylamine, di-(2-ethylhexyl)amine, di-(methoxyethyl)amine, di-(ethoxyethyl)amine, di-(phenoxyethyl)amine, di-(methylthioethyl)amine,

di-(ethylthioethyl)amine, benzylmethylamine, dibenzylamine, benzylphenylamine, diphenylamine, dipyridylamine, phenylmethylamine, phenylmethoxylethylamine, benzylmethoxyethylamine, morpholine, thiomorpholine, piperidine, pyrrolidine, dihydropyrrole, pyrrole, tetrahydro-1-quinoline, tetrahydro-2-isoquinoline, indoline, indole, carbazole, phenothiazine, phenothiazine-S-oxide, phenothiazine-S,S-dioxide, phenoxazine, N-methylpiperazine, piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or a combination thereof,
with the proviso that the secondary amine and the polymerizable reactant are not both difunctional.

Embodiment 11. The method of embodiment 9 or embodiment 10, wherein the acidic reaction medium comprises formic acid, propionic acid, a butanoic acid, a pentanoic acid, a hexanoic acid, benzoic acid, chloroacetic acid, fluoroacetic acid, oxalic acid, malonic acid, maleic acid, fumaric acid, citric acid, orthophosphoric acid, nitric acid, dichloroacetic acid, difluoroacetic acid, trichloroacetic acid, trifluoroacetic acid, methanesulfonic acid, perchloric acid, or a combination thereof.

Embodiment 12. The method of any one of embodiments 9-11, wherein the reaction temperature ranges from about 40°C to about 190°C.

Embodiment 13. The method of any one of embodiments 9-12, wherein the reacting step comprises a primary reaction step at a first reaction temperature and for a first reaction time, as a result of which more than about 500 wppm, based on the reaction medium-free composition weight, of hydroxylation byproduct is formed, followed by a secondary reaction step at a second reaction temperature greater than the first reaction temperature and for a second reaction time to reduce the hydroxylation byproduct content by at least 50%, wherein the secondary reaction step comprises introducing an acid anhydride to convert at least a portion of the hydroxylation byproduct to a corresponding acetate intermediate, and optionally converting at least a portion of the corresponding acetate intermediate to exo isomer monomer, and optionally wherein not more than about 0.3 wt%, based on the reaction medium-free composition weight, of an acid anhydride is present following the secondary reaction step.

Embodiment 14. A method of making an addition-polymerizable monomer composition comprising the steps of:

providing reactants comprising a source of formaldehyde, a source of a primary amine, and a polymerizable reactant comprising one or more of alpha-methylstyrene, alpha-methyl-para-methylstyrene, alpha-methyl-ortho-methylstyrene, alpha-methyl-para-ethylstyrene, alpha-methyl-ortho-ethylstyrene, alpha-methyl-para-propylstyrene, alpha-methyl-ortho-propylstyrene, alpha-methyl-para-butylstyrene, alpha-methyl-ortho-butylstyrene, alpha-methyl-para-methoxystyrene, alpha-methyl-ortho-methoxystyrene, alpha-methyl-para-ethoxystyrene, alpha-methyl-ortho-ethoxystyrene, isopropenylnaphthalene, isoprene, 2-methylhexa-1,3,5-triene, and 2-methyl-3-methylenepenta-1,4-diene;
providing an acidic reaction medium sufficient to provide, in the presence of the reactants, a pH from 0.8 to 4.8;
reacting the reactants in the acidic reaction medium for a sufficient reaction time and at a sufficient reaction temperature to thereby yield the amine-derivatized alpha-methyl styrene (ADAMS) monomer and/or the aminated conjugated aliphatic methylated polyene (ACAMP) monomer according to any one of embodiments 8-13.

Embodiment 15. A method of making an addition-polymerizable monomer composition comprising the steps of:

providing reactants comprising a secondary amine, a source of methyltriphenylphosphonium halide, and a ketone reactant comprising a $C_1$-$C_3$ alkyl optionally substituted phenyl or naphthalenyl ketone, a $C_1$-$C_3$ alkyl olefinic mono- or di- unsaturated ketone, a phenylene- or naphthalene- bis($C_1$-$C_3$ alkan-1-one), 1,1-ethylenebis(k-alkanone), 1,2-ethylenebis(k-alkanone), and/or bis($C_1$-$C_3$ alkyl)diketone, wherein the ketone reactant includes a terminal alkyl carbon bearing a suitable leaving group or wherein the ketone reactant does not include a terminal alkyl carbon bearing a suitable leaving group and a source of formaldehyde is added to the reaction;
first reacting the secondary amine with the ketone reactant to form an aminated ketone condensation intermediate;
then reacting the aminated ketone condensation intermediate with the source of methyltriphenylphosphonium halide under strong basic (Wittig reaction) conditions to thereby yield the amine-derivatized alpha-methyl styrene (ADAMS) monomer and/or the aminated conjugated aliphatic methylated polyene (ACAMP) monomer according to embodiment 1; and
optionally removing condensation byproduct from the first reacting step and/or triphenylphosphine oxide byproduct from the second reacting step.

Embodiment 16. The method of embodiment 15, wherein one or more of the following is satisfied:

the suitable leaving group on each terminal alkyl carbon is a halide;
the source of methyltriphenylphosphonium halide comprises methyltriphenylphosphonium chloride, methyltriphenylphosphonium iodide, methyltriphenylphosphonium bromide, or a combination thereof; and

the secondary amine comprises dimethylamine, diethylamine, di-n-propylamine, diisopropylamine, di-n-butylamine, di-sec-butylamine, di-isobutylamine, di-tert-butylamine, cyclohexylmethylamine, dicyclohexylamine, di-(2-ethylhexyl)amine, di-(methoxyethyl)amine, di-(ethoxyethyl)amine, di-(phenoxyethyl)amine, di-(methylthioethyl)amine, di-(ethylthioethyl)amine, benzylmethylamine, dibenzylamine, benzylphenylamine, diphenylamine, dipyridylamine, phenylmethylamine, phenylmethoxylethylamine, benzylmethoxyethylamine, morpholine, thiomorpholine, piperidine, pyrrolidine, dihydropyrrole, pyrrole, tetrahydro-1-quinoline, tetrahydro-2-isoquinoline, indoline, indole, carbazole, phenothiazine, phenothiazine-S-oxide, phenothiazine-S,S-dioxide, phenoxazine, N-methylpiperazine, piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or a combination thereof, with the proviso that the secondary amine and the ketone reactant are not both difunctional.

Embodiment 17. The method of any one of embodiments 15-16, wherein the source of formaldehyde comprises, at least in part, formaldehyde, trioxane, paraformaldehyde, or a combination thereof.

The invention will now be described by way of non-limiting examples only.

## Examples

Unless specified otherwise, all reactions are performed in appropriately sized glass reactors, equipped with either magnetic or overhead stirring, and run under an inert atmosphere (*e.g.*, $N_2$). All 'equivalents' listed represent molar equivalents vs. the total moles of the amine reagent used in the reaction. Microwave reactions were conducted using a Biotage Initiator+™ and with dedicated microwave vials and caps, using the internal instrument control to maintain the target temperatures and times specified below.

Yields of the product mixtures are reported as mol % of conversion of the amine reagent (whether monofunctional or polyfunctional) to the corresponding product, except for residual AMS which is reported as a residual mol % of AMS vs. the moles of amine reagent used in the reaction.

In cases where an amine precursor is used, (*e.g.*, N,N,N',N'-tetramethyl diaminomethane as a precursor to dimethylamine) equivalents are reported vs. the total moles of the amine precursor reagent (regardless of the equivalents of amine moieties in the precursor), while mol % values for yield are reported as mol % of conversion of the amine reagent to the corresponding product assuming complete conversion of the amine precursor to the appropriate equivalents of active secondary amine moiety.

All reactions, unless stated otherwise, were carried out with a scale involving ~5-20 grams total of the starting amine (normalizing to 1.0 molar equivalents), and the remaining reagents scaled appropriately based on the molar equivalents described below. All reactions can optionally be distilled according to the procedures described below to improve the purity of the product mixtures.

pH measurements were recorded using a Mettler Toledo FiveGo™ F2 in conjunction with an InLab Reach Pro™ 425 glass probe.

Where the pH of the reaction mixtures was controlled or adjusted, manual liquid (*i.e.,* acid or acid solution) dosing into the reaction was achieved using a syringe pump piped into the reactor with manual stop/start control based on real-time pH measurement using the above pH probe. Or instead, where liquid (*i.e.*, acid or acid solution) dosing into the reaction was automated in response to pH changes within the reaction mixture, a Knick Portavo™ type 904 PH equipped with a Hamilton Polilyte Plus H VP™ 425 pH probe was controlled by Radleys Ava™ software v2.0.0, which acted upon a New Era™ 1000 syringe pump piped into the reactor and which contained the acids/solutions described in the individual examples below.

$^1$H NMR spectra were recorded at 300 MHz with Bruker AVANCE™-300 instruments. Samples were prepared in $CDCl_3$ and chemical shifts ($\delta$) are quoted in parts per million (ppm), referenced to TMS contained in the NMR solvent or, preferably, to benzyl benzoate where used, calibrating the singlet to 5.36 ppm. Structural assignments were made with additional information from COSY, HSQC and HMBC experiments.

To prepare the NMR samples, ~50-100 mg of the test sample was added to a vial with -600-1000 $\mu$L of $CDCl_3$ (all masses were recorded to an accuracy of $\pm$0.1 mg). Where quantitative $^1$H NMR measurements were used, ~15-30 mg of benzyl benzoate was also added to the NMR sample as an internal standard. The vial was sealed, mixed thoroughly and then transferred to an NMR tube.

The $^1$H NMR spectra were processed as follows:

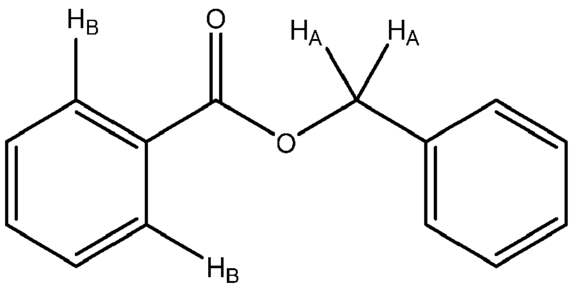

Benzyl Benzoate

Spectra were referenced to benzyl benzoate, calibrating the benzyl benzoate singlet ($H_A$) to 5.36 ppm. The spectra were then integrated between the regions of 8.12 and 8.03 ppm, corresponding to ~2 × $H_B$ signal. The integration of this peak was normalized to 1.0000.

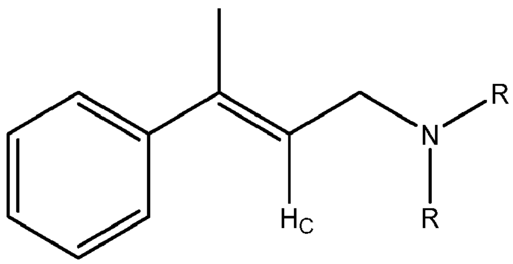

Endo-product

For endo-products, the $^1$H signal for the vinyl proton (*e.g.,* He for monomers with *k* = 2) was identified, typically appearing between ~5.70-6.00 ppm. The peak was integrated between the range defined by the midpoint of the peak ±0.046 ppm, unless the integral range was not baseline-resolved from any nearby peaks, and the minima between peaks occurred within the integrated range, in which case the integral was cut at the minima between the peaks.

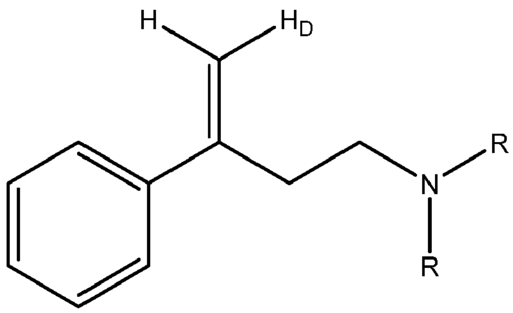

Exo-product

For exo-products, the $^1$H signal for the *trans* vinylidene proton (*e.g.,* Ho for monomers with k=2) was identified, typically appearing between ~5.10-5.15 ppm. The peak was integrated between the range defined by the midpoint of the peak ±0.030 ppm, unless the integral range was not baseline-resolved from any nearby peaks, and the minima between peaks occurred within the integrated range, in which case the integral was cut at the minima between the peaks.

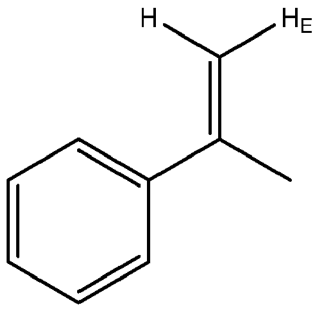

α-methylstyrene

For α-methylstyrene, the $^1$H signal for the *trans* vinylidene proton (*e.g.,* $H_E$ for monomers with k=2) was identified, typically appearing between ~5.06-5.08 ppm. The peak was integrated between the range defined by the midpoint of the peak ±0.021 ppm, unless the integral range was not baseline-resolved from any nearby peaks, and the minima between peaks occurred within the integrated range, in which case the integral was cut at the minima between the peaks.

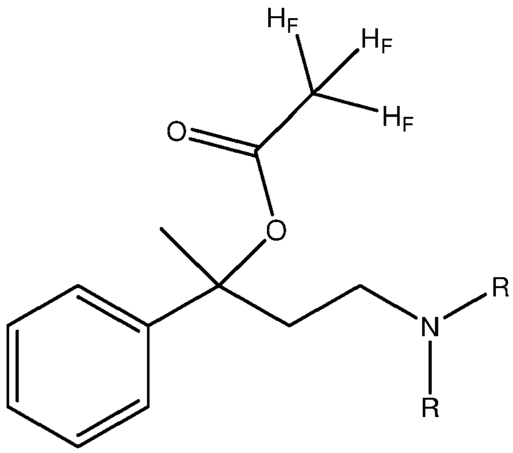

Acetate

For acetate products, the $^1$H signal for the acetate protons (*e.g.*, $H_F$ for monomers with k=2) was identified, typically appearing between -1.82-1.85 ppm. The peak was integrated between the range defined by the midpoint of the peak ±0.017 ppm, unless the integral range was not baseline-resolved from any nearby peaks, and the minima between peaks occurred within the integrated range, in which case the integral was cut at the minima between the peaks.

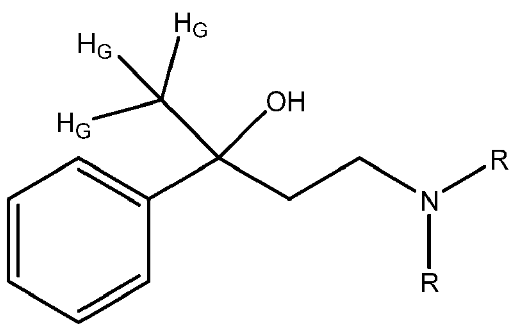

Hydrate

For hydrate products, the $^1$H signal for the terminal 'methyl' protons (*e.g.*, $H_G$ for monomers with k=2) was identified, typically appearing between ~1.50-1.55 ppm. The peak was integrated between the range defined by the midpoint of the peak ±0.019 ppm, unless the integral range was not baseline-resolved from any nearby peaks, and the

minima between peaks occurred within the integrated range, in which case the integral was cut at the minima between the peaks.

For example, in Figure 1, an acetate intermediate product was identified as the singlet peak with a midpoint chemical shift at ~1.843 ppm. The peak was integrated in the range ±0.017 ppm from the midpoint (*i.e.*, from ~1.826 to ~1.860 ppm in this example).

For example, in Figure 2, the peaks for the exo-product (identified as the peak with a chemical shift at -5.111 ppm) and residual α-methylstyrene (identified as the peak with chemical shift at ~5.074 ppm) were not fully resolved from each other. To integrate the peaks, the ranges for the respective products (*i.e.*, ±0.030 ppm and ±0.021 ppm for the exo-product and α-methylstyrene, respectively) were used, and the integral ranges cut at the minima between the peaks *(i.e.,* at -5.091 ppm in the example below).

The % yields for the product mixtures were calculated from the $^{1}$H NMR integrations via the following equations, where:

*$\%yield_{product}$ = the % yield of a given product in the reaction mixture*
*$\%comp_{product}$ = the mass % of a given product a NMR sample, relative to the mass of benzyl benzoate internal standard*
*$Int_{product}$ = the integration of the product peak in the $^{1}H$ NMR spectra*
*$Hyd_{product}$ = the number of equivalent hydrogen atoms in the product molecule corresponding to the $^{1}H$ NMR peak (e.g., 1 for the exo and/or endo products, 3 for the acetate product)*
*$MW_{product}$ = molecular weight of the product molecule, in g/mol*
*$MW_{amine}$ = molecular weight of the amine starting material (or amine precursor), in g/mol*
*$m_{BnBz}$ = mass of benzyl benzoate (internal standard) added to the $^{1}H$ sample, in mg*
*$m_{rxn}$ = total mass of the Aza-prins reaction mixture (all reagents/solvents), in g*
*$m_{nmr}$ = mass of the reaction sample used to prepare the $^{1}H$ NMR sample, in mg*
*$m_{amine}$ = total mass of the amine starting material (or amine precursor) used in the reaction, in g*

$$\%comp_{product} = 100 - \left( \frac{21224}{2 * MW_{product} * \frac{Int_{product}}{Hyd_{product}} + 212.24} \right)$$

$$\%\,yield_{product} = \frac{\%comp_{product}}{100 - \%comp_{product}} * \frac{MW_{amine}}{MW_{product}} * \left( \frac{m_{BnBz} * m_{rxn}}{m_{nmr} * m_{amine}} \right)$$

LCMS samples were prepared at ~0.5 mg/ml analyte in acetonitrile. Analysis was run on an Agilent™ single quod 6160 coupled to DAD detector equipped with an Agilent 1260 Infinity™ II quaternary pump eluting with the following conditions and solvent profile:

| | |
|---|---|
| **Column** | InfinityLab Poroshell™ 120 EC-C18, 2.1 mm × 50 mm, 2.7 μm |
| **Mobile phase A** | 10 mM Ammonium bicarbonate in water |
| **Mobile Phase B** | 100% Acetonitrile |
| **Oven Temp** | 40°C |
| **Injection Volume** | 1 μL |
| **Flow rate** | 0.5 mL/min |
| **Detection** | 250 nm ±30 nm |
| **Run time** | 7.5 mins |
| **Equilibration time** | 2.5 mins |

(continued)

| Gradient / time | A | B |
|---|---|---|
| **0** | 87.5 | 12.5 |
| **1.0** | 87.5 | 12.5 |
| **5.0** | 5 | 95 |
| **7.0** | 5 | 95 |
| **7.5** | 87.5 | 12.5 |

LCMS data analyses were performed using ACD/Spectrus™ Processor 2020.2.1.

Example AP-1

A solution of paraformaldehyde and 1-methylpiperazine was prepared by first charging paraformaldehyde (~1.2 eq.) and 1-methylpiperazine (~0.2 eq.) into a reaction vessel, with stirring, at room temperature (~20-25°C). The mixture was heated to ~70°C, and additional 1-methylpiperazine (~0.8 eq.) was dosed into the mixture in a controlled manner over ~60 minutes. The mixture was held at ~70°C until substantially complete dissolution of the paraformaldehyde, after which it was cooled to room temperature and used in the reaction.

The paraformaldehyde-amine solution was dosed over ~60 minutes into a pre-heated solution of $\alpha$-methylstyrene (~1.0 eq.) in acetic acid (~12.2 eq.) at ~120°C. The reaction was held at ~120°C for ~60 minutes (with reaction monitoring by LCMS and $^1$H NMR), before being cooled to room temperature.

The yields in the crude reaction mixture were determined by quantitative $^1$H NMR to be the following:

| | EXO | ENDO | AMS | Acetate | Hydrate |
|---|---|---|---|---|---|
| Crude | ~66% | ~12% | ~12% | ~6.4% | ~4.1% |

The crude product was not further processed or distilled.

Example AP-2

The reaction was run according to the procedure for Example AP-1, except scaled to ~74 grams of 1-methylpiperazine (normalized to 1.0 molar eq.).

The yields in the crude reaction mixture were determined by quantitative $^1$H NMR to be the following:

| | EXO | ENDO | AMS | Acetate | Hydrate |
|---|---|---|---|---|---|
| Crude | ~67% | ~12% | ~10% | ~6.4% | ~4.3% |

Volatiles were removed by vacuum distillation of the crude reaction mixture, after which further reducing the pressure afforded distillation of the product mixture, predominantly 1-methyl-4-(3-phenylbut-3-en-1-yl)piperazine, typically within the fractions distilled at ~170-190°C (vapor temperature ~145-150°C) and ~1-5 mbar.

The yields in the distilled reaction mixture were determined by quantitative $^1$H NMR to be the following:

| | EXO | ENDO | AMS | Acetate | Hydrate |
|---|---|---|---|---|---|
| Distilled | ~62% | ~10% | ~0.0% | ~1.5% | ~4.4% |

Example AP-3

The reaction was run according to the procedure for Example AP-1, except with ~11.2 eq. AcOH, where the paraformaldehyde-amine solution was dosed over ~240 minutes, and the reaction scaled to ~62 grams of 1-methylpiperazine (normalized to 1.0 molar eq.).

The yields in the crude reaction mixture were determined by quantitative $^1$H NMR to be the following:

| | EXO | ENDO | AMS | Acetate | Hydrate |
|---|---|---|---|---|---|
| Crude | ~70% | ~14% | ~8.6% | ~7.2% | ~5.2% |

The crude product was distilled according to the procedure for Example AP-2. The yields in the distilled reaction mixture were determined by quantitative $^1H$ NMR to be the following:

| | EXO | ENDO | AMS | Acetate | Hydrate |
|---|---|---|---|---|---|
| Distilled | ~68% | ~12% | ~0.0% | ~1.5% | ~3.7% |

Example AP-4

The reaction was run according to the procedure for Example AP-1, except with -11.2 eq. AcOH, the paraformaldehyde-amine solution was dosed over -120 minutes, and the reaction scaled to ~74 grams of 1-methylpiperazine (normalized to 1.0 molar eq.).

The yields in the crude reaction mixture were determined by quantitative $^1H$ NMR to be the following:

| | EXO | ENDO | AMS | Acetate | Hydrate |
|---|---|---|---|---|---|
| Crude | ~67% | ~14% | ~12% | ~5.3% | ~4.3% |

The crude product was distilled according to the procedure for Example AP-2. The yields in the distilled reaction mixture were determined by quantitative $^1H$ NMR to be the following:

| | EXO | ENDO | AMS | Acetate | Hydrate |
|---|---|---|---|---|---|
| Distilled | ~62% | ~11% | ~0.0% | ~2.4% | ~4.5% |

Example AP-5

The reaction was run according to the procedure for Example AP-1, except with -13.2 eq. AcOH, and the paraformaldehyde-amine solution was dosed over ~120 minutes.

The yields in the crude reaction mixture were determined by quantitative $^1H$ NMR to be the following:

| | EXO | ENDO | AMS | Acetate | Hydrate |
|---|---|---|---|---|---|
| Crude | ~67% | ~14% | ~9.6% | ~6.8% | ~3.7% |

The crude product was not further processed or distilled.

Example AP-6

The reaction was run according to the procedure for Example AP-1, except with ~13.2 eq. AcOH.

The yields in the crude reaction mixture were determined by quantitative $^1H$ NMR to be the following:

| | EXO | ENDO | AMS | Acetate | Hydrate |
|---|---|---|---|---|---|
| Crude | ~68% | ~14% | ~11% | ~7.0% | ~4.1% |

The crude product was not further processed or distilled.

Example AP-7

The reaction was run according to the procedure for Example AP-1, except with -13.2 eq. AcOH, and the

paraformaldehyde-amine solution was dosed over ~30 minutes.

The yields in the crude reaction mixture were determined by quantitative $^1$H NMR to be the following:

| | EXO | ENDO | AMS | Acetate | Hydrate |
|---|---|---|---|---|---|
| Crude | ~66% | ~13% | ~12% | ~7.1% | ~4.3% |

The crude product was not further processed or distilled.

Example AP-8

The reaction was run according to the procedure for Example AP-1, except with -13.2 eq. AcOH, and the paraformaldehyde-amine solution was dosed over ~5 minutes.

The yields in the crude reaction mixture were determined by quantitative $^1$H NMR to be the following:

| | EXO | ENDO | AMS | Acetate | Hydrate |
|---|---|---|---|---|---|
| Crude | ~63% | ~12% | ~16% | ~7.1% | ~5.4% |

The crude product was not further processed or distilled.

Example AP-9

The reaction was run according to the procedure for Example AP-1, except with ~13.2 eq. AcOH and where the reaction was heated to a vigorous reflux (~120°C internal temperature).

The yields in the crude reaction mixture were determined by quantitative $^1$H NMR to be the following:

| | EXO | ENDO | AMS | Acetate | Hydrate |
|---|---|---|---|---|---|
| Crude | ~61% | ~12% | ~11% | ~5.6% | ~3.2% |

The crude product was not further processed or distilled.

Example AP-10

The reaction was run according to the procedure for Example AP-1, except with ~14.2 eq. AcOH.

The yields in the crude reaction mixture were determined by quantitative $^1$H NMR to be the following:

| | EXO | ENDO | AMS | Acetate | Hydrate |
|---|---|---|---|---|---|
| Crude | ~69% | ~13% | ~10% | ~7.8% | ~3.9% |

The crude product was not further processed or distilled.

Example AP-11

The reaction was run according to the procedure for example AP-1, except with ~15.2 eq. AcOH.

The yields in the crude reaction mixture were determined by quantitative $^1$H NMR to be the following:

| | EXO | ENDO | AMS | Acetate | Hydrate |
|---|---|---|---|---|---|
| Crude | ~68% | ~13% | ~10% | ~8.2% | ~3.6% |

The crude product was not further processed or distilled.

Example AP-12

The reaction was run according to the procedure for Example AP-1, except the reaction was scaled to -60 grams of 1-methylpiperazine (normalized to 1.0 molar eq.) and with -60 grams of 3Å molecular sieves (~1-2 mm beads) added to the reaction mixture.

The yields in the crude reaction mixture were determined by quantitative $^1$H NMR to be the following:

| | EXO | ENDO | AMS | Acetate | Hydrate |
|---|---|---|---|---|---|
| Crude | ~62% | ~12% | ~13% | ~8.3% | ~3.7% |

The molecular sieves were removed by gravity filtration and washed with three ~20 mL portions of AcOH.

The crude product was distilled according to the procedure for Example AP-2. The yields in the distilled reaction mixture were determined by quantitative $^1$H NMR to be the following:

| | EXO | ENDO | AMS | Acetate | Hydrate |
|---|---|---|---|---|---|
| Distilled | ~51% | ~8.3% | ~0.0% | ~1.1% | ~2.9% |

Example AP-13

The reaction was run according to the procedure for Example AP-1, except with ~14.2 eq. AcOH and with -5.0 eq. of $Ac_2O$ added to the $\alpha$-methylstyrene/acetic acid solution at the start of the reaction.

The yields in the crude reaction mixture were determined by quantitative $^1$H NMR to be the following:

| | EXO | ENDO | AMS | Acetate | Hydrate |
|---|---|---|---|---|---|
| Crude | ~12% | ~3.6% | ~83% | ~8.1% | ~1.2% |

Crude $^1$H NMR indicated the appearance of product peaks consistent with 1 -acetyl-4-methylpiperazine.

The crude product was not further processed or distilled.

Example AP-14

The reaction was run according to the procedure for Example AP-1, except with ~14.2 eq. AcOH, and with -5.0 eq. of $Ac_2O$ dosed into the reaction mixture over -5 minutes, starting ~60 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~68% | ~11% | ~12% | ~8.4% | ~0.5% |

The crude product was not further processed or distilled.

Example AP-15

The reaction was run according to the procedure for Example AP-1, except with -0.5 eq. $Ac_2O$ dosed into the reaction mixture over -5 minutes, starting ~60 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~62% | ~13% | ~13% | ~6.5% | ~2.9% |

The crude product was not further processed or distilled.

Example AP-16

The reaction was run according to the procedure for Example AP-1, except with -1.0 eq. of $Ac_2O$ dosed into the reaction mixture over -5 minutes, starting ~60 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~67% | ~7.9% | ~15% | ~6.9% | ~2.1% |

The crude product was not further processed or distilled.

Example AP-17

The reaction was run according to the procedure for Example AP-1, except with ~2.0 eq. $Ac_2O$ dosed into the reaction mixture over -5 minutes, starting ~60 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~65% | ~9.8% | ~16% | ~7.6% | ~1.2% |

The crude product was not further processed or distilled.

Example AP-18

The reaction was run according to the procedure for Example AP-1, except with a reaction temperature of ~80°C and held at ~80°C for ~360 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~46% | ~5.8% | ~5.1% | ~29% | ~4.5% |

The crude product was not further processed or distilled.

Example AP-19

The reaction was run according to the procedure for Example AP-1, except the reaction was heated at ~90°C and held at ~90°C for ~120 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~58% | ~10% | ~8.6% | ~20% | ~5.3% |

The crude product was not further processed or distilled.

Example AP-20

The reaction was run according to the procedure for Example AP-1, except the reaction was heated at ~100°C and held at ~100°C for ~120 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~66% | ~13% | ~11% | ~8.0% | ~5.1% |

The crude product was not further processed or distilled.

Example AP-21

The reaction was run according to the procedure for Example AP-1, except the reaction was heated at ~110°C and held at ~110°C for ~120 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~68% | ~14% | ~12% | ~6.2% | ~4.0% |

The crude product was not further processed or distilled.

Example AP-22

The reaction was run according to the procedure for Example AP-1, except the reaction was scaled to -74 grams of 1-methylpiperazine (normalized to 1.0 molar eq.), heated to ~80°C, and held at ~80°C for ~60 minutes after compete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~48% | ~6.5% | ~9.0% | ~40% | ~5.0% |

The crude product was distilled according to the procedure for example AP-2. The yields in the distilled reaction mixture were determined by quantitative $^1H$ NMR to be the following:

| | EXO | ENDO | AMS | Acetate | Hydrate |
|---|---|---|---|---|---|
| Distilled | ~46% | ~5.2% | ~0.0% | ~29% | ~5.1% |

Example AP-23

The reaction was run according to the procedure for Example AP-1, except with -1.1 eq. paraformaldehyde, where the AcOH was replaced with -7.7 eq. of acetonitrile. Furthermore, the reaction was heated to ~80°C and the paraformaldehyde-amine solution was dosed over ~240 minutes, with simultaneous automated dosing of methanesulfonic acid (up to ~1.5 eq.) to maintain pH -3 in the reaction mixture. The final reaction pH was measured to be ~3.0.
The yields in the crude reaction mixture were determined by quantitative $^1H$ NMR to be the following:

| | EXO | ENDO | AMS | Acetate | Hydrate |
|---|---|---|---|---|---|
| Crude | ~48% | ~21% | ~15% | ~0.0% | ~1.8% |

The crude product was not further processed or distilled.

Example AP-24

The reaction was run according to the procedure for Example AP-1, except with ~1.1 eq. paraformaldehyde and where the reaction was heated to ~100°C. Furthermore, the acetic acid was replaced with a solution of ~2.97 eq. of AcOH premixed with ~0.33 eq. of methanesulfonic acid, and the paraformaldehyde-amine solution was dosed over ~240 minutes. The final reaction pH was measured to be ~4.7.
The yields in the crude reaction mixture were determined by quantitative $^1H$ NMR to be the following:

| | EXO | ENDO | AMS | Acetate | Hydrate |
|---|---|---|---|---|---|
| Crude | ~34% | ~5.1% | ~32% | ~11% | ~6.5% |

The crude product was not further processed or distilled.

Example AP-25

The reaction was run according to the procedure for Example AP-1, except with ~1.1 eq. paraformaldehyde and where the reaction was heated to ~100°C. Furthermore, the acetic acid was replaced with a solution of ~2.64 eq. of AcOH premixed with ~0.66 eq. of methanesulfonic acid, and the paraformaldehyde-amine solution was dosed over ~240 minutes. The final reaction pH was measured to be ~4.2.

The yields in the crude reaction mixture were determined by quantitative $^1$H NMR to be the following:

| | EXO | ENDO | AMS | Acetate | Hydrate |
|---|---|---|---|---|---|
| Crude | ~44% | ~8.5% | ~19% | ~7.4% | ~10% |

The crude product was not further processed or distilled.

Example AP-26

The reaction was run according to the procedure for Example AP-1, except with ~1.1 eq. paraformaldehyde and where the reaction was heated to ~100°C. Furthermore, the acetic acid was replaced with a solution of ~1.98 eq. of AcOH premixed with ~1.32 eq. of methanesulfonic acid, and the paraformaldehyde-amine solution was dosed over ~240 minutes. The final reaction pH was measured to be ~3.2.

The yields in the crude reaction mixture were determined by quantitative $^1$H NMR to be the following:

| | EXO | ENDO | AMS | Acetate | Hydrate |
|---|---|---|---|---|---|
| Crude | ~53% | ~13% | ~2.6% | ~1.4% | ~3.5% |

The crude product was not further processed or distilled.

Example AP-27

The reaction was run according to the procedure for Example AP-1, except with ~1.1 eq. paraformaldehyde and where the reaction was heated to ~100°C. Furthermore, the acetic acid was replaced by a solution of ~5.94 eq. of AcOH premixed with ~0.66 eq. of methanesulfonic acid, and the paraformaldehyde-amine solution was dosed over ~240 minutes. The final reaction pH was measured to be ~2.8.

The yields in the crude reaction mixture were determined by quantitative $^1$H NMR to be the following:

| | EXO | ENDO | AMS | Acetate | Hydrate |
|---|---|---|---|---|---|
| Crude | ~61% | ~13% | ~6.1% | ~6.4% | ~8.9% |

The crude product was not further processed or distilled.

Example AP-28

The reaction was run according to the procedure for Example AP-1, except with ~1.1 eq. paraformaldehyde and where the reaction was heated to ~100°C. Furthermore, the acetic acid was replaced with a solution of ~5.28 eq. of AcOH premixed with ~1.32 eq. of methanesulfonic acid and the paraformaldehyde-amine solution was dosed over ~240 minutes. The final reaction pH was measured to be ~2.4.

The yields in the crude reaction mixture were determined by quantitative $^1$H NMR to be the following:

| | EXO | ENDO | AMS | Acetate | Hydrate |
|---|---|---|---|---|---|
| Crude | ~56% | ~11% | ~1.4% | ~3.4% | ~3.3% |

The crude product was not further processed or distilled.

Example AP-29

The reaction was run according to the procedure for Example AP-1, except with ~1.1 eq. paraformaldehyde. Furthermore, the acetic acid was replaced with a solution of ~5.94 eq. of AcOH premixed with ~0.66 eq. of methanesulfonic acid, and the paraformaldehyde-amine solution was dosed over ~240 minutes.

The yields in the crude reaction mixture were determined by quantitative $^{1}H$ NMR to be the following:

| | EXO | ENDO | AMS | Acetate | Hydrate |
|---|---|---|---|---|---|
| Crude | ~59% | ~13% | ~7.7% | ~3.1% | ~5.6% |

The crude product was not further processed or distilled.

Example AP-30

The reaction was run according to the procedure for Example AP-1, except with ~1.1 eq. paraformaldehyde and where the reaction was heated to ~80°C. Furthermore, the acetic acid was replaced with a solution of ~5.94 eq. of AcOH premixed with ~0.66 eq. of methanesulfonic acid, and the paraformaldehyde-amine solution was dosed over ~240 minutes. The final reaction pH was measured to be ~3.5.

The yields in the crude reaction mixture were determined by quantitative $^{1}H$ NMR to be the following:

| | EXO | ENDO | AMS | Acetate | Hydrate |
|---|---|---|---|---|---|
| Crude | ~51% | ~6.7% | ~5.9% | ~22% | ~8.9% |

The crude product was not further processed or distilled.

Example AP-31

The reaction was run according to the procedure for Example AP-1, except with ~1.1 eq. paraformaldehyde and where the reaction was heated to ~100°C. Furthermore, the acetic acid was replaced with a solution of ~5.94 eq. of AcOH premixed with ~0.66 eq. of trifluoroacetic acid, and the paraformaldehyde-amine solution was dosed over ~240 minutes. The final reaction pH was measured to be ~3.3.

The yields in the crude reaction mixture were determined by quantitative $^{1}H$ NMR to be the following:

| | EXO | ENDO | AMS | Acetate | Hydrate |
|---|---|---|---|---|---|
| Crude | ~61% | ~9.9% | ~7.1% | ~7.9% | ~8.9% |

The crude product was not further processed or distilled.

Example AP-32

The reaction was run according to the procedure for Example AP-1, except with ~13.2 eq. AcOH and ~1.1 eq. paraformaldehyde. Furthermore, the reaction was heated to ~100°C, the paraformaldehyde-amine solution was dosed into the reaction mixture over ~240 minutes, then the reaction held at - 100°C for a further ~120 minutes after complete addition of the paraformaldehyde-amine solution. The final reaction pH was measured to be ~2.3.

The yields in the crude reaction mixture were determined by quantitative $^{1}H$ NMR to be the following:

| | EXO | ENDO | AMS | Acetate | Hydrate |
|---|---|---|---|---|---|
| Crude | ~63% | ~11% | ~8.4% | ~9.6% | ~6.7% |

The crude product was not further processed or distilled.

Example AP-33

The reaction was run according to the procedure for Example AP-1, except with ~6.6 eq. AcOH, ~1.1 eq. paraformaldehyde, and where ~7.23 eq. MeCN was added to the starting α-methylstyrene/acetic acid solution. Furthermore, the reaction was heated to ~92°C, the paraformaldehyde-amine solution was dosed over ~240 minutes, then the reaction held at ~92°C for -120 minutes after complete addition of the paraformaldehyde-amine solution. The final reaction pH was measured to be ~4.2.

The yields in the crude reaction mixture were determined by quantitative $^1$H NMR to be the following:

| | EXO | ENDO | AMS | Acetate | Hydrate |
|---|---|---|---|---|---|
| Crude | ~40% | ~3.9% | ~26% | ~21% | ~3.7% |

The crude product was not further processed or distilled.

Example AP-34

The reaction was run according to the procedure for Example AP-1, except with ~13.2 eq. AcOH and ~1.1 eq. paraformaldehyde. Furthermore, the paraformaldehyde-amine solution was dosed over -240 minutes, and where the reaction was held at ~120°C for ~180 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~63% | ~13% | ~14% | ~7.7% | ~4.0% |

The crude product was not further processed or distilled.

Example AP-35

The reaction was run according to the procedure for Example AP-1, except with -13.2 eq. AcOH, ~1.5 eq. paraformaldehyde, and the paraformaldehyde-amine solution was dosed over ~240 minutes.

The yields in the crude reaction mixture were determined by quantitative $^1$H NMR to be the following:

| | EXO | ENDO | AMS | Acetate | Hydrate |
|---|---|---|---|---|---|
| Crude | ~64% | ~13% | ~4.6% | ~7.3% | ~4.2% |

The crude product was not further processed or distilled.

Example AP-36

The reaction was run according to the procedure for Example AP-1, except with ~13.2 eq. AcOH, ~1.5 eq. α-methylstyrene, and the paraformaldehyde-amine solution was dosed over ~240 minutes.

The yields in the crude reaction mixture were determined by quantitative $^1$H NMR to be the following:

| | EXO | ENDO | AMS* | Acetate | Hydrate |
|---|---|---|---|---|---|
| Crude | ~56% | ~9.4% | ~69% | ~7.7% | ~3.2% |
| *AMS mol% values include residual material added as excess (*i.e.*, > 1.0 eq.) reagent | | | | | |

The crude product was not further processed or distilled.

Example AP-37

The reaction was run according to the procedure for Example AP-1, except with ~13.2 eq. AcOH, ~1.5 eq. α-methylstyrene, ~1.5 eq. paraformaldehyde, and the paraformaldehyde-amine solution was dosed over ~240 minutes.
The yields in the crude reaction mixture were determined by quantitative $^1$H NMR to be the following:

| | EXO | ENDO | AMS* | Acetate | Hydrate |
|---|---|---|---|---|---|
| Crude | ~68% | ~11% | ~31% | ~7.0% | ~4.5% |
| *AMS mol% values include residual material added as excess (*i.e.*, > 1.0 eq.) reagent | | | | | |

The crude product was not further processed or distilled.

Example AP-38

The reaction was run according to the procedure for Example AP-1, except with ~13.2 eq. AcOH, ~2.0 eq. paraformaldehyde, and the paraformaldehyde-amine solution was dosed over -240 minutes.
The yields in the crude reaction mixture were determined by quantitative $^1$H NMR to be the following:

| | EXO | ENDO | AMS | Acetate | Hydrate |
|---|---|---|---|---|---|
| Crude | ~58% | ~15% | ~2.4% | ~7.8% | ~4.0% |

The crude product was not further processed or distilled.

Example AP-39

The reaction was run according to the procedure for Example AP-1, except with ~26.4 eq. AcOH, ~1.1 eq. paraformaldehyde, and the paraformaldehyde-amine solution was dosed over -240 minutes.
The yields in the crude reaction mixture were determined by quantitative $^1$H NMR to be the following:

| | EXO | ENDO | AMS | Acetate | Hydrate |
|---|---|---|---|---|---|
| Crude | ~56% | ~11% | ~31% | ~15% | ~2.4% |

The crude product was not further processed or distilled.

Example AP-40

The reaction was run according to the procedure for Example AP-1, except with ~26.4 eq. AcOH, ~1.1 eq. paraformaldehyde, and where ~14.5 eq. MeCN was added to the starting α-methylstyrene/acetic acid solution. Furthermore, the reaction was heated to ~92°C, the paraformaldehyde-amine solution was dosed over ~240 minutes, and then the reaction was held at ~92°C for ~60 minutes after complete addition of the paraformaldehyde-amine solution.
The yields in the crude reaction mixture were determined by quantitative $^1$H NMR to be the following:

| | EXO | ENDO | AMS | Acetate | Hydrate |
|---|---|---|---|---|---|
| Crude | ~55% | ~3.8% | ~20% | ~26% | ~3.2% |

The crude product was not further processed or distilled.

Example AP-41

The reaction was run according to the procedure for Example AP-1, except with ~9.9 eq. AcOH, ~1.1 eq. paraformaldehyde, and the paraformaldehyde-amine solution was dosed over -240 minutes.
The yields in the crude reaction mixture were determined by quantitative $^1$H NMR to be the following:

| | EXO | ENDO | AMS | Acetate | Hydrate |
|---|---|---|---|---|---|
| Crude | ~51% | ~9.7% | ~25% | ~6.0% | ~3.4% |

The crude product was not further processed or distilled.

Example AP-42

The reaction was run according to the procedure for Example AP-1, except with ~16.5 eq. AcOH, ~1.1 eq. paraformaldehyde, and the paraformaldehyde-amine solution was dosed over ~240 minutes.
The yields in the crude reaction mixture were determined by quantitative $^1H$ NMR to be the following:

| | EXO | ENDO | AMS | Acetate | Hydrate |
|---|---|---|---|---|---|
| Crude | ~40% | ~7.2% | ~36% | ~8.6% | ~2.3% |

The crude product was not further processed or distilled.

Example AP-43

The reaction was run according to the procedure for Example AP-1, except with ~13.2 eq. AcOH, ~1.1 eq. paraformaldehyde, ~1.25 eq. α-methylstyrene, and the paraformaldehyde-amine solution was dosed over ~240 minutes.
The yields in the crude reaction mixture were determined by quantitative $^1H$ NMR to be the following:

| | EXO | ENDO | AMS* | Acetate | Hydrate |
|---|---|---|---|---|---|
| Crude | ~63% | ~12% | ~30% | ~7.3% | ~4.0% |
| *AMS mol% values include residual material added as excess (*i.e.*, > 1.0 eq.) reagent | | | | | |

The crude product was not further processed or distilled.

Example AP-44

The reaction was repeated as described in Example AP-42. The yields in the crude reaction mixture were determined by quantitative $^1H$ NMR to be the following:

| | EXO | ENDO | AMS | Acetate | Hydrate |
|---|---|---|---|---|---|
| Crude | ~42% | ~7.6% | ~29% | ~8.4% | ~2.2% |

The crude product was not further processed or distilled.

Example AP-45

The reaction was run according to the procedure for Example AP-1, except with ~13.2 eq. AcOH, ~1.1 eq. paraformaldehyde, and where ~7.49 eq. of 2-MeTHF was added to the starting α-methylstyrene/acetic acid solution. Furthermore, the reaction was heated to ~105°C, the paraformaldehyde-amine solution was dosed over ~240 minutes, then the reaction was held at ~105°C for -180 minutes after complete addition of the paraformaldehyde-amine solution.
The yields in the crude reaction mixture were determined by quantitative $^1H$ NMR to be the following:

| | EXO | ENDO | AMS | Acetate | Hydrate |
|---|---|---|---|---|---|
| Crude | ~41% | ~7.9% | ~33% | ~12% | ~2.3% |

The crude product was not further processed or distilled.

Example AP-46

The reaction was run according to the procedure for Example AP-1, except with ~13.2 eq. AcOH, ~1.1 eq. paraformaldehyde, and where ~7.49 eq. of 2-MeTHF was added to the starting α-methylstyrene/acetic acid solution. Furthermore, the reaction was heated to - 105°C, a Dean-Stark trap was added to the glassware setup, the paraformaldehyde-amine solution was dosed over ~240 minutes, and the reaction was held at - 105°C for ~60 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~28% | ~4.1% | ~50% | ~0.0% | ~2.7% |

Example AP-47

The reaction was run according to the procedure for Example AP-1, except with ~13.2 eq. AcOH and ~1.1 eq. paraformaldehyde. Instead of using the paraformaldehyde-amine solution, all paraformaldehyde was charged to the reaction vessel at the start, to which was added the α-methylstyrene/acetic acid solution. 1-methylpiperazine was dosed to the reaction mixture over ~240 minutes instead of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~48% | ~7.7% | ~15% | ~7.1% | ~2.1% |

Example AP-48

The reaction was run according to the procedure for Example AP-1, except with ~13.2 eq. AcOH, ~1.1 eq. paraformaldehyde, and the paraformaldehyde-amine solution was dosed over ~240 minutes.

The yields in the crude reaction mixture were determined by quantitative $^1$H NMR to be the following:

| | EXO | ENDO | AMS | Acetate | Hydrate |
|---|---|---|---|---|---|
| Crude | ~67% | ~13% | ~10% | ~7.8% | ~4.4% |

The crude product was not further processed or distilled.

Example AP-49

The reaction was run according to the procedure for Example AP-1, except with ~13.2 eq. AcOH and ~1.1 eq. paraformaldehyde. Furthermore, the reaction was scaled to -65 grams of 1-methylpiperazine (normalized to 1.0 molar eq.), and the paraformaldehyde-amine solution was dosed over -240 minutes.

The yields in the crude reaction mixture were determined by quantitative $^1$H NMR to be the following:

| | EXO | ENDO | AMS | Acetate | Hydrate |
|---|---|---|---|---|---|
| Crude | ~67% | ~13% | ~9.2% | ~6.8% | ~4.4% |

The crude product was not further processed or distilled.

Example AP-50

To a ~80°C solution of 1-methylpiperazine (normalized to 1.0 molar eq), ~1.0 eq. of α-methylstyrene, and ~6.6 eq. of acetic acid was charged ~1.1 eq. of paraformaldehyde in a single portion. The reaction was held at ~80°C for -420 minutes (with reaction monitoring by LCMS and $^1$H NMR), before being cooled to room temperature (~20-25°C). The final reaction pH was measured to be ~4.2.

The yields in the crude reaction mixture were determined by quantitative $^1$H NMR to be the following:

| | EXO | ENDO | AMS | Acetate | Hydrate |
|---|---|---|---|---|---|
| Crude | 32% | 3.8% | 23% | 36% | 8.4% |

Three aliquots of ~1.8 grams of reaction mixture were distributed into individual microwave vials and each heated for ~60 minutes at either ~120°C, ~140°C or ~160°C. The final reaction pH was measured to be ~4.0 in each vial. The yields in the reaction mixtures were determined by quantitative $^1$H NMR to be the following:

| | EXO | ENDO | AMS | Acetate | Hydrate |
|---|---|---|---|---|---|
| ~120°C | ~44% | ~7.0% | ~24% | ~17% | ~10% |
| ~140°C | ~48% | ~8.4% | ~29% | ~4.7% | ~9.9% |
| ~160°C | ~46% | ~8.8% | ~31% | ~4.5% | ~8.3% |

Example AP-51

The reaction was run according to the procedure for Example AP-50, except the initial reaction was heated to ~100°C. The final reaction pH was measured to be ~4.1.

The yields in the crude reaction mixture were determined by quantitative $^1$H NMR to be the following:

| | EXO | ENDO | AMS | Acetate | Hydrate |
|---|---|---|---|---|---|
| Crude | ~42% | ~6.8% | ~32% | ~8.8% | ~8.2% |

The crude product was not further processed or distilled.

Example AP-52

The reaction was run according to the procedure for Example AP-50, except with ~3.3 eq AcOH and where the reaction was heated to ~100°C. The final reaction pH was measured to be ~5.3.

The yields in the crude reaction mixture were determined by quantitative $^1$H NMR to be the following:

| | EXO | ENDO | AMS | Acetate | Hydrate |
|---|---|---|---|---|---|
| Crude | ~9.8% | ~0% | ~74% | ~6.0% | ~2.1% |

The crude product was not further processed or distilled.

Example AP-53

The reaction was run according to the procedure for Example AP-1, except with ~1.1 eq. paraformaldehyde, and ~3.3 eq AcOH. Furthermore, the reaction was heated to ~100°C and the paraformaldehyde-amine solution was dosed over ~240 minutes, after which the temperature was held at ~100°C for -180 minutes. The final reaction pH was measured to be ~5.4.

The yields in the crude reaction mixture were determined by quantitative $^1$H NMR to be the following:

| | EXO | ENDO | AMS | Acetate | Hydrate |
|---|---|---|---|---|---|
| Crude | ~22% | ~3.7% | ~44% | ~15% | ~3.6% |

The crude product was not further processed or distilled.

Example AP-54

The reaction was run according to the procedure for Example AP-1, except with ~1.1 eq. paraformaldehyde and ~3.3 eq AcOH. Furthermore, the reaction was heated to ~100°C, and the paraformaldehyde-amine solution was dosed over ~240 minutes with simultaneous manual dosing of methansulfonic acid (up to ~1.0 eq) to maintain pH -3 in the reaction mixture. After addition of the paraformaldehyde-amine solution, the reaction was held at ~100°C for an additional -180 minutes. The final reaction pH was measured to be ~3.6.

The yields in the crude reaction mixture were determined by quantitative $^1$H NMR to be the following:

| | EXO | ENDO | AMS | Acetate | Hydrate |
|---|---|---|---|---|---|
| Crude | ~51% | ~8.2% | ~14% | ~5.8% | ~11% |

The crude product was not further processed or distilled.

Example AP-55

The reaction was run according to the procedure for Example AP-1, except with ~1.1 eq. paraformaldehyde, and ~6.6 eq. AcOH. Furthermore, the reaction was heated to ~80°C, and the paraformaldehyde-amine solution was dosed over ~240 minutes, after which the temperature was held at ~80°C for an additional ~180 minutes.

The yields in the crude reaction mixture were determined by quantitative $^1$H NMR to be the following:

| | EXO | ENDO | AMS | Acetate | Hydrate |
|---|---|---|---|---|---|
| Crude | ~31% | ~3.2% | ~0% | ~31% | ~4.0% |

The crude product was not further processed or distilled.

Example AP-56

The reaction was run according to the procedure for Example AP-1, except with ~1.1 eq. paraformaldehyde, and ~6.6 eq AcOH. Furthermore, the reaction was heated to ~80°C, then held at ~80°C for an additional -180 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~29% | ~3.2% | ~0% | ~32% | ~4.4% |

The crude product was not further processed or distilled.

Example AP-57

The reaction was run according to the procedure for Example AP-1, except with ~1.1 eq. paraformaldehyde, ~6.6 eq AcOH, and addition of ~5.8 eq. water to the starting α-methylstyrene/acetic acid solution. Furthermore, the reaction was heated to ~80°C, and the paraformaldehyde-amine solution was dosed over ~240 minutes, after which the temperature was held at ~80°C for an additional -180 minutes.

The yields in the crude reaction mixture were determined by quantitative $^1$H NMR to be the following:

| | EXO | ENDO | AMS | Acetate | Hydrate |
|---|---|---|---|---|---|
| Crude | ~30% | ~2.8% | ~9.7% | ~20% | ~39% |

A sample of the crude product was collected, and volatiles were removed by evaporation at ~150°C on a rotary evaporator. The yields in the concentrated reaction mixture were determined by quantitative $^1$H NMR to be the following:

| | EXO | ENDO | AMS | Acetate | Hydrate |
|---|---|---|---|---|---|
| ~150°C under vac. | ~29% | ~3.7% | ~0% | ~17% | ~42% |

Example AP-58

The reaction was run according to the procedure for Example AP-1, except with ~6.6 eq. AcOH, and ~1.1 eq. paraformaldehyde. The reaction was heated to ~92°C and the paraformaldehyde-amine solution was dosed over ~240 minutes, after which the temperature was held at ~92°C for ~180 minutes. The final reaction pH was measured to be ~4.0.

The yields in the crude reaction mixture were determined by quantitative $^1H$ NMR to be the following:

| | EXO | ENDO | AMS | Acetate | Hydrate |
|---|---|---|---|---|---|
| Crude | ~43% | ~7.2% | ~19% | ~13% | ~4.5% |

The crude product was not further processed or distilled.

Example AP-59

The reaction was run according to the procedure for Example AP-1, except with ~13.2 eq. AcOH, and ~1.1 eq. paraformaldehyde. The reaction was heated to ~92°C and the paraformaldehyde-amine solution was dosed over ~240 minutes, after which the temperature was held at ~92°C for ~180 minutes. The final reaction pH was measured to be ~2.0.

The yields in the crude reaction mixture were determined by quantitative $^1H$ NMR to be the following:

| | EXO | ENDO | AMS | Acetate | Hydrate |
|---|---|---|---|---|---|
| Crude | ~56% | ~8.6% | ~8.8% | ~19% | ~5.1% |

The crude product was not further processed or distilled.

Example AP-60

The reaction was run according to the procedure for Example AP-1, except with ~13.2 eq. AcOH, ~1.1 eq. paraformaldehyde, and the reaction being heated to ~92°C. The α-methylstyrene was pre-mixed in the paraformaldehyde-amine solution instead of with the acetic acid, and the paraformaldehyde-amine-α-methylstyrene solution was dosed into the acetic acid over ~240 minutes, after which the temperature was held at ~92°C for ~180 minutes. The final reaction pH was measured to be ~3.9.

The yields in the crude reaction mixture were determined by quantitative $^1H$ NMR to be the following:

| | EXO | ENDO | AMS | Acetate | Hydrate |
|---|---|---|---|---|---|
| Crude | ~37% | ~6.2% | ~20% | ~14% | ~5.5% |

The crude product was not further processed or distilled.

Example AP-61

The reaction was run according to the procedure for Example AP-1, except with ~20 eq. AcOH, and ~1.1 eq. paraformaldehyde. The reaction was maintained at ~20°C and the paraformaldehyde-amine solution was dosed over -5 minutes, after which the temperature was held at ~20°C for ~8 days, with intermittent samples taken for quantitative $^1H$ NMR analysis. The final reaction pH was measured to be ~2.7.

| | EXO | ENDO | AMS | Acetate | Hydrate |
|---|---|---|---|---|---|
| ~1 day | ~28% | ~2.7% | ~30% | ~42% | ~3.9% |

(continued)

| | EXO | ENDO | AMS | Acetate | Hydrate |
|---|---|---|---|---|---|
| ~5 days | ~35% | ~3.3% | ~10% | ~58% | ~5.4% |
| ~8 days | ~35% | ~3.4% | ~7.4% | ~57% | ~5.3% |

The crude product was not further processed or distilled.

Example AP-62

The reaction was run according to the procedure for Example AP-61, except with an approximately equal mass (relative to 1-methylpiperazine) of 3Å molecular sieves (-1-2 mm beads) added to the reaction mixture. The final reaction pH was measured to be ~2.7.

| | EXO | ENDO | AMS | Acetate | Hydrate |
|---|---|---|---|---|---|
| ~1 day | ~30% | ~2.8% | ~30% | ~45% | ~2.8% |
| ~5 days | ~35% | ~3.3% | ~10% | ~58% | ~3.5% |
| ~8 days | ~36% | ~3.3% | ~7.8% | ~55% | ~3.5% |

The crude product was not further processed or distilled.

Example AP-63

The reaction was run according to the procedure for Example AP-1, except the reaction was scaled to -420 grams of 1-methylpiperazine (normalized to 1.0 molar eq.) and heated to ~100°C. The temperature was held at ~100°C for -420 minutes after complete addition of the paraformaldehyde-amine solution.

The yields in the crude reaction mixture were determined by quantitative $^1$H NMR to be the following:

| | EXO | ENDO | AMS | Acetate | Hydrate |
|---|---|---|---|---|---|
| Crude | ~64% | ~12% | ~7.1% | ~14% | ~6.1% |

A ~500g sample of the crude reaction mixture was taken, and volatiles were removed by vacuum distillation, after which further reducing the pressure afforded distillation of the product mixture, predominantly 1-methyl-4-(3-phenylbut-3-en-1-yl)piperazine, typically within the fractions distilled at ~ 170-190°C (vapor temperature ~145-150°C) and ~1-5 mbar.

The yields in the distilled mixture were determined by quantitative $^1$H NMR to be the following:

| | EXO | ENDO | AMS | Acetate | Hydrate |
|---|---|---|---|---|---|
| Distilled | ~60% | ~8.9% | ~0.0% | ~4.8% | ~6.2% |

Example AP-64

The reaction was run according to the procedure for Example AP-50, except where the acetic acid was replaced by ~6.6. eq. of -85% phosphoric acid. Furthermore, α-methylstyrene and 1-methylpiperazine were combined at ~20°C before the reaction was heated to ~100°C, phosphoric acid was charged once ~40°C was attained, and paraformaldehyde was charged once -95°C was attained.

Crude $^1$H NMR indicated decomposition of α-methylstyrene and appearance of peaks consistent with 2,4-diphenyl-4-methylpent-1-ene. Crude $^1$H NMR indicated substantially no conversion to exo/endo aza-prins products. The reaction was stopped.

Without being bound by theory, it is believed that the reaction conditions were too strongly acidic, leading to oligomerization of the α-methylstyrene reactant.

Example AP-65

The reaction was run according to the procedure for Example AP-1, except the acetic acid was replaced by ~3.5 eq. of toluene premixed with ~0.1 eq. of boron trifluoride diethyl etherate. Upon addition of the Lewis-acid solution to α-methylstyrene an exotherm was observed. Crude $^1$H NMR indicated decomposition of α-methylstyrene and appearance of product peaks consistent with 1,1,3-trimethyl-3-phenylindane. The reaction was stopped. Without being bound by theory, it is believed that the reaction conditions were too strongly Lewis-acidic, leading to oligomerization of the α-methylstyrene reactant.

Example AP-66

The reaction was run according to the procedure for Example AP-1, except the acetic acid was replaced by ~3.5 eq. of toluene containing ~0.1 eq. of anhydrous zirconium(IV) chloride. Upon addition of the Lewis-acid solution to α-methylstyrene an exotherm was observed. Crude $^1$H NMR indicated decomposition of α-methylstyrene and appearance of product peaks consistent with 1,1,3-trimethyl-3-phenylindane. The reaction was stopped. Without being bound by theory, it is believed that the reaction conditions were too strongly Lewis-acidic, leading to oligomerization of the α-methylstyrene reactant.

Example AP-67

The reaction was run according to the procedure for Example AP-1, except the acetic acid was replaced by ~0.1 eq. of sulfuric acid. Upon addition of the acid to the α-methylstyrene, an exotherm was observed. Crude $^1$H NMR indicated decomposition of α-methylstyrene and appearance of product peaks consistent with 1,1,3-trimethyl-3-phenylindane. The reaction was stopped. Without being bound by theory, it is believed that the reaction conditions were too strongly acidic, leading to oligomerization of the α-methylstyrene reactant.

Example AP-68

The reaction was run according to the procedure for Example AP-1, except the acetic acid was replaced by ~0.1 eq. of methanesulfonic acid. Upon addition of the acid to the α-methylstyrene an exotherm was observed. Crude $^1$H NMR indicated decomposition of α-methylstyrene and appearance of product peaks consistent with 1,1,3-trimethyl-3-phenylindane. The reaction was stopped. Without being bound by theory, it is believed that the reaction conditions were too strongly acidic, leading to oligomerization of the α-methylstyrene reactant.

Example AP-69

The reaction was run according to the procedure for Example AP-1, except the acetic acid was replaced by ~0.1 eq. of anhydrous aluminum(III) chloride. Upon addition of the Lewis-acid to the α-methylstyrene an exotherm was observed. Crude $^1$H NMR indicated decomposition of α-methylstyrene and appearance of product peaks consistent with 1,1,3-trimethyl-3-phenylindane. The reaction was stopped. Without being bound by theory, it is believed that the reaction conditions were too strongly acidic, leading to oligomerization of the α-methylstyrene reactant.

Example AP-70

The reaction was run according to the procedure for Example AP-1, except the acetic acid was replaced by ~0.1 eq. of anhydrous tin(IV) chloride. Upon addition of the Lewis-acid to the α-methylstyrene a mild exotherm was observed along with an increase in viscosity. Crude $^1$H NMR indicated decomposition of α-methylstyrene, and the reaction was stopped. Without being bound by theory, it is believed that the reaction conditions were too strongly Lewis-acidic, leading to oligomerization of the α-methylstyrene reactant.

Example AP-71

The reaction was run according to the procedure for Example AP-1, except the acetic acid was replaced by an approximately equal mass (relative to 1-methylpiperazine) of Amberlyst™ 36. Upon addition of the acid solution to the α-methylstyrene, no apparent reaction occurred but crude $^1$H NMR indicated partial decomposition of α-methylstyrene along with appearance of product peaks consistent with 2,4-diphenyl-4-methylpent-1-ene. The reaction was stopped. Without being bound by theory, it is believed that the reaction conditions were too strongly acidic, leading to oligomerization of the α-methylstyrene reactant.

Example AP-72

The reaction was run according to the procedure for Example AP-1, except the acetic acid was replaced by an approximately equal mass (relative to 1-methylpiperazine) of Montmorillonite K10. Upon addition of the acid to the $\alpha$-methylstyrene an exotherm was observed. Crude $^1$H NMR indicated decomposition of $\alpha$-methylstyrene and appearance of product peaks consistent with 2,4-diphenyl-4-methylpent-1-ene. The reaction was stopped. Without being bound by theory, it is believed that the reaction conditions were too strongly acidic, leading to oligomerization of the $\alpha$-methylstyrene reactant.

Example AP-73

The reaction was run according to the procedure for Example AP-50, except the acetic acid was replaced by ~0.1 eq. of p-toluenesulfonic acid monohydrate. All reagents were combined at ~20°C and then heated to ~120°C for ~30 minutes. Crude $^1$H NMR indicated substantially no conversion to exo/endo aza-prins products.

Example AP-74

The reaction was run according to the procedure for Example AP-50, except the acetic acid was replaced by a solution of ~3.5 eq. of toluene and ~1.2 eq. of *p*-toluenesulfonic acid monohydrate. All reagents were combined at ~20°C and then heated to ~120°C for ~30 minutes. The reaction mixture was uncharacteristically biphasic with solid deposition. Crude $^1$H NMR indicated substantially no conversion to exo/endo aza-prins products. The reaction was stopped.

Example AP-75

The reaction was run according to the procedure for Example AP-50, except the acetic acid was replaced by ~0.1 eq. of anhydrous zinc(II) chloride. All reagents were combined at ~20°C and then heated to ~120°C for ~30 minutes. Crude $^1$H NMR indicated substantially no conversion to exo/endo aza-prins products. The reaction was stopped.

Example AP-76

The reaction was run according to the procedure for Example AP-50, except the acetic acid was replaced by ~3.5 eq. of toluene and ~1.2 eq. of anhydrous zinc(II) chloride. The reagents were combined at ~20°C and then heated to ~120°C for ~30 minutes. The reaction mixture was uncharacteristically biphasic with solid deposition. Crude $^1$H NMR indicated substantially no conversion to exo/endo aza-prins products. The reaction was stopped.

Example AP-77

The reaction was run according to the procedure for Example AP-1, except the acetic acid was replaced by ~6.6 eq. of formic acid, the reaction was heated to ~80°C, ~1.1 eq paraformaldehyde was used, and the paraformaldehyde-amine solution was dosed over ~240 minutes. The reaction was held at ~80°C for a further -180 minutes after complete addition of the paraformaldehyde-amine solution. Crude $^1$H NMR indicated substantially no conversion to exo/endo aza-prins products and instead indicated a product consistent with 1,4-dimethylpiperazine. The reaction was stopped.

Example AP-78

The reaction was run according to the procedure for Example AP-50, except where $\alpha$-methylstyrene was replaced by isoprene (~1.2. eq.) and ~13.2 eq. AcOH was used. The reaction was performed via microwave heating in a sealed vial, where all reactants were combined at ~20°C before heating the mixture to ~120°C for ~60 minutes. LCMS analysis indicated the presence of exo and/or endo aza-prins products (although the individual isomers could not be resolved via LCMS) as well as acetate derivatives.

Example AP-79

The reaction was run according to the procedure for Example AP-1, except with ~1.1 eq. paraformaldehyde, and the acetic acid was replaced with a solution of ~6.6 eq. acetic acid and ~3.5 eq. toluene. The reaction mixture was heated to a strong reflux (~110°C) and the paraformaldehyde-amine solution was dosed over ~240 minutes, after which

the temperature was maintained for a further ~180 minutes. The product was worked up by diluting the reaction mixture with toluene (-1.2 eq.) and water (~28 eq.), adjusting the pH to -13 with -50% w/w aq. NaOH, and then separating the organic and aqueous phases. Solvent was removed from the organic phase by rotary evaporation and crude yields were determined by quantitative $^1$H NMR to be the following:

| | EXO | ENDO | AMS | Acetate | Hydrate |
|---|---|---|---|---|---|
| Crude | ~34% | ~6.0% | ~22% | ~5.8% | ~0.8% |

The crude product was not further processed or distilled.

Example AP-80

To a ~30°C solution of 1-methylpiperazine (normalized to 1.0 molar eq) and ~1.0 eq. of camphor sulfonic acid, in -62 eq. of acetonitrile, was added -10 eq. of paraformaldehyde in a single portion, followed by dosing of ~1.0 eq. of α-methylstyrene over -15 minutes. The reaction was heated to ~80°C and held at this temperature for -200 minutes (with reaction monitoring by LCMS and $^1$H NMR), before being cooled to room temperature (~20-25°C).

$^1$H NMR of the crude reaction mixture indicated minimal conversion to exo/endo ACP products, notably in poor conversion when compared with α-methylstyrene. The reaction was stopped.

Example AP-81

~6.6 eq. of acetic acid was dosed over -200 minutes into a solution of 1-methylpiperazine (normalized to 1.0 molar eq.) and ~1.0 eq. of paraformaldehyde, heated to ~80°C. After complete addition of the acetic acid, ~1.0 eq. of α-methylstyrene was dosed into the reaction over ~7 minutes, after which the reaction was held at ~80°C for an additional ~180 minutes.

$^1$H NMR of the crude reaction mixture indicated minimal conversion to exo/endo ACP products, notably in poor conversion when compared with α-methylstyrene. The reaction was stopped.

Example AP-82

To a -5 mL microwave vial containing a magnetic stir bar was added ~1.1 eq. of paraformaldehyde, followed by 1-methylpiperazine (normalized to 1.0 molar eq.), followed by ~1.2 eq. of α-methylstyrene, then ~13.2 eq. of acetic acid. The vial headspace was flushed with $N_2$, capped, and heated to ~120°C for ~60 minutes using a Biotage Initiator+™ microwave reactor.

The monomer yields in the crude reaction mixture were determined by quantitative $^1$H NMR to be the following:

| | EXO | ENDO | AMS* | Acetate | Hydrate |
|---|---|---|---|---|---|
| Crude | ~63% | ~9.6% | ~35% | ~7.0% | ~8.8% |
| *AMS mol% values include residual material added as excess (*i.e.*, > 1.0 eq.) reagent | | | | | |

The crude product was not further processed or distilled.

Example AP-83

The reaction was run according to the procedure for Example AP-82, except the 1-methylpiperazine was replaced with 1,2,4-triazole (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~1.7% | ~0.5% | ~71% | ~5.7% | ~0.1% |
| *AMS mol% values include residual material added as excess (*i.e.*, > 1.0 eq.) reagent | | | | | |

The crude product was not further processed or distilled.

Example AP-84

The reaction was run according to the procedure for Example AP-82, except the 1-methylpiperazine was replaced with morpholine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~49% | ~4.9% | ~39% | ~7.7% | ~0.0% |
| *AMS mol% values include residual material added as excess (*i.e.*, > 1.0 eq.) reagent | | | | | |

The crude product was not further processed or distilled.

Example AP-85

The reaction was run according to the procedure for Example AP-82, except the 1-methylpiperazine was replaced with thiomorpholine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~54% | ~4.9% | ~39% | ~8.0% | ~2.0% |
| *AMS mol% values include residual material added as excess (*i.e.*, > 1.0 eq.) reagent | | | | | |

The crude product was not further processed or distilled.

Example AP-86

The reaction was run according to the procedure for Example AP-82, except the 1-methylpiperazine was replaced with N-methylaniline (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~3.9% | ~0.0% | ~73% | ~9.4% | ~0.9% |
| *AMS mol% values include residual material added as excess (*i.e.*, > 1.0 eq.) reagent | | | | | |

The crude product was not further processed or distilled.

Example AP-87

The reaction was run according to the procedure for Example AP-82, except the 1-methylpiperazine was replaced with diisobutylamine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~2.9% | ~0.0% | ~59% | ~9.1% | ~0.3% |
| *AMS mol% values include residual material added as excess (*i.e.*, > 1.0 eq.) reagent | | | | | |

The crude product was not further processed or distilled.

Example AP-88

The reaction was run according to the procedure for Example AP-82, except the 1-methylpiperazine was

replaced with 1,2,3,4-tetrahydroisoquinoline (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~17% | ~2.1% | ~58% | ~7.0% | ~0.0% |
| *AMS mol% values include residual material added as excess (*i.e.*, > 1.0 eq.) reagent | | | | | |

The crude product was not further processed or distilled.

Example AP-89

The reaction was run according to the procedure for Example AP-82, except the 1-methylpiperazine was replaced with 1,2,3,4-tetrahydroquinoline (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~4.1% | ~0.0% | ~87% | ~17% | ~0.0% |
| *AMS mol% values include residual material added as excess (*i.e.*, > 1.0 eq.) reagent | | | | | |

The crude product was not further processed or distilled.

Example AP-90

The reaction was run according to the procedure for Example AP-82, except the 1-methylpiperazine was replaced with bis(2-methoxyethyl)amine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~46% | ~0.0% | ~61% | ~8.5% | ~0.2% |
| *AMS mol% values include residual material added as excess (*i.e.*, > 1.0 eq.) reagent | | | | | |

The crude product was not further processed or distilled.

Example AP-91

The reaction was run according to the procedure for Example AP-82, except the 1-methylpiperazine was replaced with carbazole (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~0.0% | ~0.0% | ~72% | ~0.0% | ~4.1% |
| *AMS mol% values include residual material added as excess (*i.e.*, > 1.0 eq.) reagent | | | | | |

The crude product was not further processed or distilled.

Example AP-92

The reaction was run according to the procedure for Example AP-82, except the 1-methylpiperazine was replaced with diphenylamine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~3.5% | ~0.0% | ~64% | ~9.4% | ~0.0% |
| *AMS mol% values include residual material added as excess (*i.e.*, > 1.0 eq.) reagent | | | | | |

The crude product was not further processed or distilled.

Example AP-93

The reaction was run according to the procedure for Example AP-82, except the 1-methylpiperazine was replaced with 2-(piperazin-1-yl)thiazole (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~53% | ~14% | ~20% | ~10% | ~3.5% |
| *AMS mol% values include residual material added as excess (*i.e.*, > 1.0 eq.) reagent | | | | | |

The crude product was not further processed or distilled.

Example AP-94

The reaction was run according to the procedure for Example AP-82, except the 1-methylpiperazine was replaced with di(pyridin-2-yl)amine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~4.3% | ~0.0% | ~31% | ~7.5% | ~0.0% |
| *AMS mol% values include residual material added as excess (*i.e.*, > 1.0 eq.) reagent | | | | | |

The crude product was not further processed or distilled.

Example AP-95

The reaction was run according to the procedure for Example AP-82, except the 1-methylpiperazine was replaced with dicyclohexylamine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~0.0% | ~0.0% | ~83% | ~0.0% | ~0.0% |
| *AMS mol% values include residual material added as excess (*i.e.*, > 1.0 eq.) reagent | | | | | |

The crude product was not further processed or distilled.

Example AP-96

The reaction was run according to the procedure for Example AP-82, except the 1-methylpiperazine was replaced with N-benzylaniline (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~2.5% | ~0.0% | ~35% | ~4.0% | ~0.0% |
| *AMS mol% values include residual material added as excess (*i.e.*, > 1.0 eq.) reagent | | | | | |

The crude product was not further processed or distilled.

Example AP-97

The reaction was run according to the procedure for Example AP-82, except the 1-methylpiperazine was replaced with dibenzylamine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~0.0% | ~0.0% | ~62% | ~7.8% | ~0.0% |
| *AMS mol% values include residual material added as excess (*i.e.*, > 1.0 eq.) reagent | | | | | |

The crude product was not further processed or distilled.

Example AP-98

The reaction was run according to the procedure for Example AP-82, except the 1-methylpiperazine was replaced with phenothiazine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~4.8% | ~0.0% | ~29% | ~0.0% | ~0.0% |
| *AMS mol% values include residual material added as excess (*i.e.*, > 1.0 eq.) reagent | | | | | |

The crude product was not further processed or distilled.

Example AP-99

The reaction was run according to the procedure for Example AP-82, except using ~2.2 eq. paraformaldehyde, ~2.4 eq. $\alpha$-methylstyrene, and the 1-methylpiperazine being replaced with 1,4-diazepane (normalized to 1.0 molar eq.).

$^1$H NMR of the crude reaction mixture indicated a complex mixture of products. The crude product was not further processed or distilled.

Example AP-100

The reaction was run according to the procedure for Example AP-82, except the 1-methylpiperazine was replaced with bis(2-ethylhexyl)amine (normalized to 1.0 molar eq.).

The monomer yields in the crude reaction mixture were determined by quantitative $^1$H NMR to be the following:

| | EXO | ENDO | AMS* | Acetate | Hydrate |
|---|---|---|---|---|---|
| Crude | ~10% | ~0.0% | ~78% | ~11% | ~0.0% |
| *AMS mol% values include residual material added as excess (*i.e.*, > 1.0 eq.) reagent | | | | | |

The crude product was not further processed or distilled.

Example AP-101

The reaction was run according to the procedure for Example AP-82, except the 1-methylpiperazine was replaced with 1,3-di(piperidin-4-yl)propane (normalized to 1.0 molar eq.).

$^1H$ NMR of the crude reaction mixture produced multiple peaks that could not be clearly resolved for the exo product. The crude reaction mixture was diluted with ~20 mL of toluene and adjusted to pH - 8 (as indicated by universal pH indicator paper) by manual addition of a saturated aq. $NaHCO_3$ with manual mixing. The organic and aqueous layers were separated, and the organic layer was washed with 3 × ~20 mL portions of distilled water. Volatiles were removed from the organic layer by rotary evaporation, leaving behind isolated product.

The monomer yields in the isolated product mixture were determined by quantitative $^1H$ NMR to be the following:

| | EXO | ENDO | AMS* | Acetate | Hydrate |
|---|---|---|---|---|---|
| Worked up | ~37% | ~2.6% | ~0.0% | ~3.5% | ~3.1% |
| *AMS mol% values include residual material added as excess (*i.e.*, > 1.0 eq.) reagent | | | | | |

The isolated product was not further processed or distilled.

Example W-1a

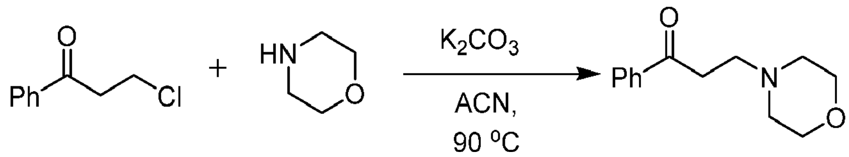


To a stirred solution of 3-chloro-1-phenyl-1-propanone (~200 grams, ~1.19 mol, ~1.0 eq.) in acetonitrile (-2.0 L), morpholine (~102 ml, ~1.19 mol, ~1.0 eq.) was added dropwise at room temperature (-20-25°C; the internal temperature exothermed up to ~50°C during addition) and stirred at the same temperature for -20 minutes. $K_2CO_3$ (~197 grams, ~1.42 mol, ~1.2 eq.) was added to the above reaction mixture portion wise to avoid foaming and heated to ~90°C for ~6 hours. After complete conversion (monitored by TLC), reaction mixture was filtered through a Buchner funnel and the filtrate concentrated under reduced pressure. The crude product was dissolved in ethyl acetate (-4.0 L) and water (-4.0 L). The layers were separated, and the aqueous layer was extracted with ethyl acetate (-3.0 L, twice). The combined organic extract was washed with brine solution (-3.0 L). The organic extract was dried over anhydrous $Na_2SO_4$, filtered, and concentrated under reduced pressure to afford the desired product 3-morpholino-1-phenylpropan-1-one (~255 grams, -98% yield) as brown liquid. It was taken for the next reaction without further purification.

Example W-1b

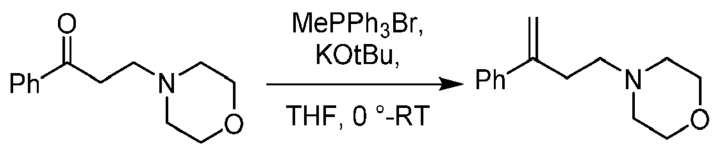


To a stirred suspension of methyltriphenylphosphonium bromide (~283 grams, ~0.79 mol, ~1.2 eq.) in THF (~1.2 L) at ~0°C, a potassium tert-butoxide solution (~921 ml, ~1.4 eq., -1M in THF) was added dropwise at ~0-10°C by cannula over a period of -1 hour. After complete addition, the reaction mixture was stirred at ~0-10°C for ~2 hours. A solution of the 3-morpholino-1-phenylpropan-1-one from reaction W-1a (~155 grams, ~0.658 mol, ~1.0 eq.) in THF (~300 ml) was added dropwise at the same temperature, and the reaction mixture was stirred at room temperature (~20-25 °C) for ~16 hours. After complete conversion (monitored by TLC), the reaction mixture was cooled to ~0°C and quenched with water (-1.5 L). The reaction mixture was diluted with ethyl acetate (-5.0 L) and water (-5.0 L). Both the layers were separated. Aqueous layer was extracted with ethyl acetate (-5.0 L, twice). The combined organic extracts were washed

with water (-3.0 L), then brine solution (-3.0 L parts), and dried over anhydrous $Na_2SO_4$, filtered, and concentrated under reduced pressure to afford the crude product as brown liquid.

Initially, a small quantity of the crude product was distilled under vacuum and collected in 4 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~140°C | ~107°C | ~3.0 mbar | ~3.4 g |
| 2 | ~145°C | ~116°C | ~3.1 mbar | ~8.9 g |
| 3 | ~155°C | ~124°C | ~3.3 mbar | ~14 g |
| 4 | ~165°C | ~126°C | ~3.1 mbar | ~33 g |

The above reaction was repeated a second time and the crude product combined with that remaining from the first instance. Both were distilled under vacuum and collected in 4 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~155°C | ~130°C | ~4.7 mbar | ~5.8 g |
| 2 | ~160°C | ~116°C | ~3.7 mbar | ~18 g |
| 3 | ~165°C | ~131°C | ~3.7 mbar | ~21 g |
| 4 | ~175°C | ~136°C | ~4.0 mbar | ~100 g |

Fraction-4 contained substantially pure 4-(3-phenylbut-3-en-1-yl)morpholine and the analytics of both the distilled products were the same.

$^1H$ NMR (~400 MHz, $CDCl_3$): $\delta$ ppm ~7.44-7.41 (m, ~2H), -7.37-7.33 (m, ~2H), -7.31-7.27 (m, ~1H), ~5.33 (d, $J \approx$ 1.2 Hz, ~1H), ~5.12 (m, -1H), ~3.74 (t, $J \approx$ 4.8 Hz, ~4H), -2.76-2.72 (m, ~2H), ~2.52-2.48 (m, ~6H); Mol. Formula: $C_{14}H_{19}NO$; Mol wt. (calc): ~217.31 Da; Mass peak observed in LCMS: ~218.1 Da $[M+1]^+$.

Example W-2a

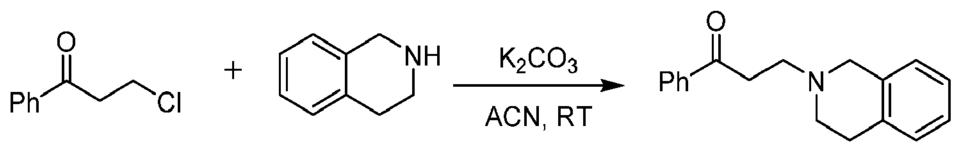


A suspension of 1,2,3,4-tetrahydroisoquinoline (~500 grams, ~3.75 mol, ~1.0 eq.) and $K_2CO_3$ (~675 grams, ~4.88 mol, ~1.3 eq.) in acetonitrile (-4.0 L) was stirred at ambient temperature for -30 minutes and cooled to ~0-5°C. 3-chloro-1-phenyl-1-propanone (~760 mL, ~4.50 mol, ~1.2 eq.) dissolved in acetonitrile (-1.0 L) was added dropwise over ~60-75 minutes to the above mixture, and the reaction mixture was allowed to stir at ambient temperature for ~16 hours. After complete conversion (monitored by TLC), the reaction mixture was filtered through a Buchner funnel and washed with ethyl acetate (-4.0 L). The solvent was removed via rotary evaporation, and the solid was dissolved in water (-4.0 L) and ethyl acetate (-5.0 L) and stirred for ~1 hour. The organic layer was extracted and washed with water (-3.0 L), then brine (-3.0 L), then dried over anhydrous $Na_2SO_4$, filtered, and concentrated over rotary evaporator to yield crude product (~870 grams).

To a stirred solution of the crude material (~865 grams, ~3.26 mol) in ethyl acetate (-1.73 L), a -4N HCl solution in 1,4-dioxane (~865 mL) was added dropwise at ~0-5°C, and the resulting suspension was stirred at ambient temperature for ~1 hour. The solid was precipitated and filtered through a Buchner funnel, then washed with hexane (-3.0 L), and dried under reduced pressure. The solid was diluted with ethyl acetate (~4.0 L) and water (-3.0 L) and basified with saturated solution of sodium bicarbonate (-3.0 L). The resulting suspension was stirred for ~1-2 hours. The organic layer

was separated, then washed with water (-3.0 L), then brine (-3.0 L), then dried over anhydrous sodium sulfate, filtered, and concentrated over rotary evaporator to get substantially pure 3-(3,4-dihydroisoquinolin-2(1H)-yl)-1-phenylpropan-1-one (~806 grams, -82% yield).

Example W-2b

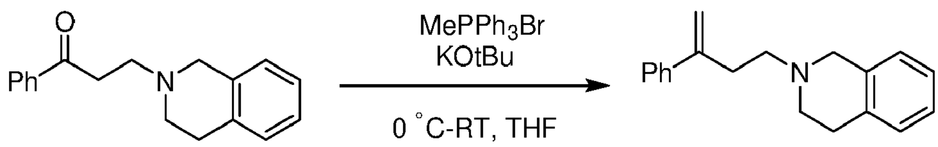


2-(3-phenylbut-3-en-1-yl)-1,2,3,4-tetrahydroisoquinoline was prepared according to the procedure for Example W-1b, except that the 3-morpholino-1-phenylpropan-1-one was replaced with ~1.0 equivalent of 3-(3,4-dihydroisoquinolin-2(1H)-yl)-1-phenylpropan-1-one, and the reaction scaled to -250 grams (-0.943 mol) of the starting ketone.

Furthermore, the reaction was repeated twice, and the combined material purified by adsorbing the material onto silica gel (-60-120 mesh) and column chromatography (eluting with -5% v/v ethyl acetate in hexanes), instead of fractional distillation. The reactions yielded -335 grams total of substantially pure 2-(3-phenylbut-3-en-1-yl)-1,2,3,4-tetrahydroisoquinoline (~71% yield).

$^1$H NMR (~400 MHz, DMSO-d6): δ -7.51-7.48 (m, ~2H), -7.41-7.28 (m, ~3H), -7.17-7.14 (m, ~3H), ~7.06 (dd, $J \approx 2.8$, 5.2 Hz, ~1H), -5.38 (d, $J \approx 1.6$ Hz, ~1H), ~5.19 (dd, $J \approx 1.2$, 2.8 Hz, ~1H), ~3.72 (s, ~1H), ~2.96 (t, $J \approx 5.6$ Hz, ~2H), ~2.88-2.86 (m, ~2H), 2.81 (t, $J \approx 6.0$ Hz, ~2H), 2.72-2.69 (m, ~2H). Mol. Formula: $C_{19}H_{21}N$, Mol wt. (calc): ~263.38 Da, Mass peak observed in LCMS: ~264.1 Da $[M+1]^+$.

Example W-3a

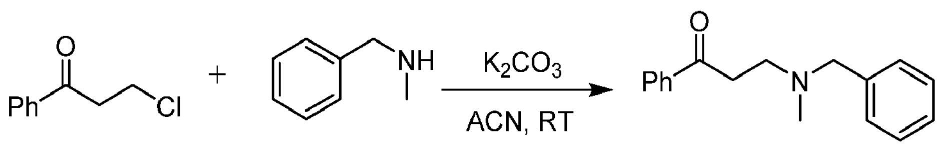


To a stirred solution of N-benzyl methylamine (~360 grams, ~2.97 mol, ~1.0 eq.) in acetonitrile (-2.0 L), $K_2CO_3$ (~822 grams, ~5.95 mol, ~2.0 eq.) was added at room temperature (~20-25°C). The suspension was stirred at room temperature for -15 minutes and cooled to ~0°C. A solution of 3-chloro-1-phenyl-1-propanone (~500 grams, ~2.97 mol, ~1.0 eq.) in acetonitrile (-2.0 L) was added portion wise to the reaction mixture while maintaining the temperature of ~0-5°C. After the addition, the reaction mixture was stirred at room temperature for -16 hours. After complete conversion (monitored by TLC), the reaction mixture was quenched with water (-2.0 L) and extracted with ethyl acetate (-2.0 L, twice). The organic phase was dried over anhydrous $Na_2SO_4$, filtered, and concentrated under reduced pressure to afford the desired product (~712 grams) as a liquid. The crude 3-(benzyl(methyl)amino)-1-phenylpropan- 1-one was used directly for the next reaction without further purification.

Example W-3b

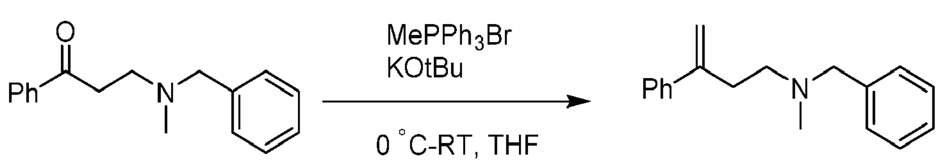


N-benzyl-N-methyl-3-phenylbut-3-en-1-amine was prepared according to the procedure for Example W-1b, except that the 3-morpholino-1-phenylpropan-1-one was replaced with ~1.0 equivalent of 3-(benzyl(methyl)amino)-1-phenylpropan-1-one, the reaction scaled to -712 grams (~2.81 mol) of the starting ketone, and ~1.5 eq. (-4.21 mol) of solid potassium *tert*-butoxide used instead of the solution.

Furthermore, instead of fractional distillation, the crude material was triturated with hexane (~10L) to remove residual triphenylphosphine oxide, then filtered, and the solvent removed by rotary evaporation, followed by column chromatography (eluting with -5% v/v ethyl acetate in hexanes) on silica gel (-100-200 mesh).

To a stirred solution of the chromatographed material (~260 grams, ~1.03 mol) in methyl tert-butyl ether, a -4N HCl solution in 1,4-dioxane was added dropwise at ~0-5°C until no further precipitate formed. The solid precipitate was filtered, then washed with further methyl tert-butyl ether. The solid was collected, dissolved in water, and then basified with saturated solution of sodium bicarbonate. The resulting suspension was extracted with dichloromethane (-2.0 L, twice) and combined organic layers were dried over anhydrous sodium sulfate, then filtered, and concentrated via rotary evaporation to yield -190 grams of substantially pure N-benzyl-N-methyl-3-phenylbut-3-en-1-amine (-35% yield).

$^1H$ NMR (~400 MHz, $CDCl_3$): δ -7.45-7.37 (m, ~2H), -7.35-7.27 (m, ~8H), ~5.35 (s, ~1H), ~5.13 (s, ~1H), ~3.55 (s, ~1H), -2.81-2.77 (m, ~2H), 2.61-2.57 (m, ~2H), 2.28 (s, ~1H); Mol. Formula $C_{18}H_{21}N$, Mol. Wt. (calc): ~251.37 Da Mass peak observed in LCMS: ~252.5 Da $[M+1]^+$.

Example W-4a

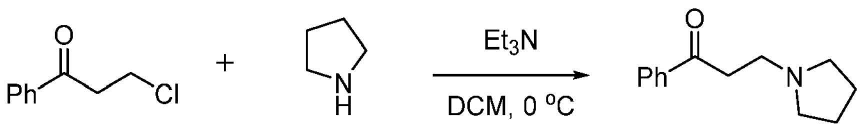


To a stirred solution of pyrrolidine (~243 mL, ~2.97 mol, ~1.0 eq.) in dichloromethane (-5.0 L) at room temperature (~20-25°C), triethylamine (~828 mL, ~5.93 mol, ~2.0 eq.) was added. The resulting reaction mixture was stirred at room temperature for -15 minutes and cooled to ~0-5°C. To this reaction mixture, 3-chloro-1-phenyl-1-propanone (~500 grams, ~2.97 mol, ~1.0 eq.) was added portion wise while maintaining the temperature of ~0-5°C. After the addition was complete, the reaction mixture was stirred at room temperature for -16 hours. After complete conversion (monitored by TLC), the reaction mixture was cooled to ~0-5°C and quenched with water (-4.0 L) and extracted with dichloromethane (-2.0 L, twice). The combined organic extract was washed with water (-5.0 L, twice) and brine solution (-5.0 L). The organic phase was dried over anhydrous $Na_2SO_4$, then filtered, and solvent removed by rotary evaporation. The crude product was purified by column chromatography (eluting with dichloromethane) on neutral alumina, to afford -440 grams of substantially pure 1-phenyl-3-(pyrrolidin-1-yl)propan-1-one (-73% yield) as a brown liquid.

Example W-4b

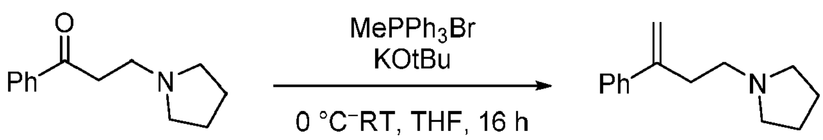


1-(3-phenylbut-3-en-1-yl)pyrrolidine was prepared according to the procedure for Example W-1b, except that the 3-morpholino-1-phenylpropan-1-one was replaced with ~1.0 equivalent of 1-phenyl-3-(pyrrolidin-1-yl)propan-1-one, and the reaction scaled to -440 grams (-2.16 mol) of the starting ketone.

Furthermore, the crude material was triturated with hexanes (~10L) to remove residual triphenylphosphine oxide, then filtered, and solvent removed by rotary evaporation, prior to fractional distillation according to the below conditions.

The crude product (~400 grams) was distilled under vacuum and collected 3 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~115°C | ~95°C | ~2.2 mbar | ~27 g |
| 2 | ~130°C | ~97°C | ~2.2 mbar | ~113 g |
| 3 | ~140°C | ~99°C | ~2.2 mbar | ~225 g |

Fractions 2 and 3 were combined to yield ~340 grams of substantially pure 1-(3-phenylbut-3-en-1-yl)pyrrolidine (~78%) as a brown liquid.

$^1$H NMR (~400 MHz, $CDCl_3$): $\delta$ ppm -7.45-7.43 (m, ~2H), -7.36-7.27 (m, ~3H), ~5.33 (s, ~1H), ~5.13 (s, ~1H), -2.80-2.75 (m, ~2H), ~2.61-2.53 (m, ~6H), -1.82-1.79 (m, ~4H); Mol. Formula: $C_{14}H_{19}N$; Mol. Wt. (calc): ~201.31 Da; Mass peak observed in LCMS: ~202.1 Da $[M+1]^+$.

Example W-5a

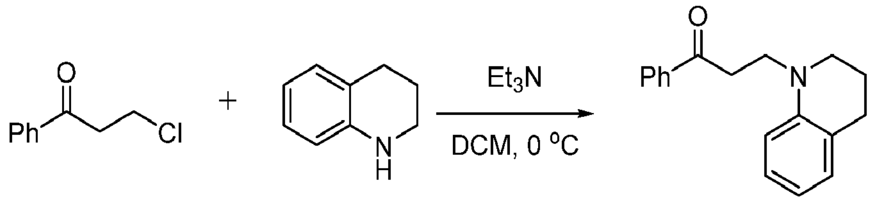


3-(3,4-dihydroquinolin-1(2H)-yl)-1-phenylpropan-1-one was prepared according to the procedure for Example W-4a (at the same scale, ~2.97 mol), except that the pyrrolidine was replaced by ~1.0 eq. of 1,2,3,4-tetrahydroquinoline, additional triethylamine was added to the reaction (~943 ml, ~6.52 mol, ~2.2 eq.), and the extraction steps were performed with dichloromethane in place of ethyl acetate. The crude 3-(3,4-dihydroquinolin-1(2H)-yl)-1-phenylpropan-1-one was used directly for the next reaction without further purification.

Example W-5b

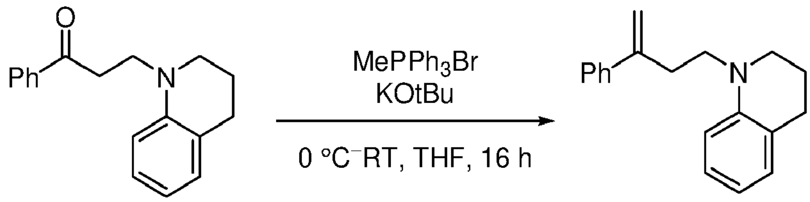


2-(3-phenylbut-3-en-1-yl)-1,2,3,4-tetrahydroquinoline was prepared according to the procedure for Example W-1b, except that the 3-morpholino-1-phenylpropan-1-one was replaced with ~1.0 equivalent of 3-(3,4-dihydroquinolin-1(2H)-yl)-1-phenylpropan-1-one, the reaction scaled to -600 grams (~2.26 mol) of the starting ketone, and ~1.6 eq. (-3.62 mol) of potassium *tert*-butoxide solution used instead.

Furthermore, instead of fractional distillation, the crude material was triturated with hexane (~10L) to remove residual triphenylphosphine oxide, filtered, and solvent removed by rotary evaporation, prior to column chromatography (eluting with hexanes) on silica gel (-60-120 mesh). The reaction yielded -340 grams of substantially pure 2-(3-phenylbut-3-en-1-yl)-1,2,3,4-tetrahydroquinoline (-57% yield) as a brown liquid.

$^1$H NMR (~400 MHz, $CDCl_3$): $\delta$ ppm ~7.54-7.51 (m, ~2H), ~7.45-7.41 (m, ~3H), -7.14-710 (m, ~1H), -7.10-7.03 (m, ~1H), ~6.65-6.61 (m, ~2H), ~5.44 (d, $J \approx$ 1.2 Hz, ~1H), ~5.20 (d, $J \approx$ 1.2 Hz, ~1H), ~3.47-3.45 (m, ~2H), ~3.32 (t, $J \approx$ 5.8 Hz, ~2H), -2.88-2.79 (m, ~4H), -2.01-1.95 (m, ~2H); Mol. Formula: $C_{19}H_{21}N$, Mol. Wt. (calc.): ~263.38 Da, Mass peak observed in LCMS: ~264.4 Da $[M+1]^+$.

Example W-6a

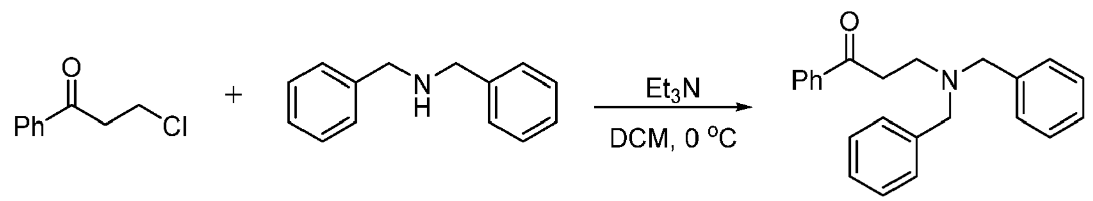


3-(Dibenzylamino)-1-phenylpropan-1-one was prepared according to the procedure for Example W-4a (at the same scale, ~2.97 mol), except that the pyrrolidine was replaced with ~1.0 eq. of dibenzylamine, additional triethylamine was added to the reaction (~943 ml, ~6.52 mol, ~2.2 eq.), and the extraction steps were performed with dichloromethane in place of ethyl acetate. The crude 3-(dibenzylamino)-1-phenylpropan- 1-one was used directly for the next reaction without further purification.

Example W-6b

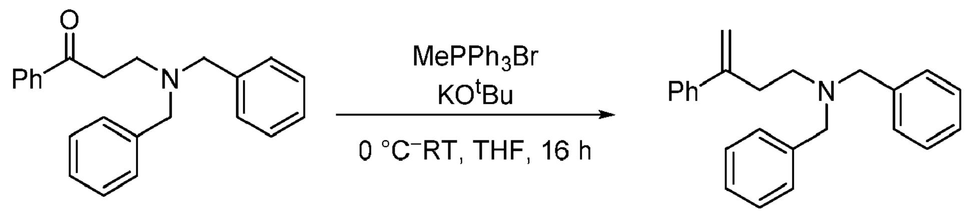


N,N-dibenzyl-3-phenylbut-3-en-1-amine was prepared according to the procedure for Example W-1b, except that the 3-morpholino-1-phenylpropan-1-one was replaced with ~1.0 eq. of 3-(dibenzylamino)-1-phenylpropan-1-one, and the reaction was scaled to -600 g (-1.82 mol) of the starting ketone, with ~1.5 eq (~2.73 mol) of methyltriphenylphosphonium bromide and ~2.0 eq. (~3.64) of solid potassium *tert*-butoxide used instead.

Furthermore, instead of fractional distillation, the crude material was triturated with hexane (~15L) to remove residual triphenylphosphine oxide, then filtered, and solvent removed by rotary evaporation, prior to column chromatography (eluting with -2% v/v ethyl acetate in hexanes) on silica gel (-60-120 mesh). The reaction yielded -330 grams of substantially pure N,N-dibenzyl-3-phenylbut-3-en-1-amine (-55% yield) as a yellow liquid.

$^1$H NMR (~400 MHz, $CDCl_3$): $\delta$ ppm -7.49-7.33 (m, ~15H), ~5.42 (d, $J \approx$ 1.6 Hz, ~1H), ~5.15 (s, ~1H), ~3.74 (s, ~4H), ~2.87 (t, $J \approx$ 7.2 Hz, ~2H), -2.76-2.73 (m, ~2H); Mol. Formula: $C_{24}H_{25}N$; Mol. Wt. (calc): ~327.47 Da; Mass peak observed in LCMS: ~328.4 Da $[M+1]^+$.

Example W-7a

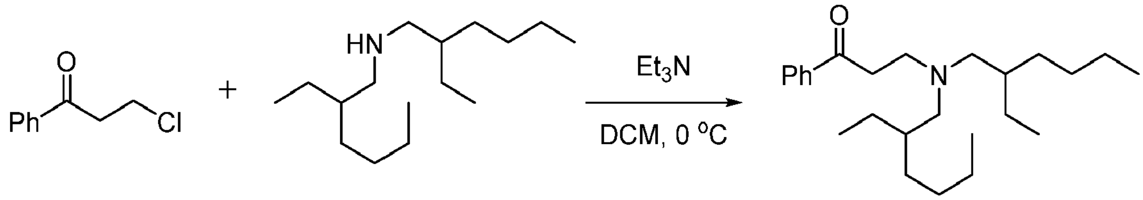


3-(bis(2-ethylhexyl)amino)-1-phenylpropan-1-one was prepared according to the procedure for Example W-4a (at the same scale, ~2.97 mol), except that the pyrrolidine was replaced with ~1.1 eq. of bis(2-ethylhexyl)amine (~984 ml, ~3.26 mol), additional triethylamine was added to the reaction (-1.23 L, ~8.90 mol, ~3.0 eq.), and the extraction steps were performed with dichloromethane in place of ethyl acetate. The crude 3-(bis(2-ethylhexyl)amino)-1-phenylpropan-1-one was used directly in the next reaction without further purification.

Example W-7b

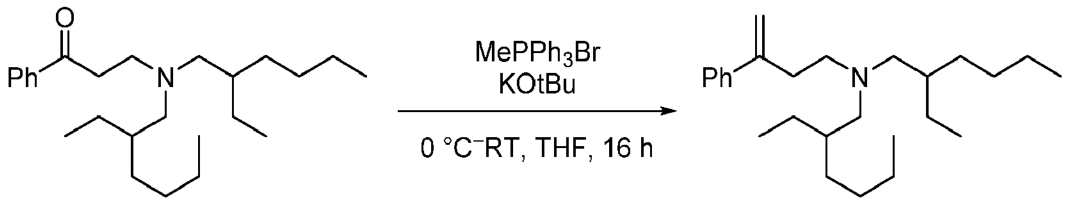


2-Ethyl-N-(2-ethylhexyl)-N-(3-phenylbut-3-en-1-yl)hexan-1-amine was prepared according to the procedure for Example W-1b, except that the 3-morpholino-1-phenylpropan-1-one was replaced with ~1.0 eq. of 3-(bis(2-ethylhexyl)amino)-1-phenylpropan-1-one, the reaction scaled to -600 grams (~1.61 mol) of the starting ketone and ~1.4 eq. (-2.25 mol) of solid potassium *tert*-butoxide used instead.

Furthermore, instead of fractional distillation, the crude material was triturated with hexane (~10L) to remove residual triphenylphosphine oxide, then filtered, and solvent removed by rotary evaporation, prior to column chromatography (eluting with -2% v/v ethyl acetate in hexanes) on silica gel (-60-120 mesh). The reaction yielded -302 grams of substantially pure 2-ethyl-N-(2-ethylhexyl)-N-(3-phenylbut-3-en-1-yl)hexan-1-amine (-50% yield) as a brown liquid.

$^1H$ NMR (~400 MHz, $CDCl_3$): $\delta$ ppm -7.44-7.42 (m, ~2H), -7.36-7.33 (m, ~2H), -7.30-7.28 (m, ~1H), ~5.31 (s, ~1H), ~5.08 (s, ~1H), -2.66-2.62 (m, ~2H), -2.50-2.47 (m, ~2H), -2.22-2.18 (m, ~4H), ~1.59-1.37 (m, ~18H), ~1.31-1.24 (m, ~12H); Mol. Formula: $C_{26}H_{45}N$; Mol. Wt. (calc): ~371.65 Da; Mass peak observed in LCMS: ~372.2 Da [M+1] $^+$.

Example W-8a

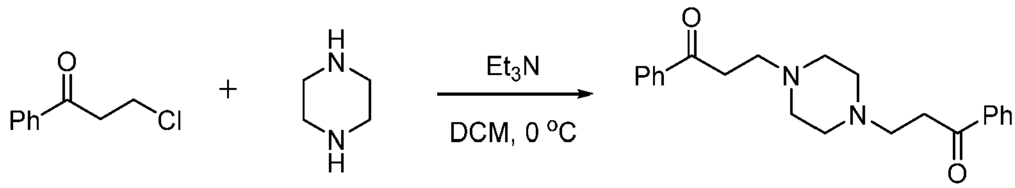


3,3'-(piperazine-1,4-diyl)bis(1-phenylpropan-1-one) was prepared according to the procedure for Example W-4a (at the same scale, ~2.97 mol), except that the pyrrolidine was replaced with ~0.5 eq. of piperazine (~127 ml, ~1.48 mol) , and less triethylamine was added to the reaction (~618 ml, ~4.45 mol, ~1.5 eq.). The crude 3,3'-(piperazine-1,4-diyl)bis(1-phenylpropan-1-one) was used directly for the next reaction without further purification.

Example W-8b

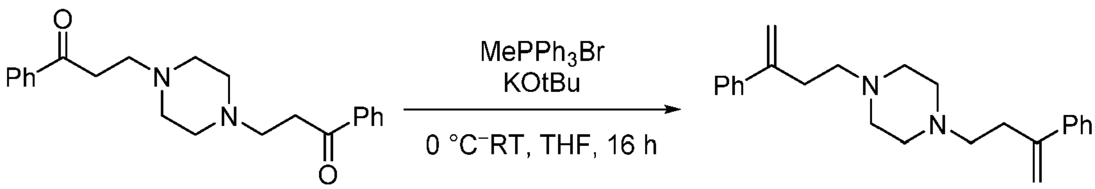


1,4-bis(3-phenylbut-3-en-1-yl)piperazine was prepared according to the procedure for Example W-1b, except that the 3-morpholino-1-phenylpropan-1-one was replaced with ~1.0 eq. of 3-(dibenzylamino)-1-phenylpropan-1-one, the reaction scaled to -500 grams (-1.43 mol) of the starting diketone, with ~2.5 eq (-3.57 mol) of methyltriphenylphosphonium bromide and ~3.0 eq. (-4.28 mol) of solid potassium *tert*-butoxide used instead.

Furthermore, instead of fractional distillation, the crude material was triturated with hexane (~10L) to remove

residual triphenylphosphine oxide, then filtered, and solvent removed by rotary evaporation, prior to column chromatography (eluting with -2% v/v ethyl acetate in hexanes) on silica gel (-60-120 mesh). The reaction yielded ~282 grams total of substantially pure 1,4-bis(3-phenylbut-3-en-1-yl)piperazine (-57% yield) as an off-white solid.

$^1$H NMR (~400 MHz, $CDCl_3$): $\delta$ ppm -7.45-7.43 (m, ~4H), -7.37-7.26 (m, ~6H), ~5.33 (d, $J \approx$ 1.6, ~2H), ~5.12 (d, $J \approx$ 1.2, ~2H), -2.76-2.72 (m, ~4H), ~2.53-2.49 (m, ~12H); Mol. Formula: $C_{24}H_{30}N_2$; Mol. Wt. (calc): ~346.52 Da; Mass peak observed in LCMS: ~347.2 Da [M+1] $^+$.

Example W-9a

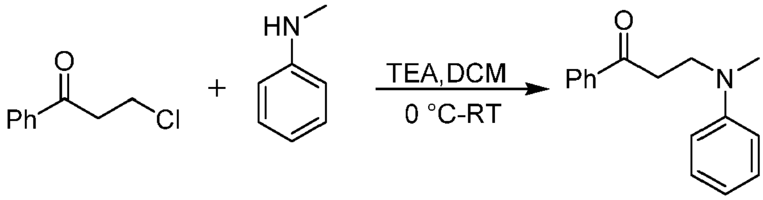


3-(methyl(phenyl)amino)-1-phenylpropan-1-one was prepared according to the procedure for Example W-4a, except that the pyrrolidine was replaced with ~1.1 eq. of N-methylaniline (~350 grams, ~3.27 mol), the reaction scaled to -496 grams (-2.94 mol) of 3-chloro-1-phenyl-1-propanone, and additional triethylamine was added to the reaction (~918 ml, ~6.53 mol, ~2.2 eq.). The crude 3-(methyl(phenyl)amino)-1-phenylpropan-1-one was used directly in the next reaction without further purification.

Example W-9b

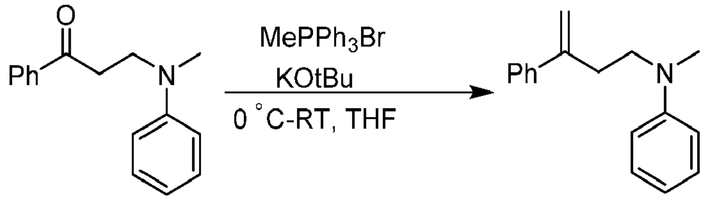


N-methyl-N-(3-phenylbut-3-en-1-yl)aniline was prepared according to the procedure for Example W-1b, except that the 3-morpholino-1-phenylpropan-1-one was replaced with ~1.0 eq. of 3-(methyl(phenyl)amino)-1-phenylpropan-1-one, the reaction scaled to -500 grams (-2.09 mol) of the starting ketone, and ~1.4 eq. (-2.92 mol) of solid potassium *tert*-butoxide used instead.

Furthermore, the crude material was triturated with hexanes (~10L) to remove residual triphenylphosphine oxide, then filtered, and solvent removed by rotary evaporation, prior to fractional distillation according to the below conditions.

The crude product (~475 grams) was distilled under vacuum and collected in 5 fractions. 4-*tert*-butyl catechol (~50 ppm) was added to the crude material before distillation and again to the distilled product fractions after the distillation was complete.

| fraction | temperature | vapor temperature | vacuum | quantity |
|---|---|---|---|---|
| 1 | ~110°C | ~46.8°C | ~1.8 mbar | ~16 g |
| 2 | ~120°C | ~54.6°C | ~1.8 mbar | ~13 g |
| 3 | ~160°C | ~126.3°C | ~1.8 mbar | ~11 g |
| 4 | ~180°C | ~145.8°C | ~1.8 mbar | ~250 g |
| 5 | ~190°C | ~149.1°C | ~1.9 mbar | ~26 g |

Fractions 3, 4, and 5 were found to contain impure N-methyl-N-(3-phenylbut-3-en-1-yl)aniline. Purification of the material by column chromatography (eluting with ~1-3% v/v ethyl acetate in hexanes) on silica gel (-60-120 mesh) yielded ~274 grams total of substantially pure N-methyl-N-(3-phenylbut-3-en-1-yl)aniline (-55% yield) as a yellow liquid.
$^1H$ NMR (~400 MHz, $CDCl_3$): $\delta$ ppm -7.44-7.39 (m, ~2H), -7.38-7.34 (m, ~3H), 7.33-7.23 (m, ~2H), -6.73-6.69 (m, ~3H), ~5.36 (d, $J \approx$ 1.2 Hz, ~1H), ~5.139-5.130 (m, ~1H), ~3.47 (t, $J \approx$ 7.6 Hz, ~2H), ~2.93 (s, ~3H), ~2.79 (t, $J \approx$ 7.2 Hz, ~2H); Mol. Formula: $C_{17}H_{19}N$; Mol. Wt. (calc): ~237.35 Da; Mass peak observed in LCMS: ~238.3 Da [M+1] $^+$.

Example W-lOa

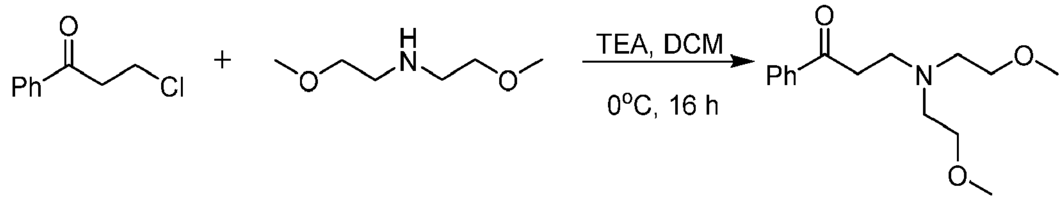


3-(bis(2-methoxyethyl)amino)-1-phenylpropan-1-one was prepared according to the procedure for Example W-4a, except that the pyrrolidine was replaced by ~1.0 eq. of bis(2-methoxyethyl)amine, and the reaction was scaled to -355 grams (-2.10 mol) of 3-chloro-1-phenyl-1-propanone. The crude 3-(bis(2-methoxyethyl)amino)-1-phenylpropan-1-one was used directly for the next reaction without further purification.

Example W-10b

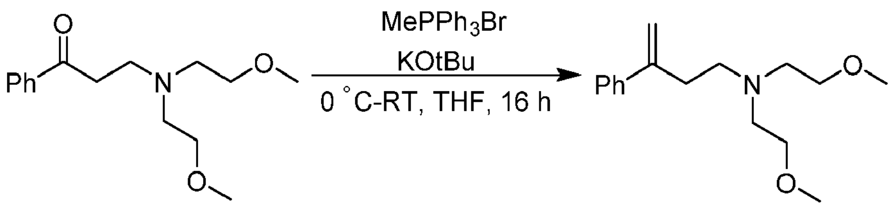


N,N-bis(2-methoxyethyl)-3-phenylbut-3-en-1-amine was prepared according to the procedure for Example W-1b, except that the 3-morpholino-1-phenylpropan-1-one was replaced with ~1.0 eq. of 3-(bis(2-methoxyethyl)amino)-1-phenylpropan-1-one, the reaction scaled to -578 grams (-2.18 mol) of the starting ketone, and ~1.4 eq. (-3.05 mol) of solid potassium *tert*-butoxide used instead.
Furthermore, the crude material was triturated with hexanes (~10L) to remove residual triphenylphosphine oxide, then filtered, and solvent removed by rotary evaporation, prior to fractional distillation according to the below conditions.
The crude product (~550 grams) was distilled under vacuum and collected in 5 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~180°C | ~140-145°C | ~2.0-2.3 mbar | ~49 g |
| 2 | | | | ~28 g |
| 3 | | | | ~87 g |
| 4 | | | | ~99 g |
| 5 | | | | ~53 g |

Fractions 2-5 were combined to yield ~267 grams of substantially pure N,N-bis(2-methoxyethyl)-3-phenylbut-3-en-1-amine (-46% yield) as a brown liquid.

$^1H$ NMR (~400 MHz, $CDCl_3$): $\delta$ ppm ~7.44-7.41 (m, ~2H), ~7.36-731 (m, ~2H), -7.30-7.26 (m, ~1H), ~5.32 (d, $J \approx 1.2$ Hz, ~1H), ~5.10 (d, $J \approx 1.2$ Hz, 1H), ~3.45 (t, $J \approx 6.0$ Hz, ~4H), ~3.34 (s, ~6H), ~2.76 (t, $J \approx 6.0$ Hz, ~4H), ~2.70 (s, ~4H); Mol. Formula: $C_{16}H_{25}NO_2$; Mol. Wt (calc): ~263.38 Da; Mass peak observed in LCMS: ~264.2 Da $[M+1]^+$.

Example W-11a

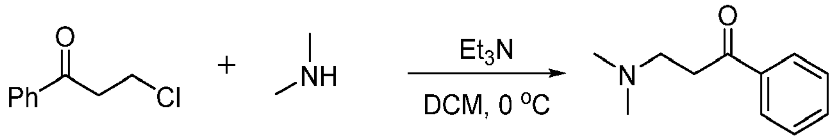


3-(dimethylamino)-1-phenylpropan-1-one was prepared according to the procedure for Example W-4a, except that the pyrrolidine was replaced by ~1.0 eq. of a -40% w/w aqueous solution of dimethylamine, and the reaction was scaled to -250 grams (-1.48 mol) of 3-chloro-1-phenyl-1-propanone. The crude 3-(dimethylamino)-1-phenylpropan-1-one was used directly for the next reaction without further purification. Additional batches were carried out at -250 grams and -300 grams scale based on 3-chloro-1-phenyl-1-propanone.

Example W-11b

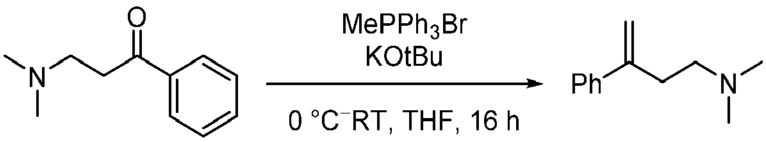


N,N-dimethyl-3-phenylbut-3-en-1-amine was prepared according to the procedure for Example W-1b, except that the 3-morpholino-1-phenylpropan-1-one was replaced with ~1.0 eq. of 3-(dimethylamino)-1-phenylpropan-1-one, the reaction scaled to -530 grams (-3.03 mol) of the starting ketone, and ~1.4 eq. (-4.24 mol) of solid potassium *tert*-butoxide used instead.

Furthermore, instead of fractional distillation, the crude material was triturated with hexane (~10L) to remove residual triphenylphosphine oxide, then filtered, and solvent removed by rotary evaporation, prior to column chromatography (eluting with ~30-50% v/v ethyl acetate in hexanes) on silica gel (~100-200 mesh). The reaction yielded -246 grams total of substantially pure N,N-dimethyl-3-phenylbut-3-en-1-amine (-46% yield) as a yellow liquid.

$^1H$ NMR (~400 MHz, $CDCl_3$): $\delta$ ppm -7.46-7.43 (m, ~2H), -7.37-7.27 (m, ~3H), ~5.34 (d, $J \approx 1.6$ Hz, ~1H), ~5.13 (d, $J \approx 1.2$ Hz, ~1H), -2.74-2.70 (m, ~2H), ~2.45-2.41 (m, ~2H), ~2.28 (s, ~6H); Mol. Formula: $C_{12}H_{17}N$; Mol. Wt. (calc): ~175.28 Da; Mass peak observed in LCMS: ~176.1 Da $[M+1]^+$.

Example W-12a

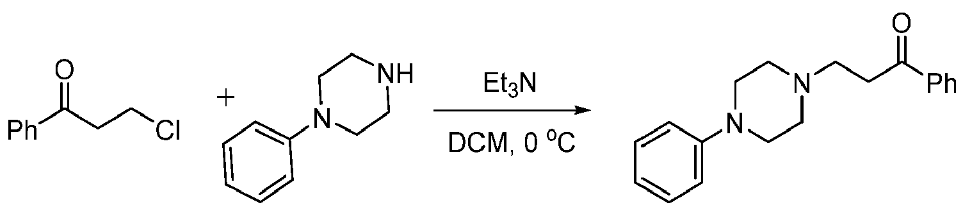


1-Phenyl-3-(4-phenylpiperazin-1-yl)propan-1-one was prepared according to the procedure for Example W-4a (at the same scale,- 2.97 mol), except that the pyrrolidine was replaced by ~1.0 eq. of 1-phenylpiperazine and ~3.0 eq. of triethylamine was added (-8.92 mol). The crude 1-phenyl-3-(4-phenylpiperazin-1-yl)propan-1-one was used directly

for the next reaction without further purification.

Example W-12b

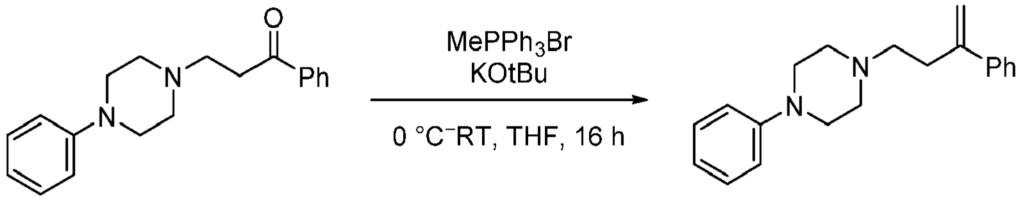


1-phenyl-4-(3-phenylbut-3-en-1-yl)piperazine was prepared according to the procedure for Example W-1b, except that the 3-morpholino-1-phenylpropan-1-one was replaced with ~1.0 eq. of 1-phenyl-3-(4-phenylpiperazin-1-yl)propan-1-one, the reaction scaled to -600 grams (~2.04 mol) of the starting ketone, and ~1.4 eq. (-2.85 mol) of solid potassium *tert*-butoxide used instead.

Furthermore, instead of fractional distillation, the crude material was triturated with hexane (~10L) to remove residual triphenylphosphine oxide, then filtered, and solvent removed by rotary evaporation, prior to column chromatography (eluting with -15% v/v ethyl acetate in hexanes) on silica gel (-60-120 mesh). The material was dissolved in methyl tert-butyl ether (~500 mL), and a solution of ~4.0 M HCl in 1,4-dioxane was added dropwise at 0-5 °C until no further precipitate was formed. The solid precipitate was filtered, dissolved in water, basified with 10% aqueous sodium bicarbonate solution. The product was extracted with dichloromethane (~3.0 L, twice). The organic layer was dried over $Na_2SO_4$, filtered, and solvent removed via rotary evaporation. As the impurities are not completely removed, the crude compound was again purified by column chromatography (eluting with -20% ethyl acetate in hexanes) on silica gel (-60-120 mesh)yielding ~240 grams of substantially pure 1-phenyl-4-(3-phenylbut-3-en-1-yl)piperazine (-40% yield) as an off-white solid.

$^1$H NMR (~400 MHz, $CDCl_3$): $\delta$-7.51-7.49 (m, ~2H), -7.41-7.38 (m, ~2H), -7.36-7.28 (m, ~1H), -7.00-6.90 (m, ~2H), ~6.41 (d, $J \approx 7.6$ Hz, 1H), ~5.40 (d, $J \approx 8$ Hz, ~1H), ~5.20 (d, $J \approx 8$ Hz, ~1H), ~3.27 (m, $J \approx 4.8$ Hz, ~2H), ~2.83 (t, $J \approx 8$ Hz, 2H), ~2.71 (t, $J \approx 5.2$ Hz, ~2H), -2.63-2.58 (m,~2H); Mol. Formula: $C_{20}H_{24}N2$; Mol. Wt. (calc): ~292.43 Da, Mass peak observed in LCMS: ~293.3 Da $[M+1]^+$.

Example W-13a

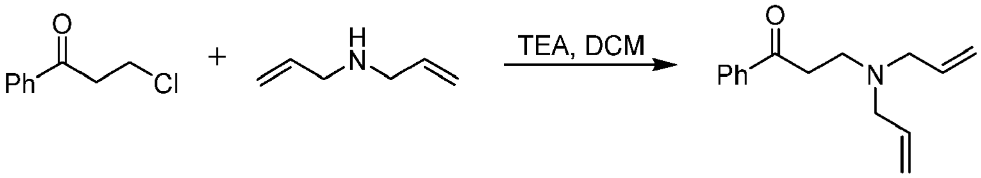


3-(diallylamino)-1-phenylpropan-1-one was prepared according to the procedure for Example W-4a (at the same scale, ~2.97 mol), except that the pyrrolidine was replaced by ~1.0 eq. of diallylamine and ~3.0 eq. of triethylamine was added (-8.96 mol). The crude 3-(diallylamino)-1-phenylpropan-1-one was used directly for the next reaction without further purification.

Example W-13b

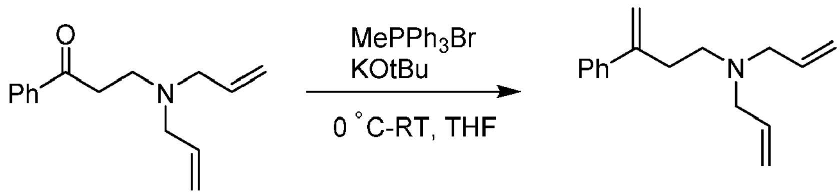


N,N-diallyl-3-phenylbut-3-en-1-amine was prepared according to the procedure for Example W-1b, except that the 3-morpholino-1-phenylpropan-1-one was replaced with ~1.0 eq. of 3-(diallylamino)-1-phenylpropan-1-one, the reaction scaled to -500 grams (-2.18 mol) of the starting ketone and ~1.4 eq. (-2.85 mol) of solid potassium *tert*-butoxide used instead.

Furthermore, the crude material was triturated with hexanes (~10L) to remove residual triphenylphosphine oxide, then filtered, and solvent removed by rotary evaporation, prior to fractional distillation according to the below conditions.

The crude product (~470 grams) was distilled under vacuum and collected in 5 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~120°C | ~95°C | ~3.0 mbar | ~38 g |
| 2 | ~120°C | ~95°C | ~2.8 mbar | ~41 g |
| 3 | ~140°C | ~110°C | ~2.5 mbar | ~250 g |
| 4 | ~140°C | ~110°C | ~2.5 mbar | |
| 5 | ~140°C | ~110°C | ~2.5 mbar | |

Fractions 3, 4, and 5 were found to contain substantially pure N-methyl-N-(3-phenylbut-3-en-1-yl)aniline and yielded a total of ~250 grams the product as a (-51% yield) as a brown liquid.

$^1H$ NMR (~400 MHz, $CDCl_3$): δ -7.46-7.43 (m, ~2H), ~7.37-7.27 (m, ~3H), -5.91-5.84 (m, ~2H), -5.34-5.11 (m, ~6H), -3.18-3.15 (m, ~4H), -2.73-2.69 (m, ~2H), -2.64-2.61 (m, ~2H). Mol. Formula $C_{16}H_{21}N$, Mol. Wt. (calc.): ~227.35 Da; Mass peak observed in LCMS: ~228.5 Da $[M+1]^+$.

Example W-14a

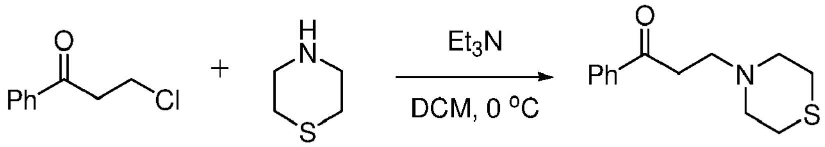


1-phenyl-3-thiomorpholinopropan-1-one was prepared according to the procedure for Example W-4a, except that the pyrrolidine was replaced by ~1.0 eq. of thiomorpholine, the reaction was scaled to ~250 grams of 3-chloro-1-phenyl-1-propanone (-1.48 mol), and ~3.0 eq. of triethylamine was added (-8.96 mol). The crude 1-phenyl-3-thiomorpholinopropan-1-one was used directly for the next reaction without further purification.

Example W-14b

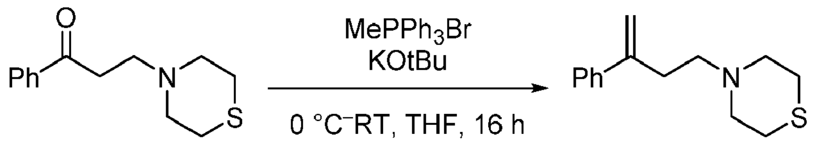


4-(3-phenylbut-3-en-1-yl)thiomorpholine was prepared according to the procedure for Example W-1b, except that the 3-morpholino-1-phenylpropan-1-one was replaced with ~1.0 eq. of 1-phenyl-3-thiomorpholinopropan-1-one, the reaction scaled to ~350 grams (-1.49 mol) of the starting ketone and ~1.4 eq. (-2.09 mol) of solid potassium *tert*-butoxide used instead. A second batch of the reaction was prepared at the same scale and mixed with the above batch prior to extraction and further purification.

Furthermore, the extracted material was triturated with hexanes (~10L) to remove residual triphenylphosphine oxide, then filtered, and solvent removed by rotary evaporation, prior to fractional distillation according to the below conditions.

The crude product (~700 grams) was distilled under vacuum and collected in 5 fractions. 4-*tert*-butyl catechol (~60 ppm) was added to the crude material before distillation and again to the distilled product fractions after the distillation was complete.

| fraction | temperature | vapor temperature | vacuum | quantity |
|---|---|---|---|---|
| 1 | ~150°C | ~110°C | ~3.0 mbar | ~18 g |
| 2 | ~150°C | ~110°C | ~3.0 mbar | ~150 g |
| 3 | ~140°C | ~110°C | ~2.5 mbar | ~180 g |
| 4 | ~140°C | ~110°C | ~2.5 mbar | ~200 g |
| 5 | ~140°C | ~110°C | ~2.5 mbar | ~110g |

Fractions 3 and 4 were found to contain substantially pure 4-(3-phenylbut-3-en-1-yl)thiomorpholine, and yielded a total of ~380 grams of the product (-52% yield) as a brown liquid.

$^1$H NMR (~400 MHz, $CDCl_3$): $\delta$ ppm -7.43-7.40 (m, ~2H), -7.32-7.26 (m, ~3H), ~5.31 (d, $J \approx$ *1.6,* ~1H), ~5.10 (dd, $J \approx$ *1.2* Hz, 1.6 Hz, ~1H), -2.77-2.67 (m, ~10H), -2.54-2.50 (m, -2H); Mol. Formula: $C_{14}H_{19}NS$; Mol. Wt. (calc): ~244.19 Da; Mass peak observed in LCMS: ~245.1 Da [M+1] $^+$.

Example W-15a

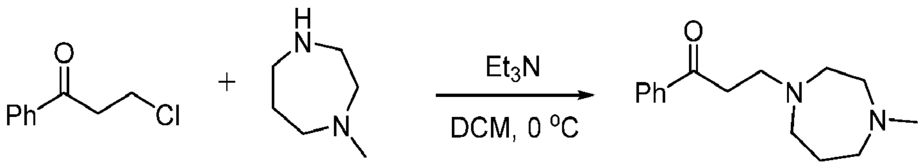


3-(4-methyl-1,4-diazepan-1-yl)-1-phenylpropan-1-one was prepared according to the procedure for Example W-4a, except that the pyrrolidine was replaced by ~1.0 eq. of 1-methyl homopiperazine, the reaction was scaled to ~330 grams of 3-chloro-1-phenyl-1-propanone (-1.96 mol), and ~3.0 eq. of triethylamine was added (-5.87 mol). The crude 3-(4-methyl-1,4-diazepan-1-yl)-1-phenylpropan-1-one was used directly for the next reaction without further purification.

Example W-15b

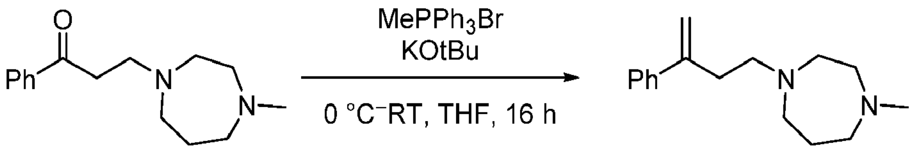


1-methyl-4-(3-phenylbut-3-en-1-yl)-1,4-diazepane was prepared according to the procedure for Example W-1b, except that the 3-morpholino-1-phenylpropan-1-one was replaced with ~1.0 equivalent of crude 3-(4-methyl-1,4-diazepan-1-yl)-1-phenylpropan-1-one, the reaction scaled to ~310 grams (-1.26 mol) of the starting ketone and ~1.4 eq. (-1.76 mol) of solid potassium *tert*-butoxide used instead.

Furthermore, instead of fractional distillation, the crude material was triturated with hexane (~10L) to remove residual triphenylphosphine oxide, then filtered, and solvent removed by rotary evaporation, prior to column chromatography (eluting with -5% v/v ethyl acetate in hexanes) on neutral alumina. The reaction yielded ~260 grams total of substantially pure 1-methyl-4-(3-phenylbut-3-en-1-yl)-1,4-diazepane (-58% yield) as a brown liquid.

$^1$H NMR (~400 MHz, $CDCl_3$): $\delta$ ppm -7.41-7.40 (m, ~2H), ~7.35-7.31 (m, ~2H), -7.29-7.27 (m, ~1H) -5.31 (d, $J \approx$ 1.6 Hz, ~1H), ~5.09 (d, $J \approx$ 1.2 Hz, ~1H), -2.78-2.72 (m, ~4H), -2.70-2.68 (m, ~2H), -2.65-2.60 (m, ~6H), ~2.36 (m, ~3H), -1.84-1.78 (m, -2H); Mol. Formula: $C_{16}H_{24}N_2$;Mol. Wt. (calc): 244.19 Da; Mass peak observed in LCMS: 245.1 Da $[M+1]^+$.

Example W-16a

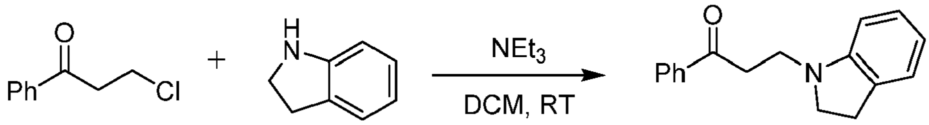


3-(indolin-1-yl)-1-phenylpropan-1-one was prepared according to the procedure for Example W-4a, except that the pyrrolidine was replaced by ~1.0 eq. of indoline, the reaction was scaled to ~564 grams of 3-chloro-1-phenyl-1-propanone (-3.36 mol), and ~3.0 eq. of triethylamine was added (-10.8 mol). The crude 3-(indolin-1-yl)-1-phenylpropan-1-one was used directly for the next reaction without further purification.

Example W-16b

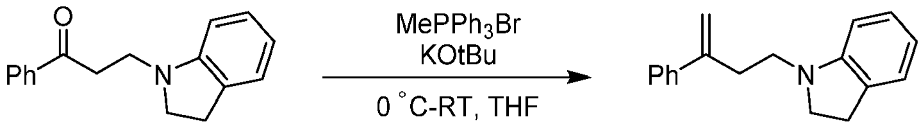


1-(3-phenylbut-3-en-1-yl)indoline was prepared according to the procedure for Example W-1b, except that the 3-morpholino-1-phenylpropan-1-one was replaced with ~1.0 equivalent of crude 3-(indolin-1-yl)-1-phenylpropan-1-one, the reaction scaled to ~600 grams (-2.39 mol) of the starting ketone and ~1.5 eq. (-3.54 mol) of solid potassium *tert*-butoxide used instead.

Furthermore, instead of fractional distillation, the crude material was triturated with hexane (~10L) to remove residual triphenylphosphine oxide, then filtered, and solvent removed by rotary evaporation, prior to column chromatography (eluting with -5% v/v ethyl acetate in hexanes) on neutral alumina. The reaction yielded ~250 grams total of substantially pure 1-(3-phenylbut-3-en-1-yl)indoline (-42% yield) as a brown liquid.

$^1$H NMR (~400 MHz, $CDCl_3$): $\delta$ -7.50-7.48 (m, ~2H), -7.42-7.40 (m, ~2H), -7.38-7.34 (m, ~1H), -7.12-7.06 (m, ~2H), -6.69-6.65 (m, ~1H), ~6.43-6.41 (d, J $\approx$ 7.6 Hz, ~1H), ~5.42 (d, $J \approx$ 8 Hz, ~1H), ~5.21 (d, $J \approx$ 12 Hz, ~1H), ~3.45-3.41 (m, ~2H), -3.28-3.24 (m, ~2H), -3.02-2.98 (m, ~2H), -2.87-2.83 (m,~2H); Mol. Formula $C_{18}H_{19}N$, Mol. Wt. (calc.): 249.36 Da, Mass peak observed in LCMS: 250.3 Da $[M+1]^+$.

Reaction W-17a

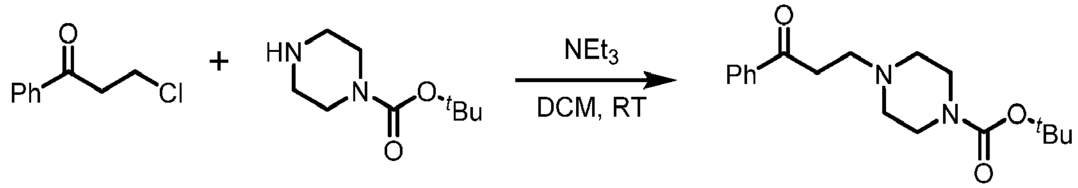


1-phenyl-3-(piperazin-1-yl)propan-1-one was prepared according to the procedure for Example W-4a, except that the pyrrolidine is replaced by ~1.0 eq. of tert-butyl piperazine-1-carboxylate, the reaction was scaled appropriately for ~430 g of the starting ketone, and ~2.2 eq. of triethylamine was added. The crude tert-butyl 4-(3-oxo-3-phenylpropyl)piperazine-1-carboxylate was used directly for the next reaction without further purification.

Reaction W-17b

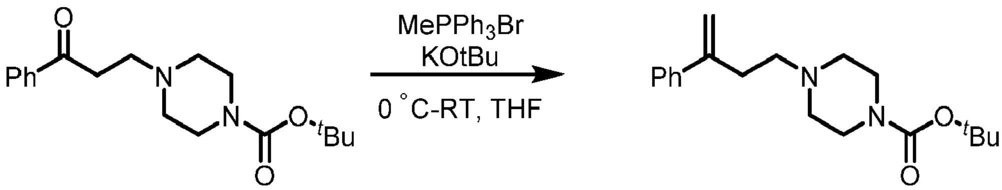


1-(3-phenylbut-3-en-1-yl)piperazine was prepared according to the procedure for Example W-1b, except that the 3-morpholino-1-phenylpropan-1-one was replaced with ~1.0 equivalent of crude tert-butyl 4-(3-oxo-3-phenylpropyl)piperazine-1-carboxylate, the reaction was scaled appropriately for ~800 g of the starting ketone, and ~2.0 eq. of solid potassium tert-butoxide was used instead. The crude product was further purified by column chromatography using neutral silica-gel and eluted with -30% EtOAc/hexane to yield substantially pure tert-butyl 4-(3-phenylbut-3-en-1-yl)piperazine-1-carboxylate (~410 g, -50% yield) as pale yellow liquid.

Reaction W-17c

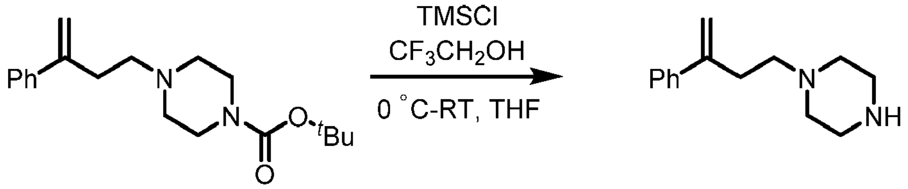


To a stirred solution of tert-butyl 4-(3-phenylbut-3-en-1-yl)piperazine-1-carboxylate (~410 g, ~1.297 mol) in 2,2,2-trifluoroethanol (-2.0 L), trimethylsilylchloride (~410 mL, ~1 vol) was added at 0 °C. The resulting reaction mixture was stirred at ambient temperature for ~2 h. After complete conversion (monitored by TLC), reaction mixture was concentrated under reduced pressure. The residue was diluted with water (-5.0 L) and washed with hexane (-5.0 L), layers were separated, and the aqueous the layer was basified with 10% aqueous sodium bicarbonate solution and extracted twice with DCM (2× ~5.0 L). The organic extract was dried over anhydrous $Na_2SO_4$, filtered, and concentrated under reduced pressure. The crude compound was dissolved in DCM and washed twice with water (2× ~5.0 L) to remove trifluoroethanol traces, dried over $Na_2SO_4$, and concentrated under reduced pressure. Finally, it was co-distilled with toluene to get desired product (~240 g, -86% yield) as a pale yellow solid.

1H-NMR (400 MHz, $CDCl_3$): δ ppm 7.41-7.30 (m, 2H), 7.27-7.24 (m, 3H), 5.30 (d, J = 0.8 Hz, 1H), 5.09 (d, J = 0.8 Hz, 1H), 3.47-3.45 (m, 2H), 4.78 (bs, 1H), 2.96 (t, J = 5.0 Hz, 4H), 2.73-2.69 (m, 2H), 2.52-2.35 (m, 4H).

Reaction W-18a

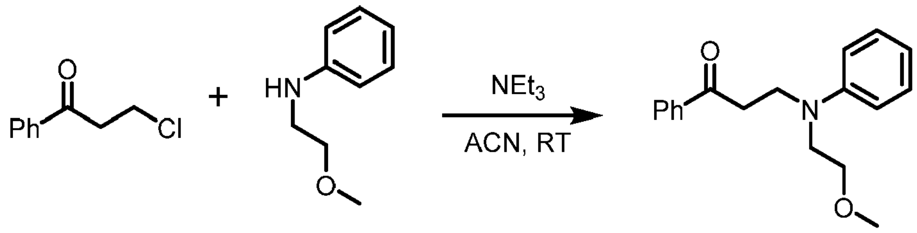


3-((2-methoxyethyl)(phenyl)amino)-1-phenylpropan-1-one was prepared according to the procedure for Example W-4a, except that the pyrrolidine was replaced by ~1.0 eq. of N-(2-methoxyethyl)aniline, the dichloromethane was replaced with acetonitrile, the reaction was scaled appropriately for ~100g of the starting ketone, and ~2.2 eq. of triethylamine was added instead. The crude 3-((2-methoxyethyl)(phenyl)amino)-1-phenylpropan-1-one was used directly for the next reaction without further purification.

Reaction W-18b

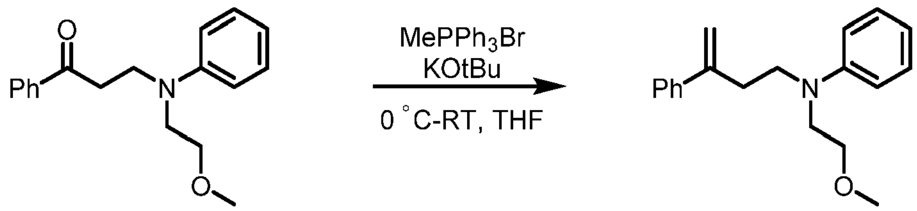


N-(2-methoxyethyl)-N-(3-phenylbut-3-en-1-yl)aniline was prepared according to the procedure for Example W-1b, except that the 3-morpholino-1-phenylpropan-1-one was replaced with ~1.0 equivalent of crude 3-((2-methoxyethyl)(phenyl)amino)-1-phenylpropan-1-one, the reaction was scaled appropriately for ~220 g of the starting ketone, and ~1.4 eq. of solid potassium *tert*-butoxide was used instead.

Furthermore, the crude material was triturated with hexane to remove residual triphenylphosphine oxide, then filtered, and solvent removed by rotary evaporation. The crude product was purified by silica-gel (60-120 mesh) column chromatography using -3% EtOAc in hexane to yield substantially pure N-(2-methoxyethyl)-N-(3-phenylbut-3-en-1-yl)aniline (166 g, -76% yield) as a light yellow liquid.

1H NMR (400 MHz, $CDCl_3$): δ ppm 7.49-7.47 (m, 2H), 7.41-7.37 (m, 2H), 7.34-7.32 (m, 1H), 7.28-7.23 (m, 2H), 6.71 (dd, J = 6,4.0 Hz, 2H), 5.38 (d, J =1.2 Hz, 1H),5.15 (d, J =1.2 Hz, 1H), 3.55-3.47 (m, 6H), 3.37 (s, 3H), 2.82 (t, J = 7.6 Hz, 2H).

Reaction W-19a

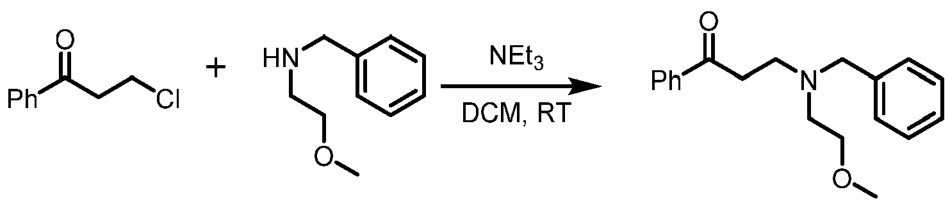


3-(benzyl(2-methoxyethyl)amino)-1-phenylpropan-1-one was prepared according to the procedure for Example W-4a, except that the pyrrolidine was replaced by ~1.0 eq. of N-benzyl-2-methoxyethan-1-amine, the reaction was scaled appropriately for ~357 g of the starting ketone, and ~2.0 eq. of triethylamine was added instead. The crude 3-(benzyl(2-methoxyethyl)amino)-1-phenylpropan-1-one is used directly for the next reaction without further purification.

Reaction W-19b

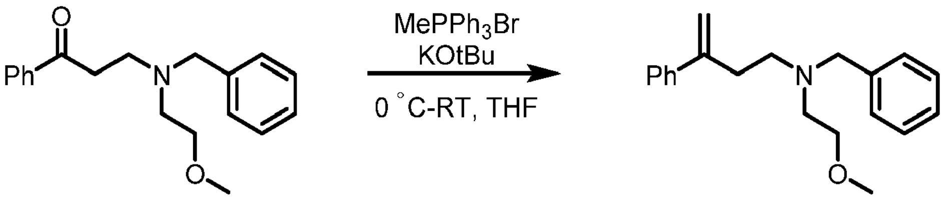


N-benzyl-N-(2-methoxyethyl)-3-phenylbut-3-en-1-amine was prepared according to the procedure for Example W-1b, except that the 3-morpholino-1-phenylpropan-1-one is replaced with ~1.0 equivalent of 3-(benzyl(2-methoxyethyl)amino)-1-phenylpropan-1-one, the reaction is scaled appropriately for ~620 g of the starting ketone, and ~1.4 eq. of solid potassium *tert*-butoxide is used instead. Furthermore, the crude material was triturated with hexane to remove residual triphenylphosphine oxide, then filtered, and solvent removed by rotary evaporation. The crude was purified by column chromatography (100-200 mesh silica gel) with EtOAc-hexane (2-8%) as an eluent to afford N-benzyl-N-(2-methoxyethyl)-3-phenylbut-3-en-1-amine (~355 g, -58% yield) as a pale-yellow liquid.

1H-NMR (400 MHz, $CDCl_3$): δ ppm 7.40-7.25 (m, 10H), 5.32 (d, J = 1.6 Hz, 1H), 5.08 (d, J = 0.8 Hz, 1H), 3.70 (s, 2H), 3.42 (t, J = 6.4 Hz, 2H), 3.30 (s, 3H), 2.74-2.67 (m, 6H).

Reaction W-20a

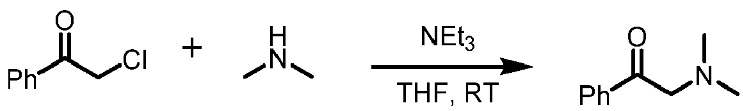


2-(dimethylamino)-1-phenylethan-1-one was prepared according to the procedure for Example W-4a, except that the 3-chloro-1-phenyl-1-propanone was replaced by ~1.0 eq. of 2-chloro-1-phenylethan-1-one, the pyrrolidine was replaced with ~1.5 eq. of dimethylamine, the reaction was scaled appropriately for ~250 g of the starting ketone, ~2.0 eq. of triethylamine was added instead, and dichloromethane was replaced with THF. The reaction was preformed twice at this scale and the crude products combined before they were purified by silica-gel (60-120 mesh) column chromatography using 15% EtOAc in hexane to yield substantially pure 2-(dimethylamino)-1-phenylethan-1-one (~370 g, -70% yield) as a dark brown liquid.

Reaction W-20b

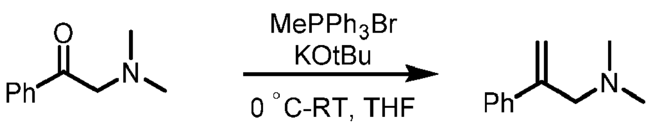


N,N-dimethyl-2-phenylprop-2-en-1-amine was prepared according to the procedure for Example W-1b, except that the 3-morpholino-1-phenylpropan-1-one was replaced with ~1.0 equivalent of 2-(dimethylamino)-1-phenylethan-1-one, the reaction was scaled appropriately for ~170 g of the starting ketone, and ~1.5 eq. of solid potassium tert-butoxide was used instead.

Furthermore, the crude material was triturated with hexane to remove residual triphenylphosphine oxide, then filtered, and solvent removed by rotary evaporation. The crude product was purified by silica-gel (60-120 mesh) column chromatography using 10-15% EtOAc in hexane to afford N,N-dimethyl-2-phenylprop-2-en-1-amine (~145 g, 85% yield) as a dark brown liquid.

$^1H$ NMR (400 MHz, $CDCl_3$): *δ* ppm 7.54-7.51 (m, 2H), 7.38-7.34 (m, 2H), 7.31-7.28 (m, 1H), 5.47 (d, *J* = 1.6 Hz, 1H), 5.25-5.24 (m, 1H), 2.26 (s, 6H).

Reaction W-21a

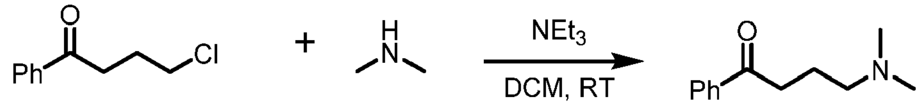


4-(dimethylamino)-1-phenylbutan-1-one is prepared according to the procedure for Example W-4a, except that the 3-chloro-1-phenyl-1-propanone is replaced by ~1.0 eq. of 4-chloro-1-phenylbutan-1-one, the reaction is scaled appropriately for the quantity of starting material, and ~3.0 eq. of triethylamine is added. The crude 4-(dimethylamino)-1-phenylbutan-1-one is used directly for the next reaction without further purification.

Reaction W-21b

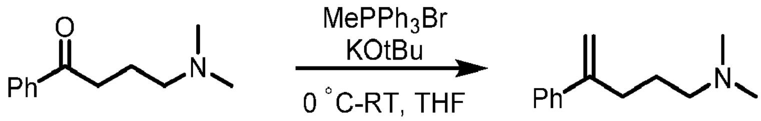


N,N-dimethyl-4-phenylpent-4-en-1-amine is prepared according to the procedure for Example W-1b, except that the 3-morpholino-1-phenylpropan-1-one is replaced with ~1.0 equivalent of 4-(dimethylamino)-1-phenylbutan-1-one, the reaction is scaled appropriately for the quantity of starting material, and ~1.4 eq. of solid potassium *tert*-butoxide is used instead.

Furthermore, the crude material is triturated with hexane to remove residual triphenylphosphine oxide, then filtered, and solvent removed by rotary evaporation. The product is optionally purified by fractional (vacuum) distillation (as in the purification steps of Example W-1b) and/or column chromatography (eluting with a solvent comprising ethyl acetate and/or dichloromethane and/or hexanes) on either silica gel or neutral alumina (as in the purification steps of Example W-3b).

Reaction W-22a

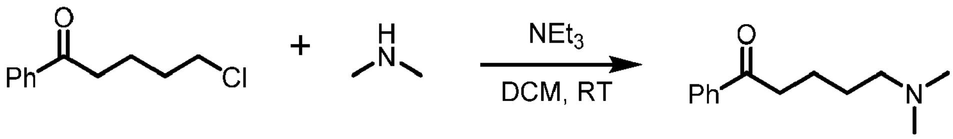


5-(dimethylamino)-1-phenylpentan-1-one is prepared according to the procedure for Example W-4a, except that the 3-chloro-1-phenyl-1-propanone is replaced by ~1.0 eq. of 5-chloro-1-phenylpentan-1-one, the reaction is scaled appropriately for the quantity of starting material, and ~3.0 eq. of triethylamine is added. The crude 5-(dimethylamino)-1-phenylpentan-1-one is used directly for the next reaction without further purification.

Reaction W-22b

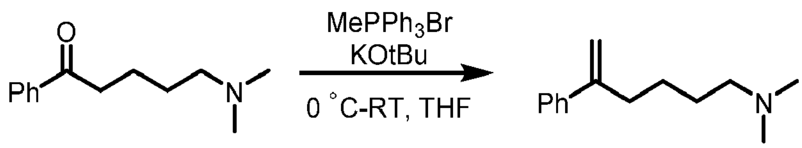


N,N-dimethyl-5-phenylhex-5-en-1-amine is prepared according to the procedure for Example W-1b, except that the 3-morpholino-1-phenylpropan-1-one is replaced with ~1.0 equivalent of 5-(dimethylamino)-1-phenylpentan-1-one, the reaction is scaled appropriately for the quantity of starting material, and ~1.4 eq. of solid potassium *tert*-butoxide is used instead.

Furthermore, the crude material is triturated with hexane to remove residual triphenylphosphine oxide, then filtered, and solvent removed by rotary evaporation. The product is optionally purified by fractional (vacuum) distillation (as in the purification steps of Example W-1b) and/or column chromatography (eluting with a solvent comprising ethyl acetate and/or dichloromethane and/or hexanes) on either silica gel or neutral alumina (as in the purification steps of

Example W-3b).

Reaction W-23a

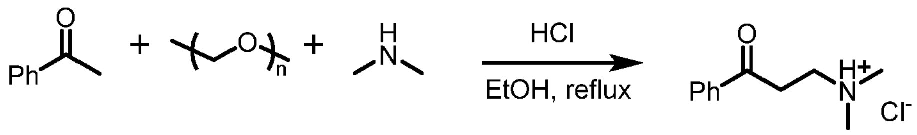


Dimethylamine hydrochloride (17.7 g, 0.219 mol), acetophenone (25 g, 0.208 mol) and paraformaldehyde (6.9 g, 0.229 mol formaldehyde) were added to an appropriately sized reactor equipped with a reflux condenser. Ethanol (33 ml) was added, followed by HCl (conc, 37%) (0.90 ml, 0.031 mol). The reaction mixture was heated to reflux (~78 °C) for 5h. The reaction mixture was allowed to cool to ambient temperature. The solid precipitate was filtered, washed with two portions of cold acetone (2× 40 ml) and dried. The filtrate, including the acetone wash, was diluted with ~50 ml heptane, and allowed to sit at ambient temperature overnight. The additional precipitate was filtered, dried, and combined with the above material yielding substantially pure 3-(dimethylamino)-1-phenylpropan-1-one hydrochloride (20.8 g, 46% yield).

Reaction W-23b

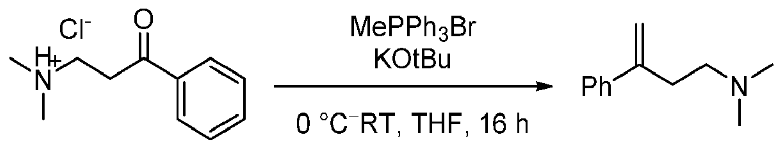


The reaction is carried out according to the procedure for Example W-3b, except that ~2.5 e.q. of potassium *tert*-butoxide is used instead of ~1.5 e.q.

Reaction W-24a

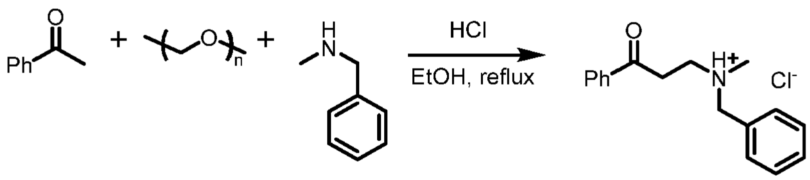


N-methyl-1-phenylmethanamine (10.09 g, 83 mmol) and ethanol (20 ml) were added to an appropriately sized reactor equipped with a reflux condenser. HCl (conc, 37%) (7.86 ml, 96 mmol) was added dropwise over 10 mins, resulting in an exotherm. The solution was allowed to cool to below 30°C, after which acetophenone (10 g, 83 mmol) was added, followed by paraformaldehyde (3.75 g, 125 mmol formaldehyde). The mixture was heated to reflux (~78 °C) for ~5h. The mixture was allowed to cool to ambient temperature overnight, under nitrogen. The solid precipitate was then filtered, washed with two portions of cold acetone (2× 20 ml) and dried under vacuum, yielding substantially pure 3-(benzyl(methyl)amino)-1-phenylpropan-1-one hydrochloride (19.90 g, 83% yield).

Reaction W-24b

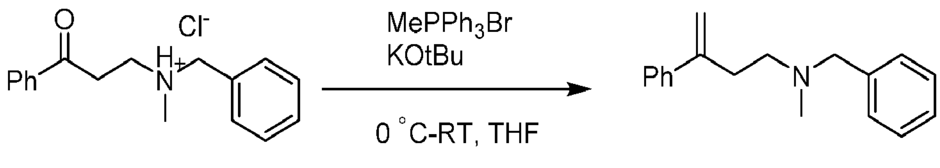


The reaction is carried out according to the procedure for Example W-11b, except that ~2.4 e.q. of potassium *tert*-butoxide is used instead of ~1.4 e.q.

Reaction W-25a

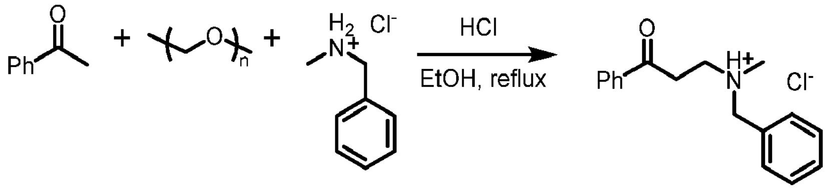


N-methyl-1-phenylmethanamine hydrochloride (6.56 g, 41.6 mmol), acetophenone (5 g, 41.6 mmol) and paraformaldehyde (1.874 g, 62.4 mmol formaldehyde) were added to an appropriately sized reactor equipped with a reflux condenser. Ethanol (14 ml) was added, followed by HCl (conc, 37%) (0.513 ml, 6.24 mmol). The reaction mixture was heated to reflux (~78 °C) for 6h. The reaction mixture was allowed to cool to ambient temperature. The solid precipitate was filtered, washed with two portions of cold acetone (2× 20 ml) and dried, yielding substantially pure 3-(benzyl(methyl)amino)-1-phenylpropan-1-one hydrochloride (9.05 g, 75% yield).

Reaction W-25b

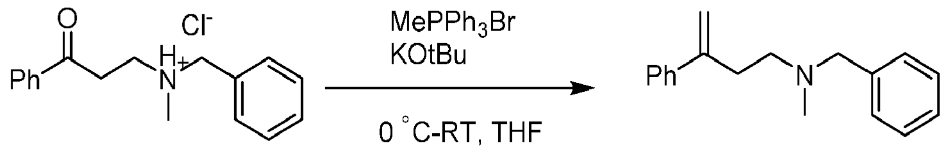


The reaction is carried out according to the procedure for Example W-3b, except that ~2.5 e.q. of potassium *tert*-butoxide is used instead of ~1.5 e.q.

All documents described herein are incorporated by reference herein, including any priority documents and/or testing procedures, to the extent they are not inconsistent with this text. As should be apparent from the foregoing general description and the specific embodiments, while forms of the invention have been illustrated and described, various modifications can be made without departing from the spirit and scope of the invention. Accordingly, it is not intended that the invention be limited thereby. The term "comprising" specifies the presence of stated features, steps, integers, or components, but does not preclude the presence or addition of one or more other features, steps, integers, components, or groups thereof. As such, the term "comprising" is considered essentially synonymous with the term "including." Similarly, whenever a composition, an element, or a group of elements is preceded with the transitional phrase "comprising," it should be understood that the same composition or group of elements is contemplated with transitional phrases "consisting essentially of," "consisting of," "selected from the group of consisting of," or "can be"/"may be"/"is" preceding the recitation of the composition, element, or elements, and *vice versa.* In juxtaposition to the well-known terms "comprising" meaning "including what follows and anything else" [open] and "consisting of' meaning "including only what follows" [closed], the term "consisting essentially of" should be understood to be semi-inclusive and to mean, in accordance with US judicial interpretation, including that which follows and other things that do not materially affect the basic and novel properties.

## Claims

1. An addition-polymerizable monomer composition comprising an amine-derivatized alpha-methyl styrene (ADAMS) monomer according to structure (I) and/or an aminated conjugated aliphatic methylated polyene (ACAMP) monomer according to structure (II):

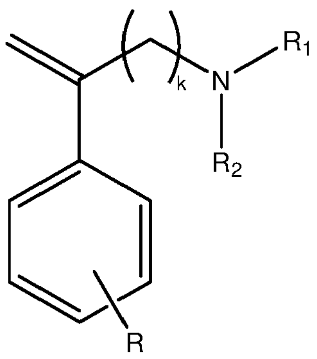

(I)

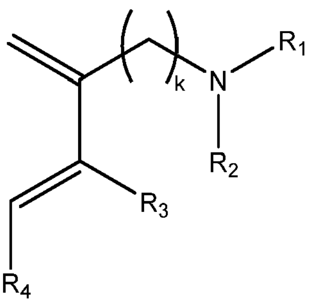

(II)

wherein:

k is an integer from 1 to 3;

$R_1$ and $R_2$ are each independently a hydrocarbyl group or a hydrocarbonaceous group having 1 to 4 additional heteroatoms selected from the group consisting of O, N, S, P, Se, and combinations thereof, or wherein $R_1$ and $R_2$ are connected to form a moiety containing at least one 5- to 12- membered ring, from 3 to 28 carbons, and optionally 1 to 6 additional heteroatoms selected from the group consisting of O, N, S, P, Se, and combinations thereof;

in structure (I), R is hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group, a $C_1$-$C_6$ hydrocarbyl group containing 1 to 4 additional heteroatoms selected from the group consisting of O, N, S, P, Se, and combinations thereof, or a second amino-functional group having structure (III)

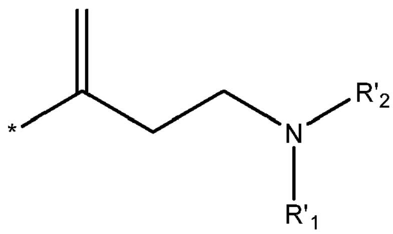

(III)

attached via the asterisk, with $R'_1$ and $R'_2$ being independently the same or different as $R_1$ and $R_2$ but defined identically; and

in structure (II), $R_3$ and $R_4$ are each independently hydrogen, a methyl group, a second amino-functional group having structure (III) attached via the asterisk, with $R'_1$ and $R'_2$ being independently the same or different as $R_1$ and $R_2$ but defined identically, or an alkenyl group having structure (IV)

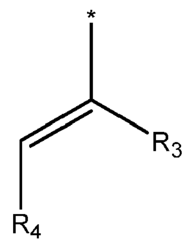

(IV)

attached via the asterisk, but specifically which do not collectively achieve aromaticity in combination with the other alkenes in structure (II), and with the proviso that $R_3$ and $R_4$ are not both alkenyl groups having structure (IV) and are not both methyl groups.

**2.** The addition-polymerizable monomer composition of claim 1, wherein the ADAMS monomer according to structure (I) comprises 1-dimethylamino-3-phenylbut-3-ene, 1-diethylamino-3-phenylbut-3-ene, 1-di-n-propylamino-3-phenylbut-3-ene, 1-diisopropylamino-3-phenylbut-3-ene, 1-di-2-propenylamino-3-phenylbut-3-ene, 1-di-n-butylamino-3-phenylbut-3-ene, 1-di-sec-butylamino-3-phenylbut-3-ene, 1-diisobutylamino-3-phenylbut-3-ene, 1-di-tert-butylamino-3-phenylbut-3-ene, 1-cyclohexylmethylamino-3-phenylbut-3-ene, 1-dicyclohexylamino-3-phenylbut-3-ene, 1-di-(2-ethylhexyl)amino-3-phenylbut-3-ene, 1-di-(methoxyethyl)amino-3-phenylbut-3-ene, 1-di-(ethoxyethyl)amino-3-phenylbut-3-ene, 1-di-(phenoxyethyl)amino-3-phenylbut-3-ene, 1-di-(methylthioethyl)amino-3-phenylbut-3-ene, 1-di-(ethylthioethyl)amino-3-phenylbut-3-ene, 1-benzylmethylamino-3-phenylbut-3-ene, 1-dibenzylamino-3-phenylbut-3-ene, 1-benzylphenylamino-3-phenylbut-3-ene, 1-diphenylamino-3-phenylbut-3-ene, 1-dipyridylamino-3-phenylbut-3-ene, 1-phenylmethylamino-3-phenylbut-3-ene, 1-phenylmethoxyethylamino-3-phenylbut-3-ene, 1-benzylmethoxyethylamino-3-phenylbut-3-ene, 1-(N-morpholinyl)-3-phenylbut-3-ene, 1-(N-thiomorpholinyl)-3-phenylbut-3-ene, 1-(N-piperidinyl)-3-phenylbut-3-ene, 1-(N-piperazinyl)-3-phenylbut-3-ene, 1-(N-diazepanyl)-3-phenylbut-3-ene, 1-(N-pyrrolidinyl)-3-phenylbut-3-ene, 1-(N-pyrrolyl)-3-phenylbut-3-ene, 1-(1,2,3,4-tetrahydro-1-quinolinyl)-3-phenylbut-3-ene, 1-(1,2,3,4-tetrahydro-2-isoquinolinyl)-3-phenylbut-3-ene, 1-(N-indolinyl)-3-phenylbut-3-ene, 1-(N-indolyl)-3-phenylbut-3-ene, 1-(N-carbazolyl)-3-phenylbut-3-ene, 1-(N-phenothiazinyl)-3-phenylbut-3-ene, 1-(N-phenothiazinyl-S-oxide)-3-phenylbut-3-ene, 1-(N-phenothiazinyl-S,S-dioxide)-3-phenylbut-3-ene, 1-(N-phenoxazinyl)-3-phenylbut-3-ene, 1-(4-methyl-1-piperazinyl)-3-phenylbut-3-ene, 1-(5-methyl-2,5-diazabicyclo[2.2.1]heptan-2-yl)-3-phenylbut-3-ene, 1-(5-methyl-2,5-diazabicyclo[2.2.2]octan-2-yl)-3-phenylbut-3-ene, 1-(4-cyclopentyl-1-piperazinyl)-3-phenylbut-3-ene, 1-(4-cyclopentadienyl-1-piperazinyl)-3-phenylbut-3-ene, 1-(4-phenyl-1-piperazinyl)-3-phenylbut-3-ene, 1-(4-(thiazolyl)-1-piperazinyl)-3-phenylbut-3-ene, 1-(4-(thiadiazolyl)-1-piperazinyl)-3-phenylbut-3-ene, 1-(4-(triazolyl)-1-piperazinyl)-3-phenylbut-3-ene, 1-(4-(1,2,3-benzotriazolyl)-1-piperazinyl)-3-phenylbut-3-ene, 1-(N'-methyl-N-diazepanyl)-3-phenylbut-3-ene, N,N'-bis(3-phenylbut-3-enyl)diazepane, N,N'-bis(3-phenylbut-3-enyl)piperazine, N,N'-bis(3-phenylbut-3-enyl)dihydrophenazine, N,N'-bis(3-phenylbut-3-enyl)dihydrobenzoindazole, N,N'-bis(3-phenylbut-3-enyl)dihydropermidine, N,N'-bis(3-phenylbut-3-enyl)octahydropyridoquinoline, N,N'-bis(3-phenylbut-3-enyl)octahydropyridoisoquinoline, N,N'-bis(3-phenylbut-3-enyl)hexahydropyrroloquinoline, N,N'-bis(3-phenylbut-3-enyl)hexahydropyrroloisoquinoline, N,N'-bis(3-phenylbut-3-enyl)hexahydropyrroloisoindole, N,N'-bis(3-phenylbut-3-enyl)diazabicyclo[2.2.1]heptane, N,N' -bis(3 -phenylbut-3 - enyl)diazabicyclo [2.2.2] octane, 1,3-bis(1-(3 -phenylbut-3 -enyl)piperidin-4-yl)propane, bis(1-dimethylamino-3-phenylbut-3-enyl)benzene, bis(1-benzylmethylamino-3-phenylbut-3-enyl)benzene, bis(1-(N-morpholinyl)-3-phenylbut-3-enyl)benzene, bis(1-(N-thiomorpholinyl)-3-phenylbut-3-enyl)benzene, bis(1-(di-methoxyethyl)amino-3-phenylbut-3-enyl)benzene, bis(1-(N-piperidinyl)-3-phenylbut-3-enyl)benzene, bis(1-(N-pyrrolidinyl)-3-phenylbut-3-enyl)benzene, bis(1-(4-methyl-1-piperazinyl))-3-phenylbut-3-enyl)benzene, or a combination thereof.

**3.** The addition-polymerizable monomer composition of claim 1 or claim 2, comprising substantially none of 1-dimethylamino-3-phenylbut-3-ene, 1-diethylamino-3-phenylbut-3-ene, N-morpholinyl-3-phenylbut-3-ene, 1-benzylmethylamino-3-phenylbut-3-ene, 1-dimethylamino-3-p-tolylbut-3-ene, 1-diethylamino-3-p-tolylbut-3-ene, N-morpholinyl-3-p-tolylbut-3-ene, N-piperidinyl-3-p-tolylbut-3-ene, and N-pyrrolidinyl-3-p-tolylbut-3-ene.

**4.** The addition-polymerizable monomer composition of claim 1 or claim 2, comprising substantially no compounds in which $R_1$ and $R_2$ are each independently a benzyl group or a $C_1$-$C_{12}$ alkyl group, and comprising substantially no compounds in which $R_1$ and $R_2$ are connected to form a singular unsubstituted nitrogen-containing 5- or 6- membered heterocyclic ring.

**5.** The addition-polymerizable monomer composition of any one of claims 1-4,

wherein an ADAMS monomer according to structure (I) comprises an exo isomer monomer, further comprising one or more of the following:

(1) at least 0.5 wt%, based on the reaction medium-free composition weight, of endo isomer monomer corresponding to the exo isomer monomer, optionally up to 15 wt%;
(2) not more than 12 wt%, based on the reaction medium-free composition weight, of saturation byproduct;
(3) at least 100 wppm, based on the reaction medium-free composition weight, of optionally ring substituted alpha-methylstyrene, optionally up to 5 wt%;
(4) not more than 500 wppm, based on the reaction medium-free composition weight, of hydroxylation byproduct; and
(5) not more than 1.0 wt%, based on the reaction medium-free composition weight, of dimerization/oligomerization byproduct.

**6.** The addition-polymerizable monomer composition of any one of claims 1-5, further comprising not more than 0.3 wt%, based on the reaction medium-free composition weight, of an acid anhydride.

**7.** The addition-polymerizable monomer composition of claim 1,
wherein the ACAMP monomer according to structure (II) comprises 1-dimethylamino-3-methylenepent-4-ene, 1-diethylamino-3-methylenepent-4-ene, 1-din-propylamino-3-methylenepent-4-ene, 1-diisopropylamino-3-methylenepent-4-ene, 1-di-2-propenylamino-3-methylenepent-4-ene, 1-di-n-butylamino-3-methylenepent-4-ene, 1-di-sec-butylamino-3-methylenepent-4-ene, 1-diisobutylamino-3-methylenepent-4-ene, 1-di-tert-butylamino-3-methylenepent-4-ene, 1-cyclohexylmethylamino-3-methylenepent-4-ene, 1-dicyclohexylamino-3-methylenepent-4-ene, 1-di-(2-ethylhexyl)amino-3-methylenepent-4-ene, 1-di-(methoxyethyl)amino-3-methylenepent-4-ene, 1-di-(ethoxyethyl)amino-3-methylenepent-4-ene, 1-di-(phenoxyethyl)amino-3-methylenepent-4-ene, 1-di-(methylthioethyl)amino-3-methylenepent-4-ene, 1-di-(ethylthioethyl)amino-3-methylenepent-4-ene, 1-benzylmethylamino-3-methylenepent-4-ene, 1-dibenzylamino-3-methylenepent-4-ene, 1-benzylphenylamino-3-methylenepent-4-ene, 1-diphenylamino-3-methylenepent-4-ene, 1-dipyridylamino-3-methylenepent-4-ene, 1-phenylmethylamino-3-methylenepent-4-ene, 1-phenylmethoxyethylamino-3-methylenepent-4-ene, 1-benzylmethoxyethylamino-3-methylenepent-4-ene, 1-(N-morpholinyl)-3-methylenepent-4-ene, 1-(N-thiomorpholinyl)-3-methylenepent-4-ene, 1-(N-piperidinyl)-3-methylenepent-4-ene, 1-(N-piperazinyl)-3-methylenepent-4-ene, 1-(N-diazepanyl)-3-methylenepent-4-ene, 1-(N-pyrrolidinyl)-3-methylenepent-4-ene, 1-(N-pyrrolyl)-3-methylenepent-4-ene, 1-(1,2,3,4-tetrahydro-1-quinolinyl)-3-methylenepent-4-ene, 1-(1,2,3,4-tetrahydro-2-isoquinolinyl)-3-methylenepent-4-ene, 1-(N-indolinyl)-3-methylenepent-4-ene, 1-(N-indolyl)-3-methylenepent-4-ene, 1-(N-carbazolyl)-3-methylenepent-4-ene, 1-(N-phenothiazinyl)-3-methylenepent-4-ene, 1-(N-phenothiazinyl-S-oxide)-3-methylenepent-4-ene, 1-(N-phenothiazinyl-S,S-dioxide)-3-methylenepent-4-ene, 1-(N-phenoxazinyl)-3-methylenepent-4-ene, 1-(4-methyl-1-piperazinyl)-3-methylenepent-4-ene, 1-(5-methyl-2,5-diazabicyclo[2.2.1]heptan-2-yl)-3-methylenepent-4-ene, 1-(5-methyl-2,5-diazabicyclo[2.2.2]octan-2-yl)-3-methylenepent-4-ene, 1-(4-cyclopentyl-1-piperazinyl)-3-methylenepent-4-ene, 1-(4-cyclopentadienyl-1-piperazinyl)-3-methylenepent-4-ene, 1-(4-phenyl-1-piperazinyl)-3-methylenepent-4-ene, 1-(4-(thiazolyl)-1-piperazinyl)-3-methylenepent-4-ene, 1-(4-(thiadiazolyl)-1-piperazinyl)-3-methylenepent-4-ene, 1-(4-(triazolyl)-1-piperazinyl)-3-methylenepent-4-ene, 1-(4-(1,2,3-benzotriazolyl)-1-piperazinyl)-3-methylenepent-4-ene, 1-(N'-methyl-N-diazepanyl)-3-methylenepent-4-ene, 1-dimethylamino-3-methylenehepta-4,6-diene, 1-diethylamino-3-methylenehepta-4,6-diene, 1-di-n-propylamino-3-methylenehepta-4,6-diene, 1-diisopropylamino-3-methylenehepta-4,6-diene, 1-di-2-propenylamino-3-methylenehepta-4,6-diene, 1-di-n-butylamino-3-methylenehepta-4,6-diene, 1-di-sec-butylamino-3-methylenehepta-4,6-diene, 1-diisobutylamino-3-methylenehepta-4,6-diene, 1-di-tert-butylamino-3-methylenehepta-4,6-diene, 1-cyclohexylmethylamino-3-methylenehepta-4,6-diene, 1-dicyclohexylamino-3-methylenehepta-4,6-diene, 1-di-(2-ethylhexyl)amino-3-methylenehepta-4,6-diene, 1-di-(methoxyethyl)amino-3-methylenehepta-4,6-diene, 1-di-(ethoxyethyl)amino-3-methylenehepta-4,6-diene, 1-di-(phenoxyethyl)amino-3-methylenehepta-4,6-diene, 1-di-(methylthioethyl)amino-3-methylenehepta-4,6-diene, 1-di-(ethylthioethyl)amino-3-methylenehepta-4,6-diene, 1-benzylmethylamino-3-methylenehepta-4,6-diene, 1-dibenzylamino-3-methylenehepta-4,6-diene, 1-benzylphenylamino-3-methylenehepta-4,6-diene, 1-diphenylamino-3-methylenehepta-4,6-diene, 1-dipyridylamino-3-methylenehepta-4,6-diene, 1-phenylmethylamino-3-methylenehepta-4,6-diene, 1-phenylmethoxyethylamino-3-methylenehepta-4,6-diene, 1-benzylmethoxyethylamino-3-methylenehepta-4,6-diene, 1-(N-morpholinyl)-3-methylenehepta-4,6-diene, 1-(N-thiomorpholinyl)-3-methylenehepta-4,6-diene, 1-(N-piperidinyl)-3-methylenehepta-4,6-diene, 1-(N-piperazinyl)-3-methylenehepta-4,6-diene, 1-(N-diazepanyl)-3-methylenehepta-4,6-diene, 1-(N-pyrrolidinyl)-3-methylenehepta-4,6-diene, 1-(N-pyrrolyl)-3-methylenehepta-4,6-diene, 1-(1,2,3,4-tetrahydro-1-quinolinyl)-3-methylenehepta-4,6-diene, 1-(1,2,3,4-tetrahydro-2-isoquinolinyl)-3-methylenehepta-4,6-diene, 1-(N-indolinyl)-3-methylenehepta-4,6-diene, 1-(N-indolyl)-3-methylenehepta-4,6-diene, 1-(N-carbazolyl)-3-methylenehepta-4,6-diene, 1-(N-phenothiazinyl)-3-methylenehepta-4,6-diene, 1-(N-phenothiazinyl-S-oxide)-3-methylenehepta-4,6-diene, 1-(N-phenothiazinyl-S,S-dioxide)-3-methylenehepta-4,6-diene, 1-(N-phenoxazinyl)-3-methylenehepta-4,6-diene, 1-(4-methyl-1-piperazinyl)-3-methylenehepta-4,6-diene, 1-(5-methyl-2,5-diazabicyclo[2.2.1]heptan-2-yl)-3-methylenehepta-4,6-diene, 1-(5-methyl-2,5-diazabicyclo[2.2.2]octan-2-yl)-3-methylenehepta-4,6-diene, 1-(4-cyclopentyl-1-piperazinyl)-3-methylenehepta-4,6-diene, 1-(4-cyclopentadienyl-1-piperazinyl)-3-methylenehepta-4,6-diene, 1-(4-phenyl-1-piperazinyl)-3-methylenehepta-4,6-diene, 1-(4-(thiazolyl)-1-piperazinyl)-3-methylenehepta-4,6-diene, 1-(4-(thiadiazolyl)-1-piperazinyl)-3-methylenehepta-4,6-diene, 1-(4-(triazolyl)-1-piperazinyl)-3-methylenehepta-4,6-diene, 1-(4-(1,2,3-benzotriazolyl)-1-piperazinyl)-3-methylenehepta-4,6-diene, 1-(N'-methyl-N-diazepanyl)-3-methylenehepta-4,6-diene, 1-dimethylamino-3,4-dimethylenehex-5-ene, 1-diethylamino-3,4-dimethylenehex-5-ene, 1-di-n-propylamino-3,4-dimethylenehex-5-ene, 1-diisopropylamino-3,4-dimethylenehex-5-ene, 1-di-2-propenylamino-3,4-dimethylenehex-5-ene, 1-di-n-butylamino-3,4-dimethylenehex-5-ene, 1-di-sec-butylamino-3,4-dimethylenehex-5-ene, 1-diisobutylamino-3,4-dimethylenehex-5-ene, 1-di-tert-butylamino-3,4-dimethylenehex-5-ene, 1-cyclohexylmethylamino-3,4-dimethylenehex-5-ene, 1-dicyclohexylamino-3,4-dimethylenehex-5-ene, 1-di-(2-ethylhexyl)amino-3,4-dimethylenehex-5-ene, 1-di-(methoxyethyl)amino-3,4-dimethylenehex-5-ene, 1-di-(ethoxyethyl)amino-3,4-dimethylenehex-5-ene, 1-di-(phenoxyethyl)amino-3,4-dimethylenehex-5-ene, 1-di-(methylthioethyl)amino-3,4-dimethylenehex-5-ene, 1-di-(ethylthioethyl)amino-3,4-dimethylenehex-5-ene, 1-benzylmethylamino-3,4-dimethylenehex-5-ene, 1-dibenzylamino-3,4-dimethylenehex-5-ene, 1-benzylphenylamino-3,4-dimethylenehex-5-ene, 1-diphenylamino-3,4-dimethylenehex-5-ene, 1-dipyridylamino-3,4-dimethylenehex-5-ene, 1-phenylmethylamino-3,4-dimethylenehex-5-ene, 1-phenylmethoxyethylamino-3,4-dimethylenehex-5-ene, 1-benzylmethoxyethylamino-3,4-dimethylenehex-5-ene, 1-(N-morpholinyl)-3,4-dimethylenehex-5-ene, 1-(N-thiomorpholinyl)-3,4-dimethylenehex-5-ene, 1-(N-piperidinyl)-3,4-dimethylenehex-5-ene, 1-(N-piperazinyl)-3,4-dimethylenehex-5-ene, 1-(N-diazepanyl)-3,4-dimethylenehex-5-ene, 1-(N-pyrrolidinyl)-3,4-dimethylenehex-5-ene, 1-(N-pyrrolyl)-3,4-dimethylenehex-5-ene, 1-(1,2,3,4-tetrahydro-1-quinolinyl)-3,4-dimethylenehex-5-ene, 1-(1,2,3,4-tetrahydro-2-isoquinolinyl)-3,4-dimethylenehex-5-ene, 1-(N-indolinyl)-3,4-dimethylenehex-5-ene, 1-(N-indolyl)-3,4-dimethylenehex-5-ene, 1-(N-carbazolyl)-3,4-dimethylenehex-5-ene, 1-(N-phenothiazinyl)-3,4-dimethylenehex-5-ene, 1-(N-phenothiazinyl-S-oxide)-3,4-dimethylenehex-5-ene, 1-(N-phenothiazinyl-S,S-dioxide)-3,4-dimethylenehex-5-ene, 1-(N-phenoxazinyl)-3,4-dimethylenehex-5-ene, 1-(4-methyl-1-piperazinyl)-3,4-dimethylenehex-5-ene, 1-(5-methyl-2,5-diazabicyclo[2.2.1]heptan-2-yl)-3,4-dimethylenehex-5-ene, 1-(5-methyl-2,5-diazabicyclo[2.2.2]octan-2-yl)-3,4-dimethylenehex-5-ene, 1-(4-cyclopentyl-1-piperazinyl)-3,4-dimethylenehex-5-ene, 1-(4-cyclopentadienyl-1-piperazinyl)-3,4-dimethylenehex-5-ene, 1-(4-phenyl-1-piperazinyl)-3,4-dimethylenehex-5-ene, 1-(4-(thiazolyl)-1-piperazinyl)-3,4-dimethylenehex-5-ene, 1-(4-(thiadiazolyl)-1-piperazinyl)-3,4-dimethylenehex-5-ene, 1-(4-(triazolyl)-1-piperazinyl)-3,4-dimethylenehex-5-ene, 1-(4-(1,2,3-benzotriazolyl)-1-piperazinyl)-3,4-dimethylenehex-5-ene, 1-(N'-methyl-N-diazepanyl)-3,4-dimethylenehex-5-ene, N,N'-bis(3-methylenepent-4-enyl)diazepane, N,N'-bis(3-methylenepent-4-enyl)piperazine, N,N'-bis(3-methylenepent-4-enyl)dihydrophenazine, N,N'-bis(3-methylenepent-4-enyl)dihydrobenzoindazole, N,N'-bis(3-methylenepent-4-enyl)dihydropermidine, N,N'-bis(3-methylenepent-4-enyl)octahydropyridoquinoline, N,N'-bis(3-methylenepent-4-enyl)octahydropyridoisoquinoline, N,N'-bis(3-methylenepent-4-enyl)hexahydropyrroloquinoline, N,N'-bis(3-methylenepent-4-enyl)hexahydropyrroloisoquinoline, N,N' -bis(3-methylenepent-4-enyl)hexahydropyrroloisoindole, N,N'-bis(3-methylenepent-4-enyl)diazabicyclo[2.2.1]heptane, N,N'-bis(3-methylenepent-4-enyl)diazabicyclo [2.2.2] octane, 1,3-bis(1-(3 -methylenepent-4-enyl)piperidin-4-yl)propane, N,N'-bis(3-methylenehepta-4,6-dien-1-yl)diazepane, N,N'-bis(3-methylenehepta-4,6-dien-1-yl)piperazine, N,N'-bis(3-methylenehepta-4,6-dien-1-yl)dihydrophenazine, N,N'-bis(3-methylenehepta-4,6-dien-1-yl)dihydrobenzoindazole, N,N'-bis(3-methylenehepta-4,6-dien-1-yl)dihydropermidine, N,N'-bis(3-methylenehepta-4,6-dien-1-yl)octahydropyridoquinoline, N,N'-bis(3-methylenehepta-4,6-dien-1-yl)octahydropyridoisoquinoline, N,N'-bis(3-methylenehepta-4,6-dien-1-yl)hexahydropyrroloquinoline, N,N'-bis(3-methylenehepta-4,6-dien-1-yl)hexahydropyrroloisoquinoline, N,N'-bis(3-methylenehepta-4,6-dien-1-yl)hexahydropyrroloisoindole, N,N'-bis(3-methylenehepta-4,6-dien-1-yl)diazabicyclo[2.2.1]heptane, N,N'-bis(3-methylenehepta-4,6-dien-1-yl)diazabicyclo[2.2.2]octane, 1,3-bis(1-(3-methylenehepta-4,6-dienyl)piperidin-4-yl)propane, N,N'-bis(3,4-dimethylenehex-5-en-1-yl)diazepane, N,N'-bis(3,4-dimethylenehex-5-en-1-yl)piperazine, N,N'-bis(3,4-dimethylenehex-5-en-1-yl)dihydrophenazine, N,N'-bis(3,4-dimethylenehex-5-en-1-yl)dihydrobenzoindazole, N,N'-bis(3,4-dimethylenehex-5-en-1-yl)dihydropermidine, N,N'-bis(3,4-dimethylenehex-5-en-1-yl)octahydropyridoquinoline, N,N'-bis(3,4-dimethylenehex-5-en-1-yl)octahydropyridoisoquinoline, N,N'-bis(3,4-dimethylenehex-5-en-1-yl)hexahydropyrroloquinoline, N,N'-bis(3,4-dimethylenehex-5-en-1-yl)hexahydropyrroloisoquinoline, N,N'-bis(3,4-dimethylenehex-5-en-1-yl)hexahydropyrroloisoindole, N,N'-bis(3,4-dimethylenehex-5-en-1-yl)diazabicyclo[2.2.1]heptane, N,N'-bis(3,4-dimethylenehex-5-en-1-yl)diazabicyclo[2.2.2]octane, 1,3-bis(1-(3,4-dimethylenehex-5-enyl)piperidin-4-yl)propane, N,N,N'N'-tetramethyl-3,6-dimethyleneoct-4-ene-1,8-diamine, N,N,N'N'-tetrakis(2-methoxyethyl)-3,6-dimethyleneoct-4-ene-1,8-diamine, N,N'-dimethyl-N,N'-dibenzyl-3,6-dimethyleneoct-4-ene-1,8-diamine, N,N'-(3,6-dimethyleneoct-4-ene-1,8-diyl)bis(morpholine), N,N'-(3,6-dimethyleneoct-4-ene-1,8-diyl)bis(thiomorpholine), N,N'-(3,6-dimethyleneoct-4-ene-1,8-diyl)bis(piperidine), N,N'-(3,6-dimethyleneoct-4-ene-1,8-diyl)bis(pyrrolidine), 4,4'-(3,6-dimethyleneoct-4-ene-1,8-diyl)bis(1-methylpiperazine), N,N,N'N'-tetramethyl-3,4-dimethylenehexane-1,6-diamine, N,N,N'N'-tetrakis(2-methoxyethyl)-3,4-dimethylenehexane-1,6-diamine, N,N'-dimethyl-N,N'-dibenzyl-3,4-dimethylenehexane-1,6-diamine, N,N' -(3,4-dimethylenehexane-1,6-diyl)bis(morpholine), N,N'-(3,4-dimethylenehexane-1,6-diyl)bis(thiomorpholine), N,N'-(3,4-dimethylenehexane-1,6-diyl)bis(piperidine), N,N'-(3,4-dimethylenehexane-1,6-diyl)bis(pyrrolidine), 4,4'-(3,4-dimethylenehexane-1,6-diyl)bis(1-methylpiperazine), N,N,N'N'-tetramethyl-3,4,5-trimethyleneheptane-1,7-diamine, N,N,N'N'-tetrakis(2-methoxyethyl)-3,4,5-trimethyleneheptane-1,7-diamine, N,N'-dimethyl-N,N'-dibenzyl-3,4,5-trimethyleneheptane-1,7-diamine, N,N'-(3,4,5-trimethyleneheptane-1,7-diyl)bis(morpholine), N,N'-(3,4,5-trimethyleneheptane-1,7-diyl)bis(thiomorpholine), N,N'-(3,4,5-trimethyleneheptane-1,7-diyl)bis(piperidine), N,N'-(3,4,5-trimethyleneheptane-1,7-diyl)bis(pyrrolidine), 4,4'-(3,4,5-trimethyleneheptane-1,7-diyl)bis(1-methylpiperazine), or a combination thereof.

**8.** A difunctional addition-polymerizable monomer composition comprising an amine-derivatized alpha-methyl styrene (ADAMS) monomer according to structure (V) and/or an aminated conjugated aliphatic methylated polyene (ACAMP) monomer according to structure (VI):

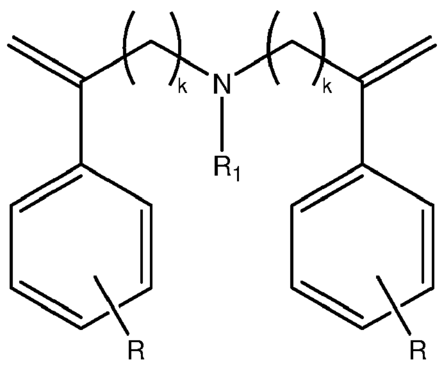
(V)

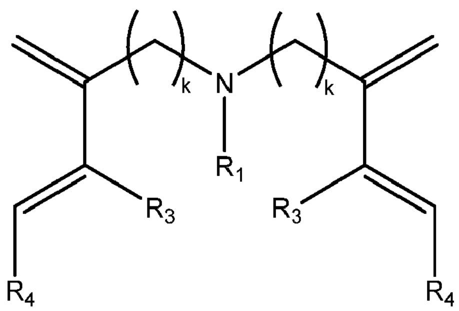
(VI)

wherein:

k is an integer from 1 to 3;
$R_1$ and $R_2$ are each independently a hydrocarbyl group or a hydrocarbonaceous group having 1 to 4 additional heteroatoms selected from the group consisting of O, N, S, P, Se, and combinations thereof;
in structure (V), each R is independently hydrogen, a phenyl ring co-attached at two neighboring ring carbon positions with the phenyl ring shown so as to form a naphthalene assembly, a phenyl group attached at a single carbon of the phenyl ring shown, a $C_1$-$C_4$ hydrocarbyl group, or a $C_1$-$C_6$ hydrocarbyl group containing 1 to 4 additional heteroatoms selected from the group consisting of O, N, S, P, Se, and combinations thereof; and
in structure (VI), $R_3$ and $R_4$ are each independently hydrogen, a methyl group, or an alkenyl group having structure (IV)

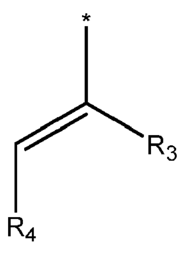
(IV)

attached via the asterisk, but specifically which do not collectively achieve aromaticity in combination with the other alkenes in structure (VI), and with the proviso that $R_3$ and $R_4$ are not both alkenyl groups having structure (IV) and are not both methyl groups.

**9.** A method of making an addition-polymerizable monomer composition comprising the steps of:

providing reactants comprising a source of formaldehyde, a source of a secondary amine, and a polymerizable reactant comprising one or more of alpha-methylstyrene, alpha-methyl-para-methylstyrene, alpha-methyl-ortho-methylstyrene, alpha-methyl-para-ethylstyrene, alpha-methyl-ortho-ethylstyrene, alpha-methyl-para-propylstyrene, alpha-methyl-ortho-propylstyrene, alpha-methyl-para-butylstyrene, alpha-methyl-ortho-butylstyrene, alpha-methyl-para-methoxystyrene, alpha-methyl-ortho-methoxystyrene, alpha-methyl-para-ethoxystyrene, alpha-methyl-ortho-ethoxystyrene, isopropenylnaphthalene, diisopropenylbenzene, diisopropenylnaphthalene, isoprene, 2,3-dimethylbuta-1,3-diene, 2-methylhexa-1,3,5-triene, 2,5-dimethylhexa-1,3,5-triene, 2-methyl-3-methylenepenta-1,4-diene, and 2,4-dimethyl-3-methylenepenta-1,4-diene;
providing an acidic reaction medium sufficient to provide, in the presence of the reactants, a pH from 0.8 to 4.8;
reacting the reactants in the acidic reaction medium for a sufficient reaction time and at a sufficient reaction temperature to thereby yield an amine-derivatized alpha-methyl styrene (ADAMS) monomer and/or an aminated conjugated aliphatic methylated polyene (ACAMP) monomer according to any one of claims 1-7.

**10.** The method of claim 9, wherein:

the source of formaldehyde comprises, at least in part, formaldehyde, trioxane, paraformaldehyde, or a combination thereof; and/or
the source of secondary amine comprises, at least in part, dimethylamine, N,N,N’,N’-tetramethyl-diaminomethane, diethylamine, N,N,N’,N’-tetraethyl-diaminomethane, di-n-propylamine, diisopropylamine, di-n-butylamine, di-sec-butylamine, di-isobutylamine, di-tert-butylamine, cyclohexylmethylamine, dicyclohexylamine, di-(2-ethylhexyl)amine, di-(methoxyethyl)amine, di-(ethoxyethyl)amine, di-(phenoxyethyl)amine, di-(methylthioethyl)amine, di-(ethylthioethyl)amine, benzylmethylamine, dibenzylamine, benzylphenylamine, diphenylamine, dipyridylamine, phenylmethylamine, phenylmethoxylethylamine, benzylmethoxyethylamine, morpholine, thiomorpholine, piperidine, pyrrolidine, dihydropyrrole, pyrrole, tetrahydro-1-quinoline, tetrahydro-2-isoquinoline, indoline, indole, carbazole, phenothiazine, phenothiazine-S-oxide, phenothiazine-S,S-dioxide, phenoxazine, N-methylpiperazine, piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or a combination thereof,
with the proviso that the secondary amine and the polymerizable reactant are not both difunctional.

**11.** The method of claim 9 or claim 10,
wherein the acidic reaction medium comprises formic acid, propionic acid, a butanoic acid, a pentanoic acid, a hexanoic acid, benzoic acid, chloroacetic acid, fluoroacetic acid, oxalic acid, malonic acid, maleic acid, fumaric acid, citric acid, orthophosphoric acid, nitric acid, dichloroacetic acid, difluoroacetic acid, trichloroacetic acid, trifluoroacetic acid, methanesulfonic acid, perchloric acid, or a combination thereof.

**12.** The method of any one of claims 9-11,
wherein the reaction temperature ranges from 40°C to 190°C.

**13.** The method of any one of claims 9-12,
wherein the reacting step comprises a primary reaction step at a first reaction temperature and for a first reaction time, as a result of which more than 500 wppm, based on the reaction medium-free composition weight, of hydroxylation byproduct is formed, followed by a secondary reaction step at a second reaction temperature greater than the first reaction temperature and for a second reaction time to reduce the hydroxylation byproduct content by at least 50%, wherein the secondary reaction step comprises introducing an acid anhydride to convert at least a portion of the hydroxylation byproduct to a corresponding acetate intermediate, and optionally converting at least a portion of the corresponding acetate intermediate to exo isomer monomer, and optionally wherein not more than 0.3 wt%, based on the reaction medium-free composition weight, of an acid anhydride is present following the secondary reaction step.

**14.** A method of making an addition-polymerizable monomer composition comprising the steps of:

providing reactants comprising a source of formaldehyde, a source of a primary amine, and a polymerizable reactant comprising one or more of alpha-methylstyrene, alpha-methyl-para-methylstyrene, alpha-methyl-ortho-methylstyrene, alpha-methyl-para-ethylstyrene, alpha-methyl-ortho-ethylstyrene, alpha-methyl-para-propylstyrene, alpha-methyl-ortho-propylstyrene, alpha-methyl-para-butylstyrene, alpha-methyl-ortho-butylstyrene, alpha-methyl-para-methoxystyrene, alpha-methyl-ortho-methoxystyrene, alpha-methyl-para-ethoxystyrene, alpha-methyl-ortho-ethoxystyrene, isopropenylnaphthalene, isoprene, 2-methylhexa-1,3,5-triene, and 2-methyl-3-methylenepenta-1,4-diene;
providing an acidic reaction medium sufficient to provide, in the presence of the reactants, a pH from 0.8 to 4.8;
reacting the reactants in the acidic reaction medium for a sufficient reaction time and at a sufficient reaction temperature to thereby yield the amine-derivatized alpha-methyl styrene (ADAMS) monomer and/or the aminated conjugated aliphatic methylated polyene (ACAMP) monomer according to any one of claims 8-13.

**15.** A method of making an addition-polymerizable monomer composition comprising the steps of:

providing reactants comprising a secondary amine, a source of methyltriphenylphosphonium halide, and a ketone reactant comprising a $C_1$-$C_3$ alkyl optionally substituted phenyl or naphthalenyl ketone, a $C_1$-$C_3$ alkyl olefinic mono- or di- unsaturated ketone, a phenylene- or naphthalene- bis($C_1$-$C_3$ alkan-1-one), 1,1-ethylenebis(k-alkanone), 1,2-ethylenebis(k-alkanone), and/or bis($C_1$-$C_3$ alkyl)diketone, wherein the ketone reactant includes a terminal alkyl carbon bearing a suitable leaving group or wherein the ketone reactant does not include a terminal alkyl carbon bearing a suitable leaving group and a source of formaldehyde is added to the reaction;
first reacting the secondary amine with the ketone reactant to form an aminated ketone condensation intermediate;
then reacting the aminated ketone condensation intermediate with the source of methyltriphenylphosphonium halide under strong basic (Wittig reaction) conditions to thereby yield the amine-derivatized alpha-methyl styrene (ADAMS) monomer and/or the aminated conjugated aliphatic methylated polyene (ACAMP) monomer according to claim 1; and
optionally removing condensation byproduct from the first reacting step and/or triphenylphosphine oxide byproduct from the second reacting step.

**16.** The method of claim 15, wherein one or more of the following is satisfied:

the suitable leaving group on each terminal alkyl carbon is a halide;
the source of methyltriphenylphosphonium halide comprises methyltriphenylphosphonium chloride, methyltriphenylphosphonium iodide, methyltriphenylphosphonium bromide, or a combination thereof; and
the secondary amine comprises dimethylamine, diethylamine, di-n-propylamine, diisopropylamine, di-n-butylamine, di-sec-butylamine, di-isobutylamine, di-tert-butylamine, cyclohexylmethylamine, dicyclohexylamine, di-(2-ethylhexyl)amine, di-(methoxyethyl)amine, di-(ethoxyethyl)amine, di-(phenoxyethyl)amine, di-(methylthioethyl)amine, di-(ethylthioethyl)amine, benzylmethylamine, dibenzylamine, benzylphenylamine, diphenylamine, dipyridylamine, phenylmethylamine, phenylmethoxylethylamine, benzylmethoxyethylamine, morpholine, thiomorpholine, piperidine, pyrrolidine, dihydropyrrole, pyrrole, tetrahydro-1-quinoline, tetrahydro-2-isoquinoline, indoline, indole, carbazole, phenothiazine, phenothiazine-S-oxide, phenothiazine-S,S-dioxide, phenoxazine, N-methylpiperazine, piperazine, diazepane, dihydrophenazine, diazabicyclo[2.2.1]heptane, diazabicyclo[2.2.2]octane, octahydropyridoisoquinoline, octahydropyridoquinoline, hexahydropyrroloisoquinoline, hexahydropyrroloquinoline, hexahydropyrroloisoindole, dihydrobenzoindazole, dihydropermidine, 1,3-di(piperidin-4-yl)propane, or a combination thereof, with the proviso that the secondary amine and the ketone reactant are not both difunctional.

**17.** The method of any one of claims 15-16, wherein the source of formaldehyde comprises, at least in part, formaldehyde, trioxane, paraformaldehyde, or a combination thereof.

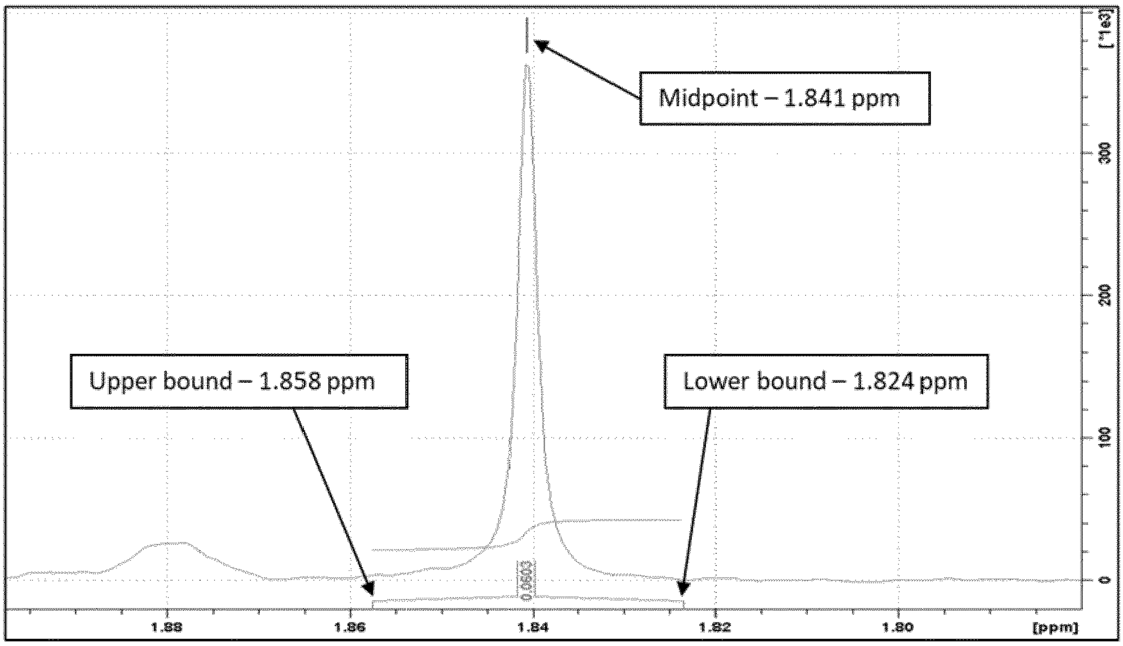


Figure 1.

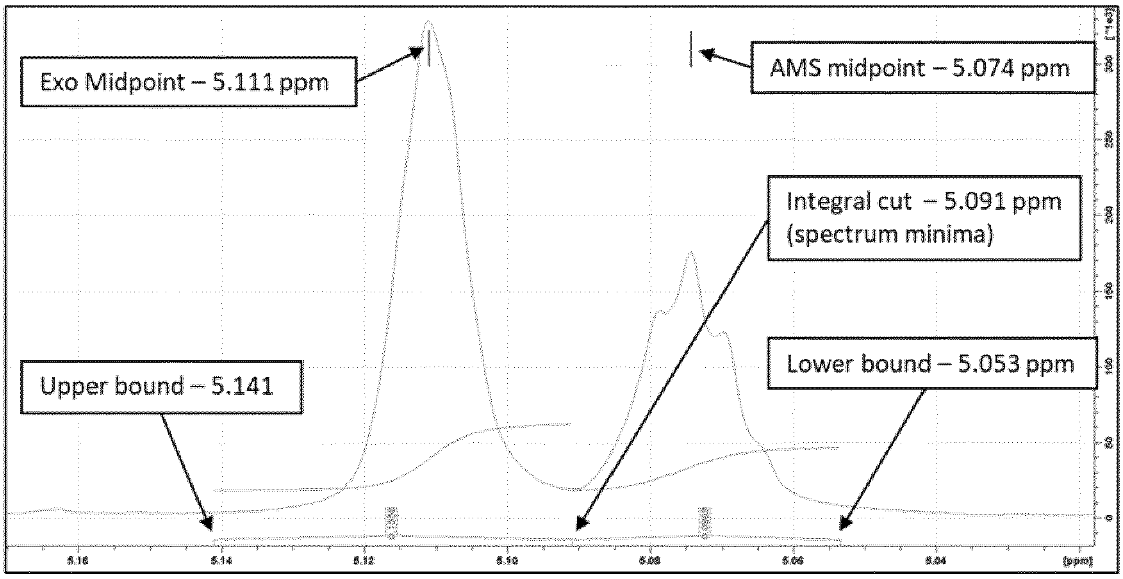


Figure 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2778826 A
- GB 1381755 A
- US 7790661 B
- US 7960320 B
- US 8778854 B
- US 10414999 B
- WO 2021127183 A


### Non-patent literature cited in the description


- **T. COHEN et al.** *J. Org. Chem.,* 1983, vol. 48, 4531-37